# EUROPEAN PATENT APPLICATION

(11) **EP 4 818 178 A1**
(43) Date of publication of application: **07.10.2026**
(21) Application number: 25747663.0
(22) Date of filing: 21.01.2025
(51) Int. Cl.: C07D 519/00, A61K 31/519, A61P 35/00, A61P 35/02

(54) **PREPARATION AND USE OF PYRIMIDOTHIOPYRANONE KRAS MUTANT PROTEIN INHIBITOR**

(30) Priority: 30.01.2024 CN 202410133666; 26.11.2024 CN 202411703261
(71) Applicant: Ya Therapeutics Inc, Shanghai 201808 (CN)
(72) Inventor: LIANG, Yonghong, Shanghai 201808 (CN)
(74) Representative: Cleanthous, Marinos
(86) International application number: PCT/CN2025/073749
(87) International publication number: WO 2025/162091

(57) **Abstract**

The present invention relates to KRAS G 12D mutant protein pyrimidothiopyranone inhibitors and their uses. Specifically, the present invention provides a compound shown in formula (I), where each substitution is defined as described in the specification. In addition, it also involves the combination of the inhibitor and its application. The compound of the present invention has good activity in inhibiting tumor growth, and it has good security.

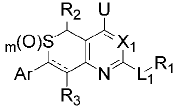

## Description

### CROSS-REFERENCE TO RELATED APPLICATIONS

This application claims priority to International Application Nos. PCT/CN 202410133666, filed on Jan 30, 2024, the content of each of which is incorporated herein by reference in its entirety.

### BACKGROUD OF THE INVENTION

RAS represents a population of 189 amino acid monomeric globular proteins (21 kDa molecular weight) that are associated with the plasma membrane and bind to GDP or GTP, and RAS acts as a molecular switch. When the RAS contains bound GDP, it is in a stationary or closed position and is "inactive." When cells are exposed to certain growth-promoting stimuli, RAS is induced to exchange their bound GDP for GTP. In the case of binding to GTP, RAS is "opened" and is capable of interacting with other proteins (its "downstream targets") and activating the proteins. The RAS protein itself has an inherently low ability to hydrolyze GTP back to GDP, thereby turning itself into a closed state. Closing RAS requires an exogenous protein called GTPase activating protein (GAP) that interacts with RAS and greatly accelerates the conversion of GTP to GDP. Any mutation in RAS that affects its ability to interact with GAP or convert GTP back to GDP will result in prolonged protein activation, and thus conduction to the cell to inform its signaling of continued growth and division. Since these signals cause cell growth and division, over-activated RAS signaling can ultimately lead to cancer.

Structurally, the RAS protein contains a G domain responsible for the enzymatic activity of RAS-guanine nucleotide binding and hydrolysis (GTPase reaction). It also contains a C-terminal extension called the CAAX cassette, which can be post-translationally modified and responsible for targeting the protein to the membrane. The G domain is approximately 21-25 kDa in size and contains a phosphate binding ring (P-ring). The P-loop represents a pocket of a binding nucleotide in a protein, and this is a rigid portion of a domain with conserved amino acid residues necessary for nucleotide binding and hydrolysis (glycine 12, threonine 26 and lysine 16). The G domain also contains a so-called switch I region (residues 30-40) and a switch II region (residues 60-76), both of which are dynamic parts of the protein, since the dynamic portion is converted between stationary and loaded states. The ability is often expressed as a "spring loaded" mechanism. The primary interaction is the hydrogen bond formed by threonine-35 and glycine-60 with the gamma-phosphate of GTP, which maintains the active conformation of the switch 1 region and the switch 2 region, respectively. After hydrolysis of GTP and release of phosphate, the two relax into an inactive GDP conformation.

The most notable members of the RAS subfamily are HRAS, KRAS and NRAS, which are primarily involved in many types of cancer. Mutation of any of the three major isoforms of the RAS gene (HRAS, NRAS or KRAS) is one of the most common events in human tumor formation. Approximately 30% of all tumors in human tumors were found to carry some mutations in the RAS gene. It is worth noting that KRAS mutations were detected in 25%-30% of tumors. In contrast, the rate of carcinogenic mutations in NRAS and HRAS family members was much lower (8% and 3%, respectively). The most common KRAS mutations were found at residues G12 and G13 in the P-loop as well as at residue Q61.G12C is a frequently occurring KRAS gene mutation (glycine-12 is mutated to cysteine). This mutation has been found in about 13% of cancers, about 43% in lung cancer, and almost 100% in MYH-associated polyposis (familial colon cancer syndrome).

As a frontier target, KRAS^{G12C} mutant protein has attracted extensive attention. Among them, AMG-510 developed by Amgen was approved by the FDA for market last year. In recent years, other companies have filed multiple patents for KRAS inhibitors, such as WO2016164675, WO2016168540, WO2021141628, WO2022098625, WO2022087371, WO2020101736, WO2022109485, WO2022109487, and WO2020146613. Therefore, despite the progress made in this field, there remains a need for improved compounds and methods for treating cancer, such as through the inhibition of KRAS, HRAS, or NRAS. This invention addresses this need and provides additional related advantages.

In short, the present invention provides compounds capable of inhibiting KRAS mutations, including their stereoisomers, pharmaceutically acceptable salts, tautomers, and prodrugs.

### SUMMARY

The present invention provides a compound of formula (I), a pharmaceutically acceptable salt thereof or an isomer thereof,

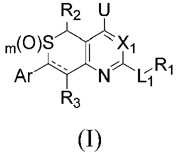

Each L1 is selected from a single bond, OC₀₋₆ alkyl, NHC₀₋₆ alkyl, C₁₋₆ alkyl, COC₀₋₆ alkyl, or SC₀₋₆ alkyl;

Each Ar is independently selected from 6-10 membered aromatic groups or 5-12 membered heteraromatic groups in each occurrence, and the heteroaryl group in each occurrence independently comprises 1, 2, 3, or 4 heteroatoms selected from N, O, or S, wherein the heteroaryl group is optionally substituted by one or more G¹ atoms;

Each X₁ is independently selected from N, CR₄;

Each R₂, R₃, R₄ is independently selected from H, D, CN, halogen, C₁₋₆ alkyl;

Each R ₁is independently selected from H, D, C₁₋₆ alkyl, C₂₋₆ alkenyl, C₂₋₆ ynyl, CN, C₃₋₆ carbocyclic, 3-10 membered heterocycles, 4-10 membered heterofused rings, 5-12 membered spirocyclic groups; the 3-10 membered heterocycles, 4-10 membered heterofused rings, and 5-12 membered spirocyclic groups independently contain 1, 2, 3, or 4 heteroatoms selected from N, O, or S each time they appear; each R1 is independently and optionally substituted or unsubstituted by 1, 2, 3, 4, 5, or 6 substituents selected from deuterium, halogens, C1-6 alkyl, -C1-6 alkoxy, oxo, C1-6 alkyl, C3-6 carbocyclic, and 3-10 membered heterocycles;

Each U is selected from a single bond, 3-8 membered cycloalkyl, 3-8 membered heterocycloalkyl, 5-12 membered fused cycloalkyl, 5-12 membered fused heterocycloalkyl , 5-12 membered spiro cycloalkyl, 5-12 membered spiro heterocycloalkyl, 3-8 membered cycloalkyl, 3-8 membered heterocycloalkyl, 5-12 membered fused cycloalkyl, 5-12 membered fused heterocycloalkyl , 5-12 membered spiro cycloalkyl, 5-12 membered spiro heterocycloalkyl are optionally substituted with 1, 2 or 3 G²;
G¹, G² is independently selected from , CN, halogen, C₁₋₆alkyl, C₂₋₆ alkenyl, C₂₋₆ ynyl, C₃₋₈ carbocyclic, 3-8 membered heterocycles,, C₆₋₁₀ aromatic groups, 5-10 membered heteraromatic groups, -OR₅, -OC(O)NR₅R₆, -C(O)OR₅, -C(O)NR₅R₆, -C(O)R₅, -NR₅R₆, -NR₅C(O)R₆, -NR₅C(O)NR₆R₇, -S(O)ᵢR₅ or -NR₅S(O)ᵢR₆, wherein the alkyl, vinyl, alkynyl, cyclic, heterocyclic, aromatic, and heteroaromatic groups are optionally substituted with one or more D, cyano, halogen, C₁₋₆ alkyl, C₂₋₆ alkenyl, C₂₋₆ alkynyl, C₃₋₈ alkyl, or 3-8-membered heterocyclic, C₆₋₁₀ aryl, 6-10 membered heteroaromatic groups, -OR₈, -OC(O)NR₈R₉, -C(O)OR₈, -C(O)NR₈R₉, -C(O)R₈, -NR₈R₉, -NR₈C(O)R₉, -NR₈C(O)NR₉R₁₀, -S(O)ᵢR₈ or -NR₈S(O)ᵢR_{9;}
R₅, R₆, R₇, R₈, R₉ and R₁₀ are each independently selected from H, D, CN, halogen, C₁₋₆ alkyl, 3-8 membered cycloalkyl, 3-8 membered heterocycloalkyl, sing ring heteroaromatic or phenyl group;
m, i is selected from 1 or 2.

In some embodiments, the compound of general formula (I), its pharmaceutically acceptable salt, or its stereoisomer, is further represented as (II-A), (II-B), (II-C), or (II-D).

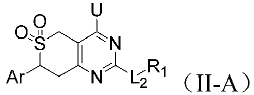

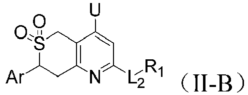

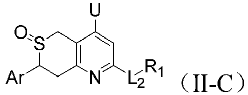

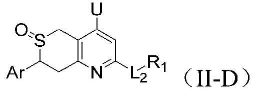

In some embodiments, the compound of general formula (I), its pharmaceutically acceptable salt, or its stereoisomer, is further represented as (II-A), (II-B), (II-C), (II-D), (II-E), or (II-F).

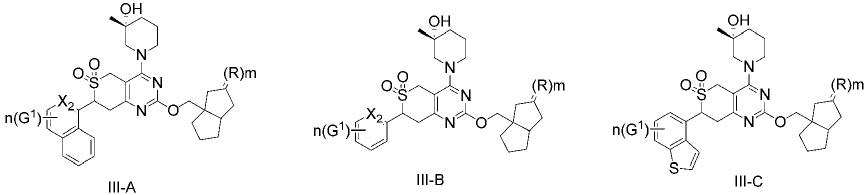

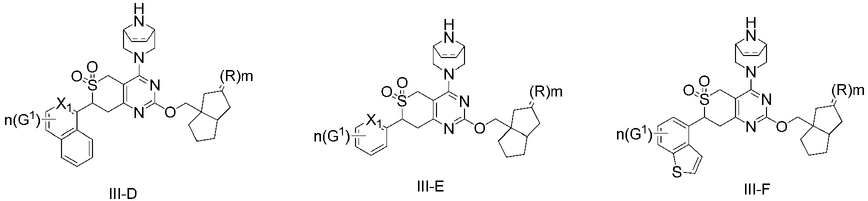

In some embodiments, the compound of formula (I) or an isomer, solvate or precursor thereof, or a pharmaceutically acceptable salt thereof is selected from the group consisting of the following compounds, isomers, solvates thereof or their precursors, or their pharmaceutically acceptable salts:

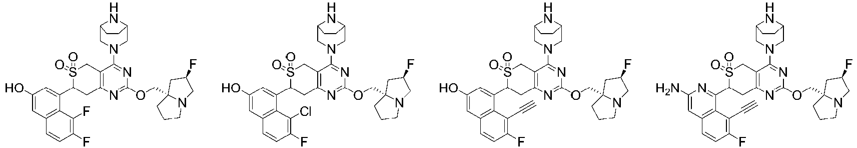

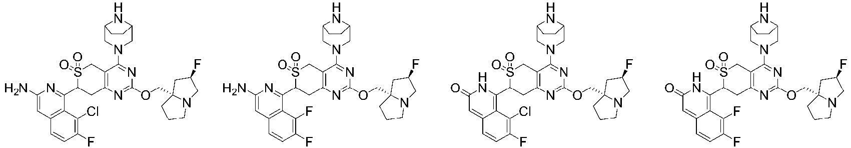

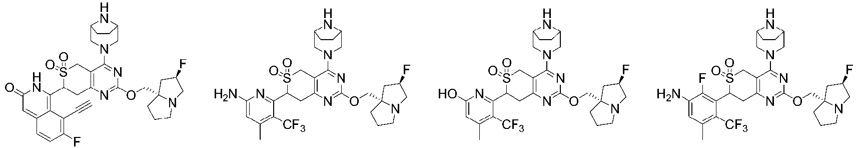

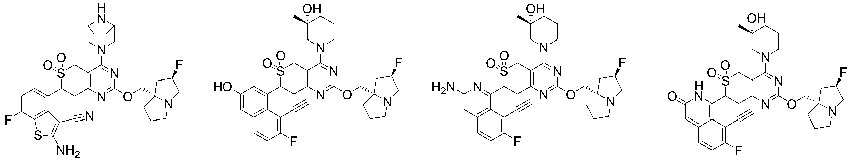

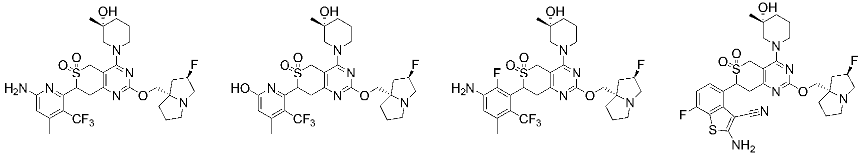

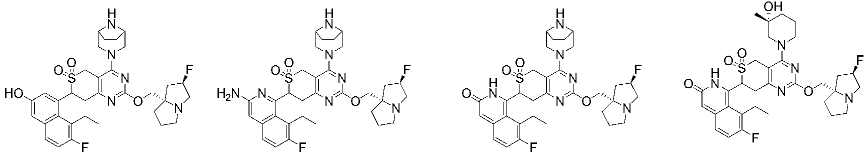

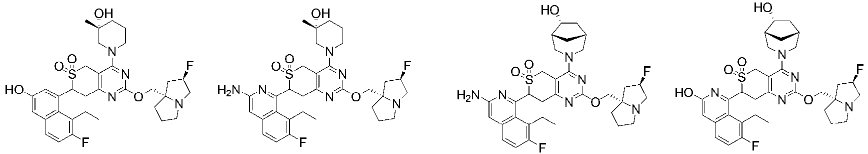

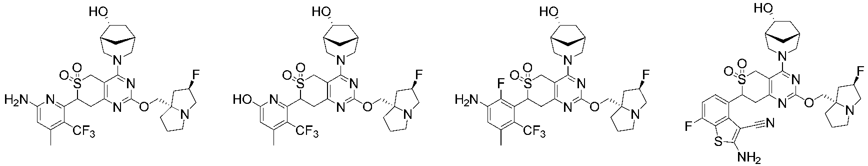

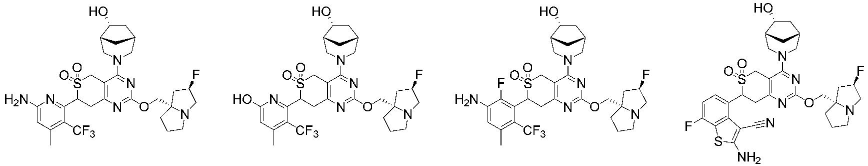

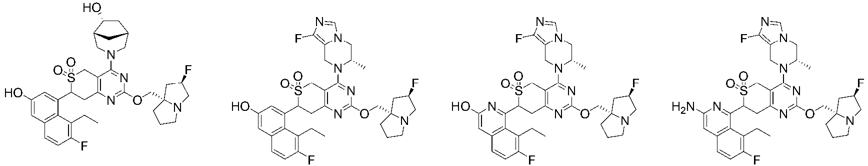

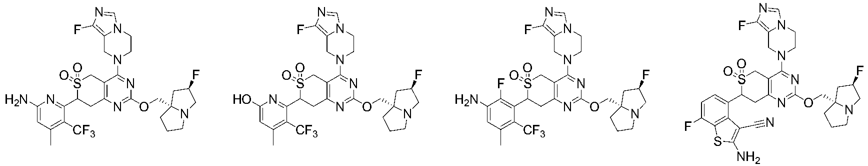

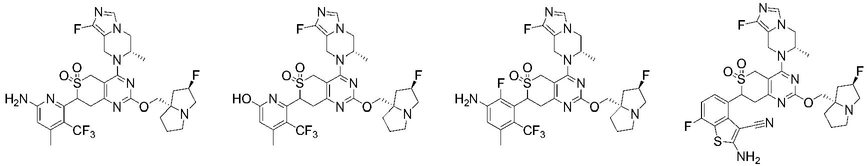

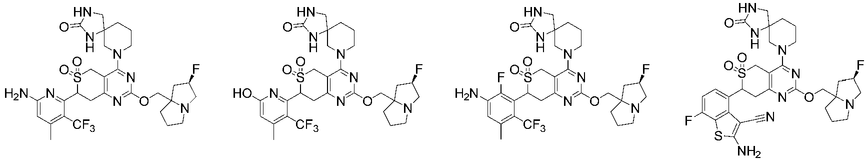

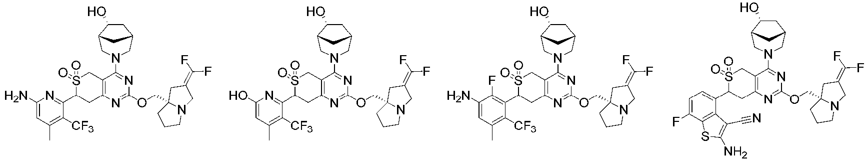

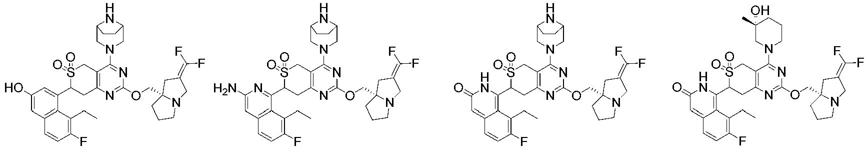

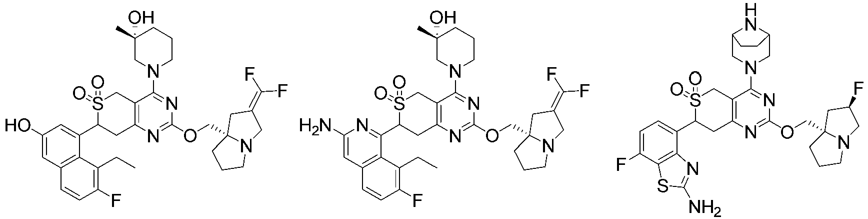

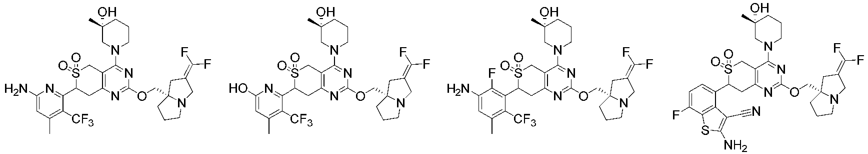

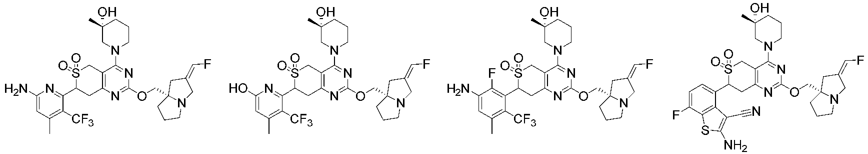

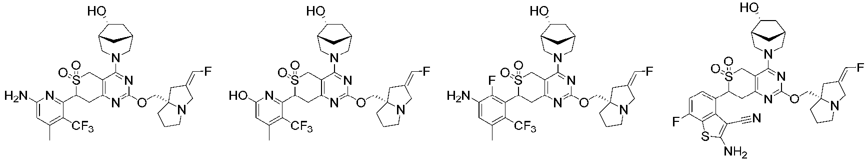

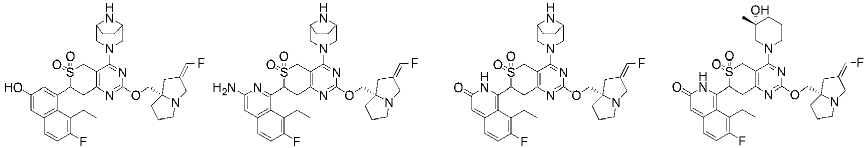

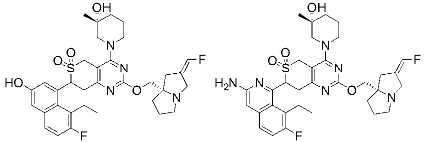

In some embodiments, the compound of formula (I) or an isomer, solvate or precursor thereof, or a pharmaceutically acceptable salt thereof is selected from the group consisting of the following compounds, isomers, solvates thereof or their precursors, or their pharmaceutically acceptable salts:

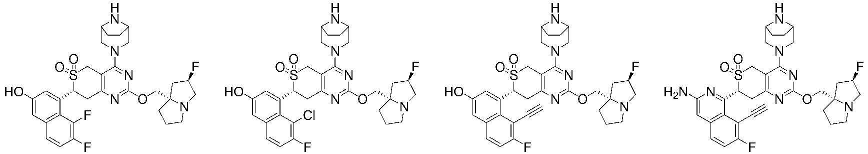

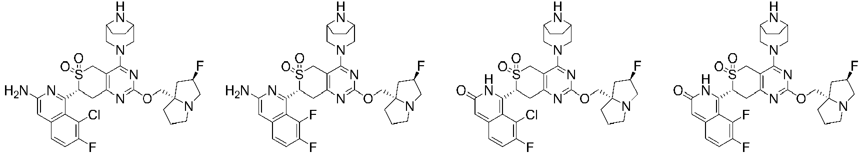

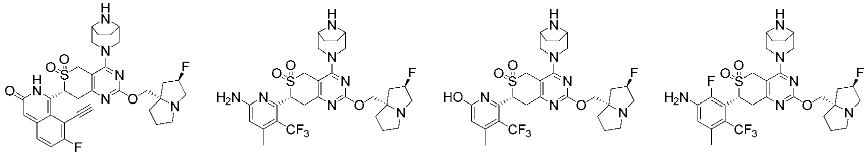

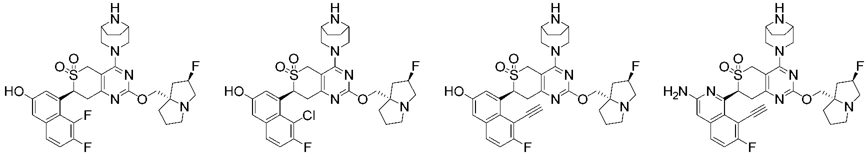

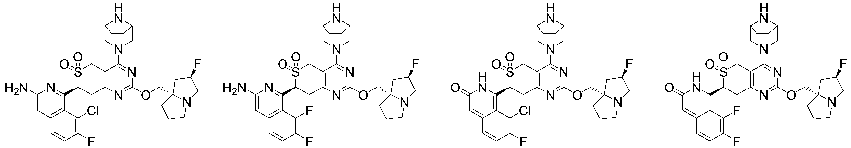

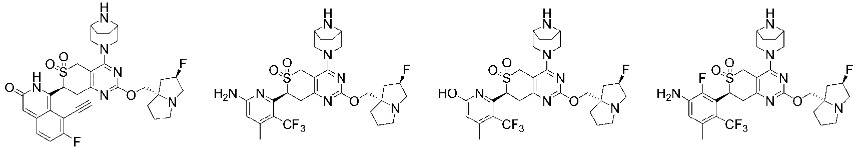

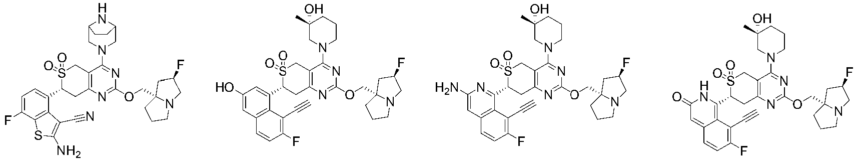

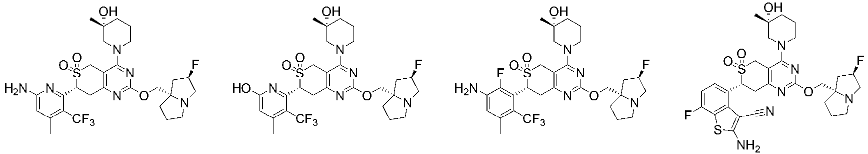

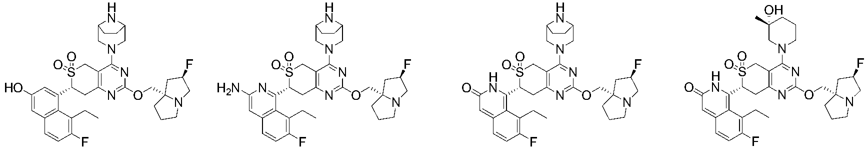

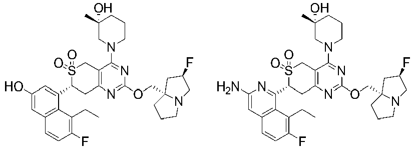

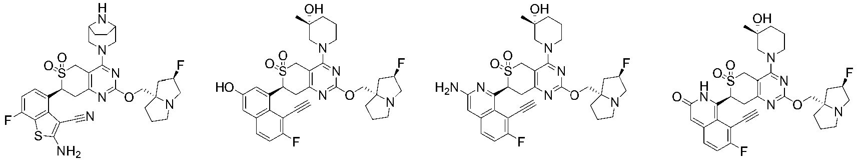

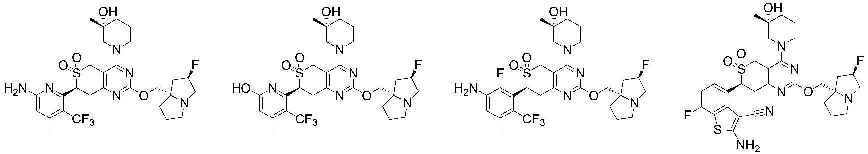

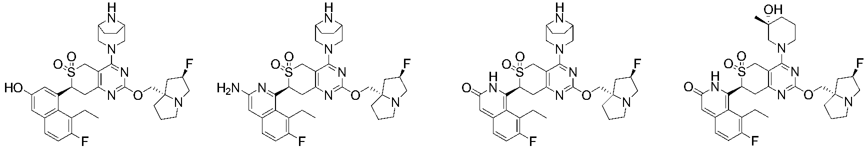

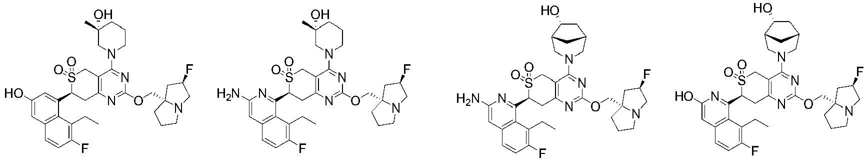

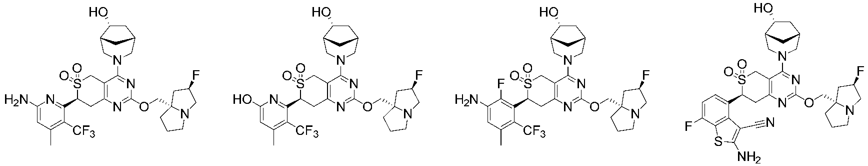

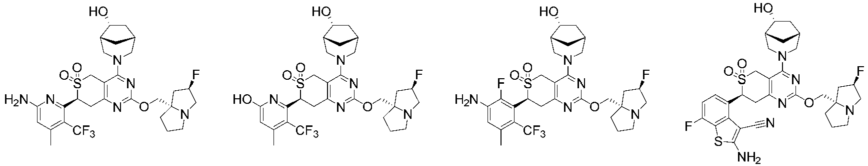

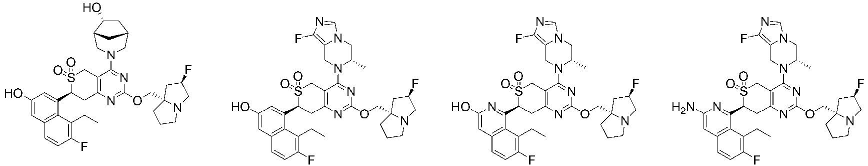

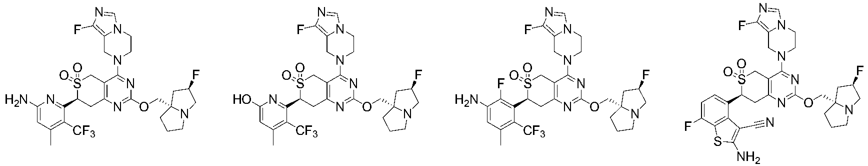

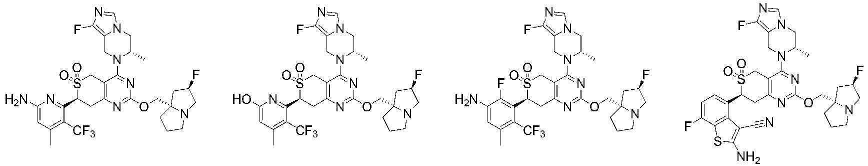

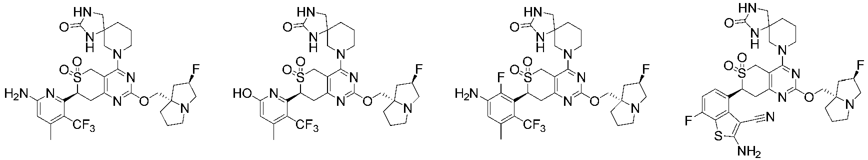

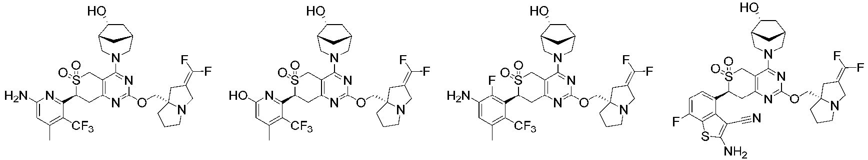

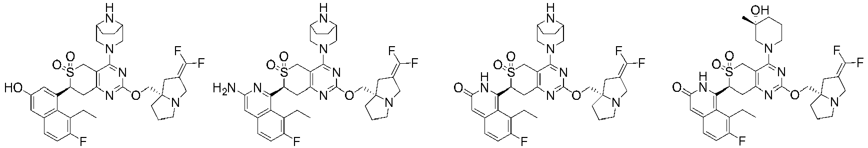

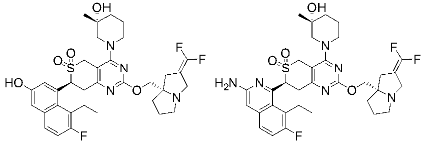

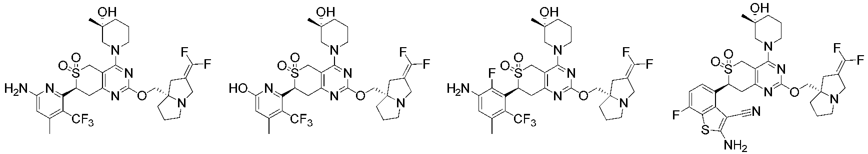

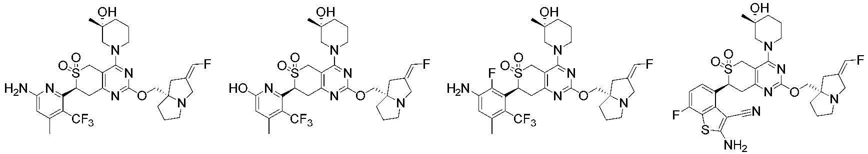

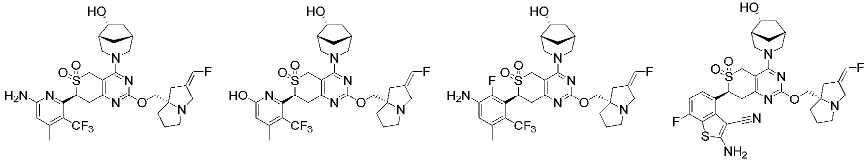

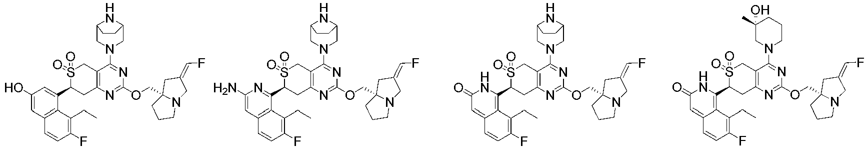

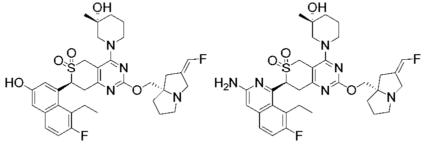

On the other hand, the present invention also provides pharmaceutical compositions comprising compounds of formulas (I) and (II) or pharmaceutically acceptable salts thereof and pharmaceutically acceptable excipients thereof.

On the other hand, the present invention relates to a method for treating diseases in mammals related to KRas, KRas G12A, KRas G12C, KRas G12D, KRas G12R, KRas G12S, KRas G12V, KRas G13D or KRas Q61H, comprising administering to a mammal, preferably a human, a therapeutically effective amount of a compound of formulas (I) and (II) or a pharmaceutically acceptable salt thereof, or a pharmaceutical composition thereof.

On the other hand, the present invention relates to the use of compounds of formulas (I) and (II) or pharmaceutically acceptable salts thereof in the prevention or treatment of diseases related to KRas, KRas G12A, KRas G12C, KRas G12D, KRas G12R, KRas G12S, KRas G12V, KRas G13D, or KRas Q61H.

On the other hand, the present invention relates to compounds of formulas (I) and (II) or pharmaceutically acceptable salts thereof, or pharmaceutical compositions thereof, in the prevention or treatment of diseases related to KRas, KRas G12A, KRas G12C, KRas G12D, KRas G12R, KRas G12S, KRas G12V, KRas G13D, or KRas Q61H.

### Chemical terms

Unless stated to the contrary, the following terms are used in the specification and claims.

The notation "C_{x-y}" as used herein means a range of carbon atoms, where x and y are both integers, e.g. C₃₋₈cycloalkyl means a cycloalkyl group having 3-8 carbon atoms , ie a cycloalkyl group having 3, 4, 5, 6, 7 or 8 carbon atoms. It should also be understood that "C₃₋₈" also includes any subrange therein, such as C₃₋₇, C₃₋₆, C₄₋₇, C₄₋₆, C₅₋₆, and the like.

"Alkyl" refers to a straight line containing 1 to 20 carbon atoms, such as 1 to 18 carbon atoms, 1 to 12 carbon atoms, 1 to 8 carbon atoms, 1 to 6 carbon atoms, or 1 to 4 carbon atoms chain or branched hydrocarbyl group. Non-limiting examples of alkyl groups include methyl, ethyl, n-propyl, isopropyl, n-butyl, isobutyl, tert-butyl, sec-butyl, n-pentyl, 1,1-dimethylpropyl 1,2-dimethylpropyl, 2,2-dimethylpropyl, 1-ethylpropyl, 2-methylbutyl, 3-methylbutyl, n-hexyl, 1-ethyl -2-methylpropyl, 1,1,2-trimethylpropyl, 1,1-dimethylbutyl, 1,2-dimethylbutyl, 2,2-dimethylbutyl, 1 , 3-dimethylbutyl and 2-ethylbutyl. The alkyl group may be substituted or unsubstituted.

"Alkenyl" refers to a straight or branched chain hydrocarbyl group containing at least one carbon-carbon double bond and usually 2 to 20 carbon atoms, such as 2 to 8 carbon atoms, 2 to 6 carbon atoms, or 2 to 4 carbon atoms group. Non-limiting examples of alkenyl groups include vinyl, 1-propenyl, 2-propenyl, 1-butenyl, 2-butenyl, 3-butenyl, 2-methyl-2-propenyl, 1-butenyl , 4-pentadienyl and 1,4-butadienyl. The alkenyl group may be substituted or unsubstituted.

"Alkynyl" refers to a straight or branched chain hydrocarbon group containing at least one carbon-carbon triple bond and usually 2 to 20 carbon atoms, eg, 2 to 8 carbon atoms, 2 to 6 carbon atoms, or 2 to 4 carbon atoms group. Non-limiting examples of alkynyl groups include ethynyl, 1-propynyl, 2-propynyl, 1-butynyl, 2-butynyl, and 3-butynyl. The alkynyl group may be substituted or unsubstituted.

"Cycloalkyl" refers to a saturated cyclic hydrocarbyl substituent containing 3 to 14 carbon ring atoms. Cycloalkyl groups may be monocarbocyclic rings, usually containing 3 to 7 carbon ring atoms. Non-limiting examples of monocyclic cycloalkyl groups include cyclopropyl, cyclobutyl, cyclopentyl, cyclohexyl, and cycloheptyl. Cycloalkyl groups may optionally be bi- or tricyclic rings fused together, such as decalinyl, which may be substituted or unsubstituted.

"Heterocyclyl", "heterocycloalkyl" and "heterocycle" refer to a stable 3-18-membered monovalent non-aromatic ring, including 2-12 carbon atoms, 1-6 atoms selected from nitrogen, oxygen and sulfur heteroatoms. Unless otherwise specified, a heterocyclyl group may be a monocyclic, bicyclic, tricyclic or tetracyclic ring system, which may contain fused, spiro or bridged ring systems, with nitrogen, carbon or sulfur selectivity on the heterocyclyl group is oxidized, the nitrogen atom can be selectively quaternized, and the heterocyclic group can be partially or fully saturated. A heterocyclyl group can be attached to the rest of the molecule by a single bond through a carbon atom or a heteroatom in the ring. A heterocyclyl group containing a fused ring may contain one or more aromatic or heteroaromatic rings, so long as the attachment to the remainder of the molecule is an atom on a non-aromatic ring. For the purposes of this application, the heterocyclyl group is preferably a stable 4-11 membered monovalent non-aromatic monocyclic or bicyclic ring containing 1-3 heteroatoms selected from nitrogen, oxygen and sulfur, more preferably a stable 4-8 membered monovalent non-aromatic monocyclic ring containing 1-3 heteroatoms selected from nitrogen, oxygen and sulfur. Non-limiting examples of heterocyclyl groups include azepanyl, azetidinyl, decahydroisoquinolyl, dihydrofuranyl, indoline, dioxolane, 1,1- Dioxo-thiomorpholinyl, imidazolidinyl, imidazolinyl, isothiazolidinyl, isoxazolidinyl, morpholinyl, octahydroindolyl, octahydroisoindolyl, oxazinyl, piper oxazinyl, piperidinyl, 4-piperidinyl, pyranyl, pyrazolidine, pyrrolidinyl, quinazinyl, quinuclidinyl, tetrahydrofuranyl, tetrahydropyranyl and the like.

"Spiroheterocyclyl" refers to a 5- to 20-membered polycyclic heterocyclic group with one atom (called a spiro atom) shared between the monocyclic rings, wherein one or more ring atoms are selected from nitrogen, oxygen or S(O)m (where m is an integer from 0 to 2) and the remaining ring atoms are carbon. These may contain one or more double bonds, but none of the rings have a fully conjugated electron system preferably 6 to 14 membered, more preferably 7 to 10 membered. Spirocycloalkyl groups are classified into mono-spiroheterocyclyl, bis-spiro-heterocyclyl or poly-spiro-heterocyclyl according to the number of spiro atoms shared between rings, preferably mono-spirocycloalkyl and bis-spirocycloalkyl. More preferably, it is a 4-membered/4-membered, 4-membered/5-membered, 4-membered/6-membered, 5-membered/5-membered or 5-membered/6-membered monospirocyclic group. Non-limiting examples of spiroheterocyclyl include:

"Fused heterocyclic group" refers to a 5- to 20-membered polycyclic heterocyclic group in which each ring in the system shares an adjacent pair of atoms with other rings in the system, and one or more rings may contain one or more bicyclic bonds, but none of the rings have a fully conjugated pi electron system, where one or more ring atoms are selected from nitrogen, oxygen, or a heteroatom of S(O)ₘ (where m is an integer from 0 to 2), and the remaining ring atoms are carbon. Preferably it is 6 to 14 yuan, more preferably 7 to 10 yuan. According to the number of formed rings, it can be divided into bicyclic, tricyclic, tetracyclic or polycyclic fused heterocycloalkyl, preferably bicyclic or tricyclic, more preferably 5-membered/5-membered or 5-membered/6-membered bicyclic fused heterocyclic group . Non-limiting examples of fused heterocyclyl groups include:
"Aryl" refers to an aromatic monocyclic or fused polycyclic group containing 6 to 14 carbon atoms, preferably 6 to 10 membered, such as phenyl and naphthyl, more preferably phenyl. The aryl ring can be fused to a heteroaryl, heterocyclyl or cycloalkyl ring, wherein the ring attached to the parent structure is an aryl ring.

"Heteroaryl" or "heteroaryl" refers to a 5-16 membered ring system comprising 1-15 carbon atoms, preferably 1-10 carbon atoms, 1-4 selected from nitrogen, oxygen and sulfur heteroatoms, at least one aromatic ring. Unless otherwise specified, a heteroaryl group may be a monocyclic, bicyclic, tricyclic or tetracyclic ring system, which may contain fused or bridged ring systems, so long as the point of attachment to the rest of the molecule is an aromatic ring atom, the Nitrogen, carbon and sulfur atoms can be selectively oxidized, and nitrogen atoms can be selectively quaternized. For the purposes of the present invention, the heteroaryl group is preferably a stable 4-11 membered monoaromatic ring containing 1-3 heteroatoms selected from nitrogen, oxygen and sulfur, more preferably a stable 5-8 membered monoaromatic ring , which contains 1-3 heteroatoms selected from nitrogen, oxygen and sulfur. Non-limiting examples of heteroaryl groups include acridinyl, azepinyl, benzimidazolyl, benzindolyl, benzodioxinyl, benzodioxinyl, benzofuranonyl, benzoyl furanyl, benzonaphthofuryl, benzopyranone, benzopyranyl, benzopyrazolyl, benzothiadiazolyl, benzothiazolyl, benzotriazolyl, furanyl, Imidazolyl, indazolyl, indolyl, oxazolyl, purinyl, pyrazinyl, pyrazolyl, pyridazinyl, pyridyl, pyrimidinyl, pyrrolyl, quinazolinyl, quinolinyl, quinine base, tetrazolyl, thiadiazolyl, thiazolyl, thienyl, triazinyl, triazolyl, etc. In the present application, the heteroaryl group is preferably a 5-8 membered heteroaryl group containing 1-3 heteroatoms selected from nitrogen, oxygen and sulfur, more preferably pyridyl, pyrimidinyl, thiazolyl. The heteroaryl group may be substituted or unsubstituted.

"Halogen" refers to fluorine, chlorine, bromine or iodine.

"Hydroxy" refers to -OH, "amino" refers to -NH₂, "amido" refers to -NHCO-, "cyano" refers to -CN, "nitro" refers to -CN, "isocyano" refers to -NC, "tri "Fluoromethyl" refers to -CF₃.

The term "heteroatom" or "hetero" as used herein, alone or as part of other components, refers to atoms other than carbon and hydrogen, the heteroatoms being independently selected from oxygen, nitrogen, sulfur, phosphorus, silicon, selenium, and tin, Without being limited to these atoms, in embodiments where two or more heteroatoms are present, the two or more heteroatoms may be the same as each other, or some or all of the two or more heteroatoms may be different. .

The term "fused" or "fused ring" as used herein, alone or in combination, refers to a cyclic structure in which two or more rings share one or more bonds.

The term "spiro" or "spirocycle" as used herein, alone or in combination, refers to a cyclic structure in which two or more rings share one or more atoms.

"Optional" or "optionally" means that the subsequently described event or circumstance may but need not occur, and that the description includes instances where the event or circumstance occurs or does not occur. For example, "heterocyclyl optionally substituted with alkyl "Group" means that an alkyl group may, but need not, be present, and the description includes the case where the heterocyclic group is substituted with the alkyl group and the case where the heterocyclic group is not substituted with the alkyl group.

"Substituted" means that one or more atoms in a group, preferably 5, more preferably 1 to 3 atoms, are independently of each other substituted with the corresponding number of substituents. It goes without saying that the substituents are in their possible chemical positions, and the person skilled in the art can determine (either experimentally or theoretically) possible or impossible substitutions without undue effort. For example, carbon atoms with free amine or hydroxyl groups bound to carbon atoms with unsaturated (e.g., olefinic) bonds may be unstable. The substituents include, but are not limited to, hydroxyl, amine, halogen, cyano, C₁₋₆ alkyl, C₁₋₆ alkoxy, C₂₋₆ alkenyl, C₂₋₆ alkynyl, C₃₋₈ cycloalkyl, etc..

"Pharmaceutical composition" refers to a composition comprising one or more of the compounds described herein, or a pharmaceutically acceptable salt or prodrug thereof, and other components such as pharmaceutically acceptable carriers and excipients. The purpose of the pharmaceutical composition is to facilitate the administration to the organism, facilitate the absorption of the active ingredient and then exert the biological activity.

"Isomers" refer to compounds that have the same molecular formula but differ in the nature or order in which their atoms are bonded or in the spatial arrangement of their atoms. ". Stereoisomers include optical isomers, geometric isomers and conformational isomers. The compounds of the present invention may exist in the form of optical isomers. These optical isomers are of the "R" or "S" configuration, depending on the configuration of the substituents around the chiral carbon atom. Optical isomers include enantiomers and diastereomers, and methods of making and separating optical isomers are known in the art.

The compounds of the present invention may also exist as geometric isomers. The present invention contemplates various geometric isomers and mixtures thereof resulting from the distribution of substituents around carbon-carbon double bonds, carbon-nitrogen double bonds, cycloalkyl or heterocyclic groups. Substituents around a carbon-carbon double bond or carbon-nitrogen bond are designated as the Z or E configuration, and substituents around a cycloalkyl or heterocycle are designated as the cis or trans configuration.

The compounds of the present invention may also exhibit tautomerism, such as keto-enol tautomerism.

It is to be understood that the present invention includes any tautomeric or stereoisomeric form and mixtures thereof, and is not limited to any one tautomeric or stereoisomeric form used in the naming of the compounds or chemical formulae.

"Isotopes" are all isotopes of atoms occurring in the compounds of the present invention. Isotopes include those atoms with the same atomic number but different mass numbers. Examples of isotopes suitable for incorporation into the compounds of the present invention are hydrogen, carbon, nitrogen, oxygen, phosphorus, fluorine, and chlorine, such as, but not limited to, ²H, ³H, ¹³C, ¹⁴C, ¹⁵N, ¹³O, ³¹P, ³²P, ³⁵S, ¹⁸F, respectively, and ³⁶Cl. Isotopically labeled compounds of the present invention can generally be prepared by conventional techniques known to those skilled in the art or by methods analogous to those described in the accompanying Examples, using the appropriate isotopically labeled reagents in place of non-isotopically labeled reagents. Such compounds have various potential uses, e.g., as standards and reagents in the determination of biological activity. In the case of stable isotopes, such compounds have the potential to advantageously alter biological, pharmacological or pharmacokinetic properties.

"Prodrug" means that a compound of the present invention can be administered in the form of a prodrug. Prodrugs refer to derivatives that are converted into biologically active compounds of the present invention under physiological conditions in vivo, e.g., by oxidation, reduction, hydrolysis, etc., each with or without enzymes. Examples of prodrugs are compounds in which the amine group in the compounds of the present invention is acylated, alkylated or phosphorylated, such as eicosanoylamino, propylamineamido, pivaloyloxymethylamine , or wherein the hydroxyl group is acylated, alkylated, phosphorylated or converted to a boronate salt such as acetoxy, palmitoyloxy, pivaloyloxy, succinyloxy, fumaryloxy, alanyloxy Oxygen groups, or in which the carboxyl group is esterified or amidated, or in which the sulfhydryl group forms disulfide bridges with carrier molecules such as peptides that selectively deliver drugs to the target and/or to the cytosol of cells, these compounds may be used by the present invention The compounds are prepared according to known methods.

"Pharmaceutically acceptable salt" or "pharmaceutically acceptable" means prepared from pharmaceutically acceptable bases or acids, including inorganic bases or acids and organic bases or acids. Where the compounds of the present invention contain one or more acidic or basic groups, the present invention also includes their corresponding pharmaceutically acceptable salts. Thus, the compounds of the invention which contain acidic groups can exist in salt form and can be used according to the invention, for example as alkali metal salts, alkaline earth metal salts or as ammonium salts. More precise examples of such salts include sodium, potassium, calcium, magnesium salts or with amines or organic amines such as primary, secondary, tertiary, cyclic, etc., such as ammonia, isopropylamine, trimethylamine, dimethine Particularly preferred organic bases such as ethylamine, triethylamine, tripropylamine, ethanolamine, diethanolamine, ethanolamine, dicyclohexylamine, ethylenediamine, purine, piperazine, piperidine, choline and caffeine are isopropylamine, Salts of ethylamine, ethanolamine, trimethylamine, dicyclohexylamine, choline and caffeine. The compounds of the invention which contain basic groups can exist in salt form and can be used according to the invention in the form of their additions to inorganic or organic acids. Examples of suitable acids include hydrochloric acid, hydrobromic acid, phosphoric acid, sulfuric acid, phosphoric acid, methanesulfonic acid, p-toluenesulfonic acid, naphthalenedisulfonic acid, oxalic acid, acetic acid, tartaric acid, lactic acid, salicylic acid, benzoic acid, formic acid, propylene acid, pivalic acid, malonic acid, succinic acid, pimelic acid, fumaric acid, maleic acid, malic acid, sulfamic acid, phenylpropionic acid, gluconic acid, ascorbic acid, isonicotinic acid, citric acid , adipic acid and other acids known to those skilled in the art. If the compounds of the present invention contain both acidic and basic groups in the molecule, the present invention includes, in addition to the salt forms mentioned, inner or betaine salts. The respective salts are obtained by conventional methods known to those skilled in the art, for example by contacting these with organic or inorganic acids or bases in solvents or dispersants or by anion exchange or cation exchange with other salts.

Accordingly, when referring to "a compound", "a compound of the present invention" or "a compound of the present invention" in this application, it includes all such compound forms, such as prodrugs, stable isotope derivatives, pharmaceutically acceptable salts, Isomers, mesomers, racemates, enantiomers, diastereomers and mixtures thereof.

As used herein, the term "tumor" includes benign tumors and malignant tumors (e.g., cancer).

As used herein, the term "cancer" includes various malignancies in which Kras are involved, including but not limited to non-small cell lung cancer, esophageal cancer, melanoma, rhabdomyosarcoma, cell carcinoma, multiple myeloma, Breast ovarian cancer, uterine lining cancer, cervical cancer, stomach cancer, node cancer, bladder cancer, pancreatic cancer, lung cancer, breast cancer, prostate cancer and liver cancer (e.g. hepatocellular carcinoma), more particularly liver cancer, stomach cancer and bladder cancer.

As used herein, the terms "effective amount," "therapeutically effective amount," or "pharmaceutically effective amount" refer to at least one agent or compound sufficient to alleviate, to some extent, one or more symptoms of the disease or disorder being treated upon administration. amount. The result can be a reduction and/or remission of signs, symptoms or causes or any other desired change in a biological system. For example, an "effective amount" for treatment is that amount of a composition comprising a compound disclosed herein required to provide clinically significant relief of a condition. An effective amount appropriate in any individual case can be determined using techniques such as dose escalation assays.

As used herein, the term "polymorph" or "polymorph (phenomenon)" means that the compounds of the present invention have multiple crystal lattice forms. Some compounds of the present invention may have more than one crystal form. The present invention covers All polymorphs or mixtures thereof.

Intermediate compounds of the compounds of the present invention and polymorphs thereof are also within the scope of the present invention.

Crystallization often results in solvates of the compounds of the present invention, and the term "solvate" as used herein refers to a complex consisting of one or more molecules of the compound of the present invention in combination with one or more molecules of a solvent.

The solvent may be water, in which case the solvate is a hydrate. In addition, an organic solvent may be used. Accordingly, the compounds of the present invention may exist as hydrates, including monohydrates, dihydrates, hemihydrates, trihydrates, tetrahydrates, and the like, as well as the corresponding solvated forms. The compounds of the present invention may be true solvates, but in other cases, the compounds of the present invention may only accidentally retain water or a mixture of water and some other solvent. The compounds of the present invention may be reacted in a solvent or in a solvent Precipitation or crystallization. Solvates of the compounds of the present invention are also included within the scope of the present invention.

The term "acceptable" as used herein in relation to a formulation, composition or ingredient means no persistent detrimental effect on the general health of the subject being treated.

As used herein, the term "pharmaceutically acceptable" refers to a substance (eg, a carrier or diluent) that does not affect the biological activity or properties of the compounds of the present invention, and is relatively non-toxic, i.e., the substance can be administered to an individual without causing adverse biological effects React or interact in an undesirable manner with any component contained in the composition.

"Pharmaceutically acceptable carrier" includes, but is not limited to, adjuvants, carriers, excipients, auxiliaries, deodorants, diluents, preservatives, Dyes/colorants, flavor enhancers, surfactants and wetting agents, dispersing agents, suspending agents, stabilizers, isotonic agents, solvents, or emulsifiers.

The terms "subject", "patient", "subject" or "individual" as used herein refer to an individual suffering from a disease, disorder or condition, etc., including mammals and non-mammals, examples of mammals include, but are not limited to, the class of mammals Any member of: humans, non-human primates (e.g. chimpanzees and other apes and monkeys); domestic animals such as cattle, horses, sheep, goats, pigs; domestic animals such as rabbits, dogs and cats; laboratory animals , including rodents such as rats, mice and guinea pigs. Examples of non-human mammals include, but are not limited to, birds, fish, and the like. In one embodiment of the related methods and compositions provided herein, the mammal is a human.

The term "treatment" as used herein refers to the treatment of related disease conditions in mammals, particularly humans, including
(i) preventing mammals, particularly mammals who have been previously exposed to a disease or disorder but have not been diagnosed with the disease or disorder, from developing a corresponding disease or disorder;
(ii) inhibiting the disease or disorder, i.e. controlling its development;
(iii) alleviating the disease or condition, i.e. causing regression of the disease or condition;
(iv) Relief of symptoms caused by a disease or disorder.

As used herein, the terms "disease" and "disorder" can be used interchangeably or have different meanings, because some specific diseases or conditions have no known causative agent (so the cause of the disease is not clear), so they cannot be considered as A disease can only be seen as an unwanted condition or syndrome which has more or less specific symptoms that have been confirmed by clinical researchers.

The terms "administering," and the like, as used herein, refer to methods that enable the delivery of a compound or composition to the desired site of biological action. These include, but are not limited to, oral routes, duodenal routes, parenteral injection (including intravenous, subcutaneous, intraperitoneal, intramuscular, intraarterial injection or infusion), topical administration, and rectal administration. In preferred embodiments, the compounds and compositions discussed herein are administered orally.

### Examples

The present invention also provides methods for preparing the compounds. The preparation of the compounds of the general formula (I) of the present invention can be accomplished by the following exemplary methods and examples, but these methods and examples should not be construed as limiting the scope of the present invention in any way. The compounds of the present invention can also be synthesized using synthetic techniques known to those skilled in the art, or a combination of methods known in the art and methods described in the present invention. The products obtained in each step are obtained by separation techniques known in the art, including but not limited to extraction, filtration, distillation, crystallization, chromatographic separation, and the like. The starting materials and chemical reagents required for the synthesis can be routinely synthesized or purchased according to the literature (reaxys).

Unless otherwise stated, temperatures are in degrees Celsius. Reagents were purchased from commercial suppliers such as Chemblocks Inc and 3wpharm, and these reagents were used without further purification unless otherwise stated.

Unless otherwise stated, the following reactions were performed at room temperature, in anhydrous solvent, under positive pressure of nitrogen or gas, or using a drying tube; glassware drying and/or heat drying.

Unless otherwise stated, column chromatography used 200-300 mesh silica gel from Qingdao Ocean Chemical Factory; preparative thin layer chromatography used thin layer chromatography silica gel prefabricated plate (HSGF254) produced by Yantai Chemical Industry Research Institute; MS was measured with Therno LCD Fleet type (ESI) liquid chromatography-mass spectrometer.

Nuclear magnetic data (¹H NMR) use BrukerAvance-400MHz or Varian Oxford-400Hz nuclear magnetic instrument, the solvents used for nuclear magnetic data are CDCl₃, CD3OD, D₂O, DMS-*d*₆, etc., based on tetramethylsilane (0.000ppm) or residual solvent As the benchmark (CDCl₃: 7.26 ppm; CD₃OD: 3.31ppm; D₂O: 4.79 ppm; d6-DMSO: 2.50 ppm) When the peak shape diversity is indicated, the following abbreviations indicate different peak shapes: s (single peak), d (double peak) ), t (triplet), q (quartet), m (multiplet), br (broad), dd (double doublet), dt (double triplet). If a coupling constant is given, it is in Hertz (Hz).

### Preparation of intermediates

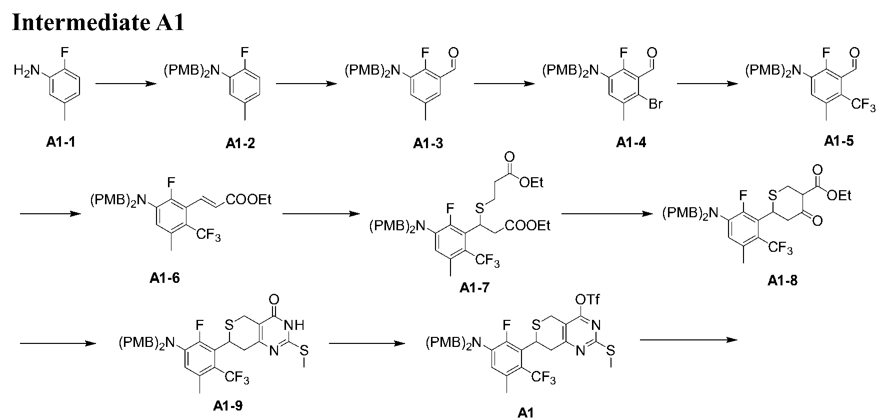

### Step A

In a dry 2L three necked bottle, NaH (0.8 g, 19 mmol) was added to DMF (10 mL), and the reaction system was heterogeneous gray. The temperature was lowered to 0 ° C, and a solution of compound **A1-1** (1 g, 8 mmol) in DMF (200 mL) was added dropwise under N₂ protection. The reaction was continued at 0 ° C for 0.5 hours, and para methoxybenzyl chloride (2.75 g, 17 mmol, 2.4 mL) was added. The temperature was slowly raised to 20 ° C, and stirring was continued for 7.5 hours under nitrogen protection. Slowly add the reaction solution to 10 mL of saturated ammonium chloride, extract with MTBE (10 mL × 2), wash the combined organic phases with 10 mL of saturated saline solution, dry with anhydrous Na₂SO₄, filter, and remove the organic solvent under reduced pressure. The crude product obtained is purified by silica gel column chromatography to obtain compound **A1-2.** LC-MS(ESI): m/z = 365.4[M+H]⁺.

### Step B

Add 2,2.6,6-tetramethylpiperidine (1.14 g, 8.21 mmol) to anhydrous THF (300 mL), cool to -5 ° C, add n-butyl lithium (2.5 M, 4 mL) dropwise, react at -5~0 ° C for 15 minutes, cool to -60 ° C, add compound **A1-2** (0.98 g, 2.7 mmol) in THF (3 mL) solution, react at -60 ° C for 0.5 hours, quickly add DMF (4 g, 0.05 mol), and continue stirring the reaction solution at -60 ° C for 10 minutes. Add 20 mL of saturated ammonium chloride to the reaction solution, extract with MTBE (10 mL × 2), wash the combined organic phases with 10 mL of saturated saline solution, dry with anhydrous Na₂SO₄, filter, and remove the organic solvent under reduced pressure. The crude product obtained is subjected to solvent pulping for 0.5 hours, filtered, dried from the filter cake, concentrated from the filtrate, and purified by silica gel column chromatography. Combine the filter cake and column chromatography to obtain compound A1-3.

LC-MS(ESI): m/z = 393.5[M+H]⁺.

### Step C

Add compound **A1-3** (0.98 g, 2.5 mmol) to 10 mL of DMF, and NBS (0.45 g, 2.5 mmol). Continue stirring the reaction mixture at 20 °C for 20 minutes. Add the reaction solution to 15 mL of water, extract with MTBE(8 mL × 2), wash the combined organic phases with saturated saline (5 mL × 2), dry with anhydrous Na₂SO₄, filter, and remove the organic solvent under reduced pressure. The crude product obtained is beaten with a mixed solvent for 0.5 hours, filtered, and the filter cake is dried to obtain compound **A1-4.** LC-MS(ESI): m/z = 472.4[M+H]⁺.

### Step D

Add compound **A1-4** (0.97 g, 2.04 mmol) to DMF (9.5 mL), and add cuprous iodide (0.78 g, 4.1 mmol) and methyl fluorosulfonyldifluoroacetate (2 g, 10 mmol) to the reaction solution under nitrogen. Heat the reaction solution to 100 ° C and continue stirring for 1 hour. Cool, filter the reaction solution through diatomaceous earth, add the filtrate to 15 mL of water, extract with MTBE (7.5 mL × 2), wash the combined organic phases with saturated saline (10 mL × 2), dry with anhydrous sodium sulfate, filter, and remove the organic solvent under reduced pressure. The crude product obtained is purified by silica gel column chromatography to obtain compound **A1-5.** LC-MS(ESI): m/z =461.5[M+H]⁺.

### Step E

Add anhydrous THF (50 mL) and NaH (0.3 g, 7.3 mmol) to a dry three necked flask, cool to 0 °C, and add ethyl acetate (0.85 g, 7.3 mmol, 0.8 mL) dropwise under nitrogen protection. Continue stirring the reaction mixture at 0 ° C for 0.5 hours, and add n-butyl lithium (2.5 M, 3 mL) dropwise. Continue stirring the reaction mixture at this condition for 0.5 hours, cool to -60 ° C, and add compound **A1-5** (1.15 g, 2.5 mmol) in THF (5 mL) dropwise. Continue stirring the reaction mixture at -60 ° C for 0.5 hours. Add 25 mL of saturated ammonium chloride solution to the reaction solution, extract with 10 mL of ethyl acetate, wash the organic phase with 20 mL of saturated saline solution, dry with anhydrous sodium sulfate, filter, and remove the organic solvent under reduced pressure. The crude product obtained is purified by silica gel column chromatography to obtain compound **A1-6.** LC-MS(ESI): m/z = 531.6[M+H]⁺.

### Step F

Add compound A1-6 (1 g, 1.9 mmol) to dichloromethane (10 mL), add 3-mercaptopropionic acid ethyl ester (0.3 g, 2.3 mmol) and titanium tetrachloride, and stir the reaction at room temperature for 48 hours. Filter, reduce pressure to remove organic solvents, and purify the crude product by silica gel column chromatography to obtain compound A1-7. LC/MS(ESI): m/z =665.7[M+H]⁺

### Step G

Compound **A1-7** (0.80 g, 1.2 mmol) and sodium ethoxide (0.57 mg, 1.9 mmol) were dissolved in 10 mL of THF and reacted with the solution at room temperature for 24 hours. The mixture was then filtered and dried to obtain **A1-8** .LC/MS(ESI): m/z =619.7[M+H]⁺.

### Step H

To a suspension of **A1-8** (3.3 g, 5.3 mmol) in water (10 mL), S-methylisothiourea sulfate (1.7 g, 6.2 mmol) and DMF (1.6 g, 11.7 mmol) were added at room temperature. The reaction mixture was stirred for 20 hours. The precipitate was collected, washed twice with water and isopropyl ether, and dried to give **A1-9.** LC/MS(ESI): m/z = 645.7[M+H]⁺.

### Step I

Compound **A1-9** (1 g, 1.6 mmol) was dissolved in dichloromethane (10 mL), and N,N-diisopropylethylamine (0.6 g, 4.8 mmol) was added. The mixture was cooled to 0-10°C, and trifluoromethanesulfonic anhydride (0.7 g, 2.4 mmol, 400 µL) was slowly added to the reaction solution. The reaction was carried out at this temperature for 15 minutes. The reaction solution was poured into a saturated ammonium chloride aqueous solution (8 mL), and the mixture was separated. The aqueous phase was extracted with dichloromethane (5 mL × 2). The combined organic phases were dried over anhydrous sodium sulfate, filtered, and the organic solvent was removed under reduced pressure. The crude product was pulped, filtered, and the filter cake was dried to obtain **A1.** LC/MS(ESI): m/z =777.8[M+H]⁺.

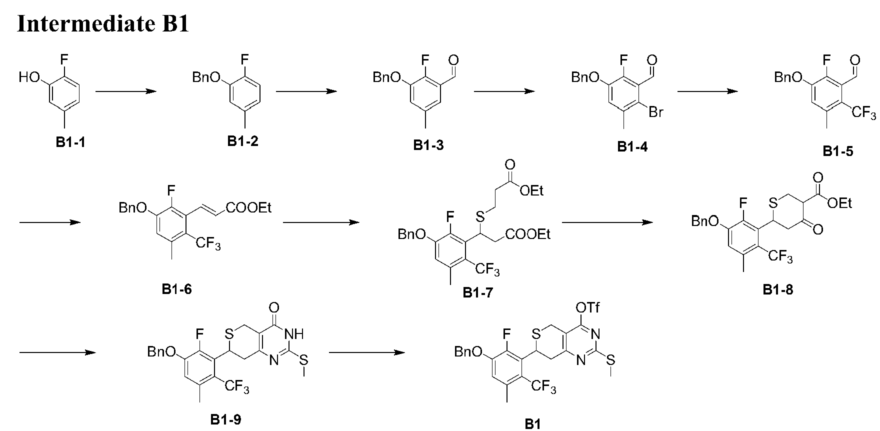

### Step A

To a solution of **B1-1** (25.3 g, 201 mmol) in N,N-dimethylformamide (500 mL), bromomethylbenzene (41.2 g, 241 mmol) and DMF (55.5 g, 402 mmol) were added. After stirring at 25 °C for 15 hours, the reaction mixture was diluted with ethyl acetate and washed with water and brine. The organic layer was dried over anhydrous sodium sulfate, filtered, and concentrated under reduced pressure. Purification was performed by silica gel column chromatography to obtain **B1-2.** LC-MS(ESI): m/z = 216.3[M+H]⁺.

### Step B

2,2,6,6-Tetramethylpiperidine (11.4 g, 82.1 mmol) was added to anhydrous THF(500 mL), the mixture was cooled to -5 °C, and n-butyllithium (2.5 M, 40 mL) was added dropwise. The reaction was carried out at -5 to 0 °C for 15 minutes. The mixture was then cooled to -60 °C, and a THF(30 mL) solution of compound **B1-2** (5.8 g, 27 mmol) was added. The reaction was carried out at -60 °C for 0.5 hours. N,N-Dimethylformamide (40 g, 0.5 mol) was added rapidly, and the reaction mixture was stirred at -60 °C for 10 minutes. 200 mL of saturated ammonium chloride was added to the reaction mixture, and the mixture was extracted with MTBE (100 mL × 2). The combined organic phases were then extracted with 100 mL of MTBE. Wash with mL of saturated saline solution, dry with anhydrous sodium sulfate, filter, remove organic solvent under reduced pressure, and the crude product is slurried with solvent for 0.5 hours, filtered, the filter cake is dried, the filtrate is concentrated, and then purified by silica gel column chromatography. The filter cake and column chromatography residues are combined to obtain the compound **B1-3.** LC-MS(ESI): m/z = 245.1[M+H]⁺.

### Step C

Compound **B1-3** (6.1 g, 25 mmol) was added to 100 mL of N,N-dimethylformamide, followed by bromosuccinimide (4.5 g, 25 mmol). The reaction mixture was stirred at 20 °C for 20 minutes. The reaction mixture was then added to 150 mL of water and extracted with MTBE (80 mL × 2). The combined organic phases were washed with saturated brine (50 mL × 2), dried over anhydrous sodium sulfate, filtered, and the organic solvent was removed under reduced pressure. The crude product was then slurried with a mixed solvent for 0.5 hours, filtered, and the filter cake was dried to obtain the compound **B1-4.** LC-MS(ESI) : m/z = 323.2[M+H]⁺.

### Step D

Compound **B1-4** (6.57 g, 20.4 mmol) was added to N,N-dimethylformamide (95 mL). Under nitrogen atmosphere, cuprous iodide (7.8 g, 41 mmol) and methyl fluorosulfonyl difluoroacetate (20 g, 100 mmol) were added to the reaction mixture. The mixture was heated to 100 °C and stirred for 1 hour. After cooling, the reaction mixture was filtered through diatomaceous earth. The filtrate was added to 150 mL of water and extracted with MTBE (75 mL × 2). The combined organic phases were washed with saturated brine (100 mL × 2), dried over anhydrous sodium sulfate, filtered, and the organic solvent was removed under reduced pressure. The crude product was purified by silica gel column chromatography to obtain the compound **B1-5.** LC-MS(ESI): m/z = 313.3[M+H]⁺.

### Step E

Under nitrogen protection, **B1-5** (4.06 g, 13 mmol) and lithium hydroxide (0.82 g, 19.5 mmol) were dissolved in 100 mL of THF at room temperature. Then, dimethyl ethylphosphonoacetate (3.55 g, 19.5 mmol) was added. The reaction mixture was stirred at room temperature for 24 hours. The THF was recovered and added to 150 mL of water. The mixture was extracted with MTBE (75 mL × 2). The combined organic phases were washed with saturated brine (100 mL × 2), dried over anhydrous sodium sulfate, filtered, and the organic solvent was removed under reduced pressure. The crude product was purified by silica gel column chromatography to obtain the compound **B1-6.** LC-MS(ESI): m/z = 383.2 [M+H]⁺.

### Step F

Compound **B1-6** (7.64 g, 20 mmol) and piperidine (0.2 mL) were added to dichloromethane (100 mL), followed by ethyl 3-mercaptopropionate (3.2 g, 24 mmol) and benzyltrimethylammonium hydroxide (1 mL, 30% methanol). The mixture was stirred at 60°C for 48 hours. The mixture was extracted from the dichloromethane (75 mL × 2) with dilute hydrochloric acid. The combined organic phases were washed with saturated sodium bicarbonate (50 mL) and saturated brine (50 mL × 2), dried over anhydrous sodium sulfate, filtered, and the organic solvent was removed under reduced pressure. The crude product was purified by silica gel column chromatography to obtain the compound **B1-7.** LC-MS(ESI): m/z =517.2 [M+H]⁺.

### Step G

Compound **B1-7** (6.2 g, 12 mmol) and sodium ethoxide (1.36 g, 19 mmol) were dissolved in 400 mL of toluene and reacted with stirring at room temperature for 24 hours. The mixture was poured into 100 mL of water and extracted with dichloromethane (100 mL × 2). The combined organic phases were washed with saturated brine (50 mL × 2), dried over anhydrous sodium sulfate, filtered, and the organic solvent was removed under reduced pressure. The crude product was purified by silica gel column chromatography to obtain the compound **B1-8.** LC-MS(ESI): m/z =471.1[M+H]⁺.

### Step H

S-methylisothiourea sulfate (1.7 g, 6.2 mmol) and DMF (1.6 g, 11.7 mmol) were added to a suspension of **B1-8** (2.49 g, 5.3 mmol) in water (10 mL) at room temperature. The reaction mixture was stirred for 20 hours. The precipitate was collected, washed twice with water and isopropyl ether, dried, and the crude product was purified by silica gel column chromatography to obtain the compound **B1-9.** LC-MS(ESI): m/z = 497.1[M+H]⁺.

### Step I

Compound **B1-9** (0.79 g, 1.6 mmol) was dissolved in dichloromethane (10 mL), and N,N-diisopropylethylamine (0.6 g, 4.8 mmol) was added. The mixture was cooled to 0-10°C, and trifluoromethanesulfonic anhydride (0.7 g, 2.4 mmol, 400 µL) was slowly added to the reaction solution. The reaction was allowed to proceed at this temperature for 15 minutes. The reaction solution was then poured into a saturated ammonium chloride aqueous solution (8 mL), and the mixture was separated. The aqueous phase was extracted with dichloromethane (5 mL × 2). The combined organic phases were dried over anhydrous sodium sulfate, filtered, and the organic solvent was removed under reduced pressure. The crude product was purified by silica gel column chromatography to obtain the compound **B1.** LC/MS(ESI): m/z =629.0[M+H]⁺.

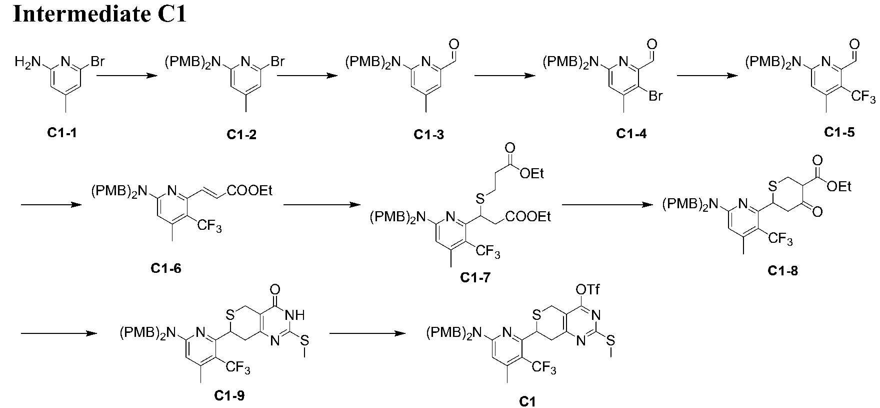

Compound **C1** was obtained using a similar preparation method to intermediate A1 (with the starting material replaced by 6-bromo-4-methylpyridine-2-amine). LC/MS(ESI): m/z =760.8[M+H]⁺.

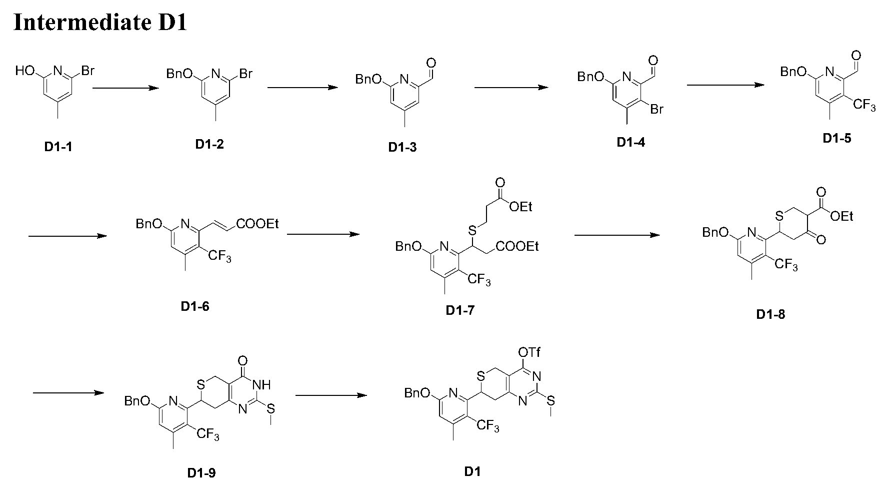

Compound **D1** was obtained using a similar preparation method to intermediate B1 (with the starting material replaced by 6-bromo-4-methylpyridin-2-ol). LC/MS(ESI): m/z =611.6[M+H]⁺.

### Intermediate E1

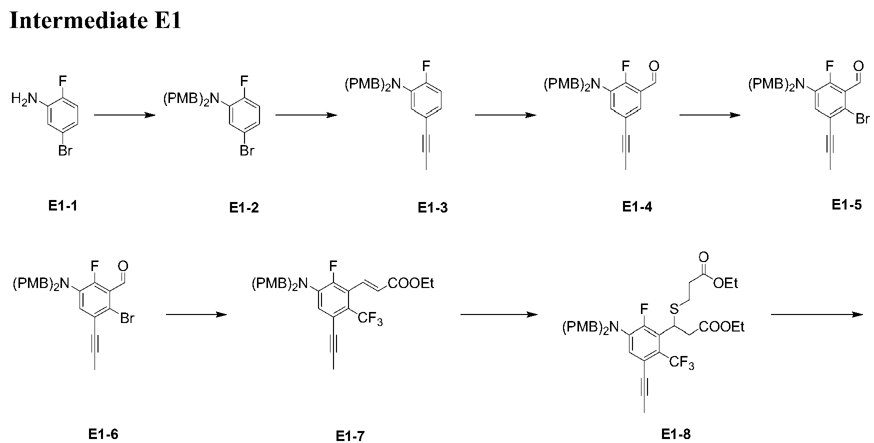

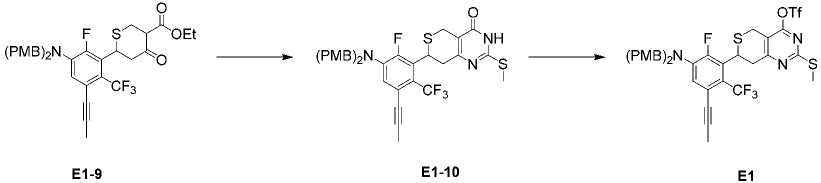

### Step A

In a dry 1L three-necked flask, DMF (27.6 g, 200 mmol) was added to N,N-dimethylformamide (100 mL). The mixture was cooled to 0 °C, and under nitrogen protection, a solution of compound E1-1 (10 g, 80 mmol) in N,N-dimethylformamide (500 mL) was added dropwise. The reaction was continued at 0 °C for 0.5 h. Then, p-methoxybenzyl chloride (27.5 g, 170 mmol) was added, and the mixture was slowly heated to 20 °C and stirred under nitrogen protection for 7.5 h. The reaction solution was slowly added to 100 mL of saturated ammonium chloride and extracted with MTBE (100 mL × 2). The combined organic phases were washed with 100 mL of saturated brine, dried over anhydrous sodium sulfate, filtered, and the organic solvent was removed under reduced pressure. The crude product was purified by silica gel column chromatography to obtain the compound **E1-2.** LC-MS(ESI): m/z = 430.1[M+H]⁺.

### Step B

Compound **E1-2** (34.6 g, 80.7 mmol) was reacted with toluene (150 mL), dichlorobis[di-tert-butyl-(4-dimethylaminophenyl)phosphine]palladium (0.286 g, 0.404 mmol), and tributyl(1-propynyl)tin (26.6 g, 80.7 mmol) at 120°C for 2 hours under nitrogen protection. The reaction solution was concentrated under reduced pressure and separated by column chromatography to obtain the compound **E1-3.** LC-MS(ESI): m/z = 390[M+H]⁺.

### Step C

2,2,6,6-Tetramethylpiperidine (22.3 g, 156 mmol) was added to anhydrous THF(300 mL), the mixture was cooled to -5 °C, and n-butyllithium (2.5 M, 50 mL) was added dropwise. The reaction was carried out at -5 to 0 °C for 15 minutes. The mixture was then cooled to -60 °C, and a THF(100 mL) solution of compound **E1-3** (24.27 g, 62.4 mmol) was added. The reaction was carried out at -60 °C for 0.5 hours. N,N-Dimethylformamide (22.8 g, 0.312 mol) was quickly added, and the reaction mixture was stirred at -60 °C for 10 minutes. 200 mL of saturated ammonium chloride was added to the reaction mixture, and the mixture was extracted with MTBE (100 mL × 2). The combined organic phases were then extracted with 100 mL of MTBE. Wash with mL of saturated saline solution, dry with anhydrous sodium sulfate, filter, remove organic solvent under reduced pressure, and the crude product is slurried with solvent for 0.5 hours, filtered, the filter cake is dried, the filtrate is concentrated, and then purified by silica gel column chromatography. The filter cake and column chromatography residues are combined to obtain the compound **E1-4.** LC-MS(ESI): m/z = 418.1[M+H]⁺.

### Step D

Compound **E1-4** (10.43 g, 25 mmol) was added to 100 mL of N,N-dimethylformamide, followed by bromosuccinimide (4.5 g, 25 mmol). The reaction mixture was stirred at 20 °C for 20 minutes. The reaction mixture was then added to 150 mL of water and extracted with MTBE (80 mL × 2). The combined organic phases were washed with saturated brine (50 mL × 2), dried over anhydrous sodium sulfate, filtered, and the organic solvent was removed under reduced pressure. The crude product was then slurried with a mixed solvent for 0.5 hours, filtered, and the filter cake was dried to obtain the compound **E1-5.** LC-MS(ESI) : m/z = 496.0[M+H]⁺.

### Step E

Compound **E1-5** (9.7 g, 20.4 mmol) was added to N,N-dimethylformamide (95 mL). Under nitrogen atmosphere, cuprous iodide (7.8 g, 4.1 mmol) and methyl fluorosulfonyl difluoroacetate (20 g, 100 mmol) were added to the reaction mixture. The mixture was heated to 100 °C and stirred for 1 hour. After cooling, the reaction mixture was filtered through diatomaceous earth. The filtrate was added to 150 mL of water and extracted with MTBE (75 mL × 2). The combined organic phases were washed with saturated brine (100 mL × 2), dried over anhydrous sodium sulfate, filtered, and the organic solvent was removed under reduced pressure. The crude product was purified by silica gel column chromatography to obtain the compound **E1-6.** LC-MS(ESI): m/z = 496.2[M+H]⁺.

### Step E

Under nitrogen protection, **E1-5** (6.44 g, 13 mmol) and lithium hydroxide (0.82 g, 19.5 mmol) were dissolved in 100 mL of THF at room temperature. Then, dimethyl ethylphosphonoacetate (3.55 g, 19.5 mmol) was added. The reaction mixture was stirred at room temperature for 24 hours. The THF was recovered and added to 150 mL of water. The mixture was extracted with MTBE (75 mL × 2). The combined organic phases were washed with saturated brine (100 mL × 2), dried over anhydrous sodium sulfate, filtered, and the organic solvent was removed under reduced pressure. The crude product was purified by silica gel column chromatography to obtain the compound **E1-6.** LC-MS(ESI): m/z = 556.2[M+H]⁺.

### Step F

Compound **E1-6** (11.1 g, 20 mmol) and piperidine (0.2 mL) were added to dichloromethane (100 mL), followed by ethyl 3-mercaptopropionate (3.2 g, 24 mmol) and benzyltrimethylammonium hydroxide (1 mL, 30% methanol). The mixture was stirred at 60°C for 48 hours. The mixture was extracted from dichloromethane (75 mL × 2) with dilute hydrochloric acid. The combined organic phases were washed with saturated sodium bicarbonate (50 mL) and saturated brine (50 mL × 2), dried over anhydrous sodium sulfate, filtered, and the organic solvent was removed under reduced pressure. The crude product was purified by silica gel column chromatography to obtain the compound **E1-7.** LC-MS(ESI): m/z =690.3[M+H]⁺.

### Step G

Compound **E1-8** (7.97 g, 12 mmol) and sodium ethoxide (1.36 g, 19 mmol) were dissolved in 400 mL of toluene and reacted with stirring at room temperature for 24 hours. The mixture was poured into 100 mL of water and extracted with dichloromethane (100 mL × 2). The combined organic phases were washed with saturated brine (50 mL × 2), dried over anhydrous sodium sulfate, filtered, and the organic solvent was removed under reduced pressure. The crude product was purified by silica gel column chromatography to obtain the compound **E1-9.** LC-MS(ESI): m/z =619.7[M+H]⁺.

### Step H

S-methylisothiourea sulfate (1.7 g, 6.2 mmol) and DMF (1.6 g, 11.7 mmol) were added to a suspension of **E1-9** (3.28 g, 5.3 mmol) in water (10 mL) at room temperature. The reaction mixture was stirred for 20 hours. The precipitate was collected, washed twice with water and isopropyl ether, dried, and the crude product was purified by silica gel column chromatography to obtain the compound**E1-10.** LC-MS(ESI): m/z = 644.2[M+H]⁺.

### Step I

Compound **E1-10** (1.03 g, 1.6 mmol) was dissolved in dichloromethane (10 mL), and N,N-diisopropylethylamine (0.6 g, 4.8 mmol) was added. The mixture was cooled to 0-10°C, and trifluoromethanesulfonic anhydride (0.7 g, 2.4 mmol, 400 µL) was slowly added to the reaction solution. The reaction was carried out at this temperature for 15 minutes. The reaction solution was poured into a saturated ammonium chloride aqueous solution (8 mL), and the mixture was separated. The aqueous phase was extracted with dichloromethane (5 mL × 2). The combined organic phases were dried over anhydrous sodium sulfate, filtered, and the organic solvent was removed under reduced pressure. The crude product was purified by silica gel column chromatography to obtain the compound **E1.** LC-MS(ESI): m/z = 802.1[M+H]⁺.

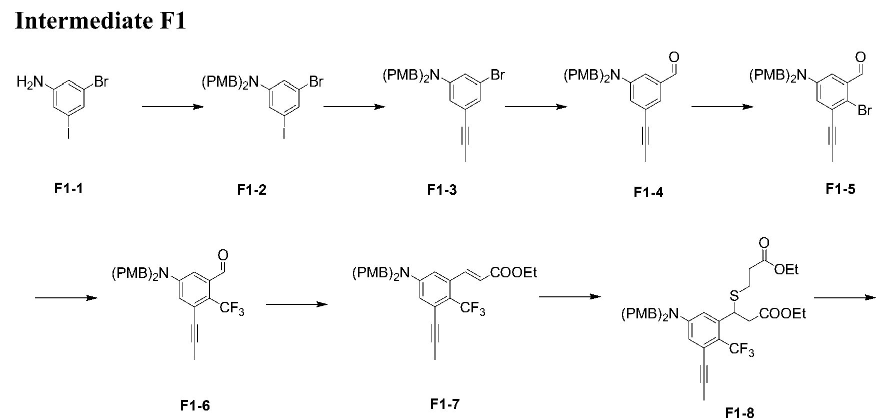

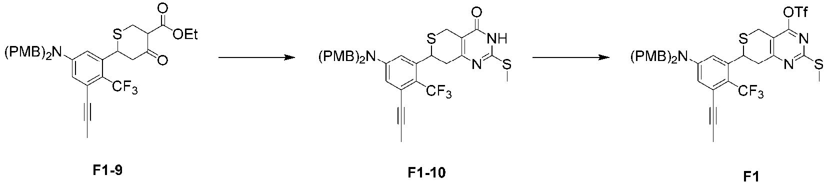

### Step A

In a dry 1L three-necked flask, K₂CO₃ (27.6 g, 200 mmol) was added to DMF (100 mL). The mixture was cooled to 0 °C, and under nitrogen protection, a solution of compound E1-1 (23.76 g, 80 mmol) in DMF (500 mL) was added dropwise. The reaction was continued at 0 °C for 0.5 h. Then, p-methoxybenzyl chloride (27.5 g, 170 mmol) was added, and the mixture was slowly heated to 20 °C and stirred under nitrogen protection for 7.5 h. The reaction solution was slowly added to 100 mL of saturated ammonium chloride and extracted with MTBE (100 mL × 2). The combined organic phases were washed with 100 mL of saturated brine, dried over anhydrous sodium sulfate, filtered, and the organic solvent was removed under reduced pressure. The crude product was purified by silica gel column chromatography to obtain the compound **F1-2.** LC-MS(ESI): m/z = 567.9[M+H]⁺.

### Step B

Compound **F1-2** (45.67 g, 80.7 mmol) was reacted with toluene (150 mL), dichlorobis[di-tert-butyl-(4-dimethylaminophenyl)phosphine]palladium (0.286 g, 0.404 mmol), and tributyl(1-propynyl)tin (26.6 g, 80.7 mmol) at 120°C for 2 hours under nitrogen protection. The reaction solution was concentrated under reduced pressure and separated by column chromatography to obtain the compound **F1-3.** LC-MS(ESI): m/z = 451[M+H]⁺.

### Step C

2,2,6,6-Tetramethylpiperidine (22.3 g, 156 mmol) was added to anhydrous THF(300 mL), and the mixture was cooled to -5 °C. Butyllithium (2.5 M, 50 mL) was added dropwise, and the reaction was carried out at -5 to 0 °C for 15 minutes. The mixture was then cooled to -60 °C, and a THF(100 mL) solution of compound **E1-3** (28.08 g, 62.4 mmol) was added. The reaction was carried out at -60 °C for 0.5 hours. DMF (22.8 g, 0.312 mol) was quickly added, and the reaction mixture was stirred at -60 °C for 10 minutes. 200 mL of saturated ammonium chloride was added to the reaction mixture, and the mixture was extracted with MTBE (100 mL × 2). The combined organic phases were then extracted with 100 mL of MTBE. Wash with mL of saturated saline solution, dry with anhydrous sodium sulfate, filter, remove organic solvent under reduced pressure, and the crude product is slurried with solvent for 0.5 hours, filtered, the filter cake is dried, the filtrate is concentrated, and then purified by silica gel column chromatography. The filter cake and column chromatography residues are combined to obtain the compound **F1-4.** LC-MS(ESI) : m/z = 400.2[M+H]⁺.

### Step D

Compound **F1-4** (10.43 g, 25 mmol) was added to 100 mL of DMF, followed by NBS(4.5 g, 25 mmol). The reaction mixture was stirred at 20 °C for 20 minutes. The reaction mixture was then added to 150 mL of water and extracted with MTBE (80 mL × 2). The combined organic phases were washed with saturated brine (50 mL × 2), dried over anhydrous sodium sulfate, filtered, and the organic solvent was removed under reduced pressure. The crude product was then slurried with a mixed solvent for 0.5 hours, filtered, and the filter cake was dried to obtain the compound **F1-5.** LC-MS(ESI): m/z = 478.0[M+H]⁺.

### Step E

Compound **F1-5** (9.77 g, 20.4 mmol) was added to N,N-dimethylformamide (95 mL). Under nitrogen atmosphere, cuprous iodide (7.8 g, 4.1 mmol) and methyl fluorosulfonyl difluoroacetate (20 g, 100 mmol) were added to the reaction mixture. The mixture was heated to 100 °C and stirred for 1 hour. After cooling, the reaction mixture was filtered through diatomaceous earth. The filtrate was added to 150 mL of water and extracted with MTBE (75 mL × 2). The combined organic phases were washed with saturated brine (100 mL × 2), dried over anhydrous sodium sulfate, filtered, and the organic solvent was removed under reduced pressure. The crude product was purified by silica gel column chromatography to obtain the compound **F1-6.** LC-MS(ESI): m/z = 468.1[M+H]⁺.

### Step E

Under nitrogen protection, **F1-6** (6.07 g, 13 mmol) and lithium hydroxide (0.82 g, 19.5 mmol) were dissolved in 100 mL of THF at room temperature. Then, dimethyl ethylphosphonoacetate (3.55 g, 19.5 mmol) was added. The reaction mixture was stirred at room temperature for 24 hours. The THF was recovered and added to 150 mL of water. The mixture was extracted with MTBE (75 mL × 2). The combined organic phases were washed with saturated brine (100 mL × 2), dried over anhydrous sodium sulfate, filtered, and the organic solvent was removed under reduced pressure. The crude product was purified by silica gel column chromatography to obtain the compound **F1-7**. LC-MS(ESI): m/z = 538.2[M+H]⁺.

### Step F

Compound **F1-7** (10.7 g, 20 mmol) and piperidine (0.2 mL) were added to dichloromethane (100 mL), followed by ethyl 3-mercaptopropionate (3.2 g, 24 mmol) and benzyltrimethylammonium hydroxide (1 mL, 30% methanol). The mixture was stirred at 60°C for 48 hours. The mixture was extracted from dichloromethane (75 mL × 2) with dilute hydrochloric acid. The combined organic phases were washed with saturated sodium bicarbonate (50 mL) and saturated brine (50 mL × 2), dried over anhydrous sodium sulfate, filtered, and the organic solvent was removed under reduced pressure. The crude product was purified by silica gel column chromatography to obtain the compound **F1-8.** LC-MS(ESI): m/z =670.1[M+H]⁺.

### Step G

Compound **F1-8** (8.39 g, 12 mmol) and sodium ethoxide (1.36 g, 19 mmol) were dissolved in 400 mL of toluene and reacted with stirring at room temperature for 24 hours. The mixture was poured into 100 mL of water and extracted with dichloromethane (100 mL × 2). The combined organic phases were washed with saturated brine (50 mL × 2), dried over anhydrous sodium sulfate, filtered, and the organic solvent was removed under reduced pressure. The crude product was purified by silica gel column chromatography to obtain the compound **F1-9.** LC-MS(ESI): m/z =625.2[M+H]⁺.

### Step H

S-methylisothiourea sulfate (1.7 g, 6.2 mmol) and DMF (1.6 g, 11.7 mmol) were added to a suspension of **E1-9** (3.45 g, 5.3 mmol) in water (10 mL) at room temperature. The reaction mixture was stirred for 20 hours. The precipitate was collected, washed twice with water and isopropyl ether, dried, and the crude product was purified by silica gel column chromatography to obtain the compound **F1-10.** LC-MS(ESI): m/z = 652.2[M+H]⁺.

### Step I

Compound **F1-10** (1.04 g, 1.6 mmol) was dissolved in dichloromethane (10 mL), and N,N-diisopropylethylamine (0.6 g, 4.8 mmol) was added. The mixture was cooled to 0-10°C, and trifluoromethanesulfonic anhydride (0.7 g, 2.4 mmol, 400 µL) was slowly added to the reaction solution. The reaction was carried out at this temperature for 15 minutes. The reaction solution was poured into a saturated ammonium chloride aqueous solution (8 mL), and the mixture was separated. The aqueous phase was extracted with dichloromethane (5 mL × 2). The combined organic phases were dried over anhydrous sodium sulfate, filtered, and the organic solvent was removed under reduced pressure. The crude product was purified by silica gel column chromatography to obtain the compound **F1.** LC-MS(ESI): m/z = 784.1[M+H]⁺.

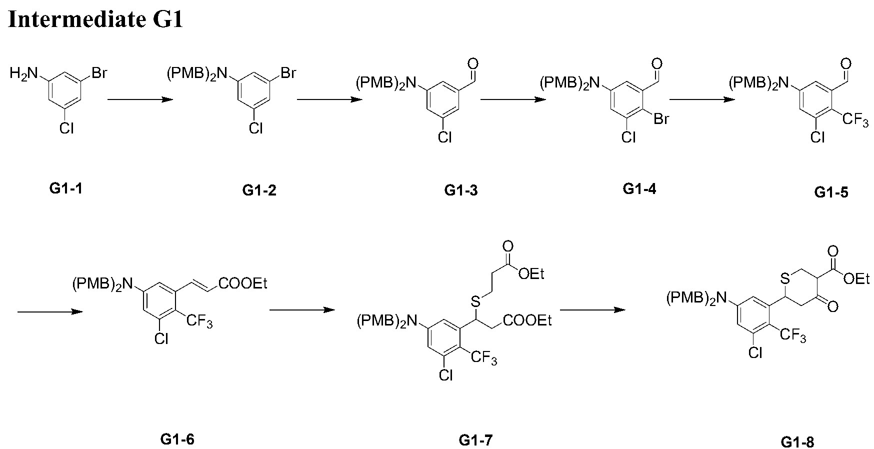

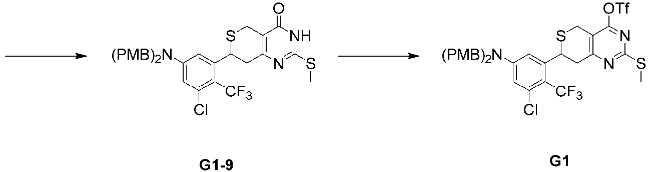

### Step A

In a dry 1L three-necked flask, DMF (27.6 g, 200 mmol) was added to N,N-dimethylformamide (100 mL). The mixture was cooled to 0 °C, and under nitrogen protection, a solution of compound **G1-1** (16.4 g, 80 mmol) in N,N-dimethylformamide (500 mL) was added dropwise. The reaction was continued at 0 °C for 0.5 h. Then, p-methoxybenzyl chloride (27.5 g, 170 mmol) was added, and the mixture was slowly heated to 20 °C and stirred under nitrogen protection for 7.5 h. The reaction solution was slowly added to 100 mL of saturated ammonium chloride and extracted with MTBE (100 mL × 2). The combined organic phases were washed with 100 mL of saturated brine, dried over anhydrous sodium sulfate, filtered, and the organic solvent was removed under reduced pressure. The crude product was purified by silica gel column chromatography to obtain the compound **G1-2.** LC-MS(ESI): m/z = 467.0[M+H]⁺.

### Step B

2,2,6,6-Tetramethylpiperidine (22.3 g, 156 mmol) was added to anhydrous THF(300 mL), and the mixture was cooled to -5 °C. Butyllithium (2.5 M, 50 mL) was added dropwise, and the reaction was carried out at -5 to 0 °C for 15 minutes. The mixture was then cooled to -60 °C, and a THFsolution (100 mL) of compound **G1-2** (29.08 g, 62.4 mmol) was added. The reaction was carried out at -60 °C for 0.5 hours. N,N-Dimethylformamide (22.8 g, 0.312 mol) was quickly added, and the reaction mixture was stirred at -60 °C for 10 minutes. 200 mL of saturated ammonium chloride was added to the reaction mixture, and the mixture was extracted with MTBE (100 mL × 2). The combined organic phases were then extracted with 100 mL of MTBE. Wash with mL of saturated saline solution, dry with anhydrous sodium sulfate, filter, remove organic solvent under reduced pressure, and the crude product is slurried with solvent for 0.5 hours, filtered, the filter cake is dried, the filtrate is concentrated, and then purified by silica gel column chromatography. The filter cake and column chromatography residues are combined to obtain the compound **G1-3.** LC-MS(ESI): m/z = 396.1[M+H]⁺.

### Step C

Compound **G1-3** (9.88 g, 25 mmol) was added to 100 mL of N,N-dimethylformamide, followed by bromosuccinimide (4.5 g, 25 mmol). The reaction mixture was stirred at 20 °C for 20 minutes. The reaction mixture was then added to 150 mL of water and extracted with MTBE (80 mL × 2). The combined organic phases were washed with saturated brine (50 mL × 2), dried over anhydrous sodium sulfate, filtered, and the organic solvent was removed under reduced pressure. The crude product was then slurried with a mixed solvent for 0.5 hours, filtered, and the filter cake was dried to obtain the compound **G1-4.** LC-MS(ESI) : m/z = 474.0[M+H]⁺.

### Step D

Compound G1-4 (9.65 g, 20.4 mmol) was added to N,N-dimethylformamide (95 mL). Under nitrogen atmosphere, cuprous iodide (7.8 g, 4.1 mmol) and methyl fluorosulfonyl difluoroacetate (20 g, 100 mmol) were added to the reaction mixture. The mixture was heated to 100 °C and stirred for 1 hour. After cooling, the reaction mixture was filtered through diatomaceous earth. The filtrate was added to 150 mL of water and extracted with MTBE (75 mL × 2). The combined organic phases were washed with saturated brine (100 mL × 2), dried over anhydrous sodium sulfate, filtered, and the organic solvent was removed under reduced pressure. The crude product was purified by silica gel column chromatography to obtain the compound **G1-5.** LC-MS(ESI): m/z = 464.1[M+H]⁺.

### Step E

Under nitrogen protection, G1-5 (6.02 g, 13 mmol) and lithium hydroxide (0.82 g, 19.5 mmol) were dissolved in 100 mL of THF at room temperature. Then, dimethyl ethylphosphonoacetate (3.55 g, 19.5 mmol) was added. The reaction mixture was stirred at room temperature for 24 hours. The THF was recovered and added to 150 mL of water. The mixture was extracted with MTBE (75 mL × 2). The combined organic phases were washed with saturated brine (100 mL × 2), dried over anhydrous sodium sulfate, filtered, and the organic solvent was removed under reduced pressure. The crude product was purified by silica gel column chromatography to obtain the compound G1-6. LC-MS(ESI): m/z = 534.1[M+H]⁺.

### Step F

Compound **G1-6** (10.6 g, 20 mmol) and piperidine (0.2 mL) were added to dichloromethane (100 mL), followed by ethyl 3-mercaptopropionate (3.2 g, 24 mmol) and benzyltrimethylammonium hydroxide (1 mL, 30% methanol). The mixture was stirred at 60°C for 48 hours. The mixture was extracted from dichloromethane (75 mL × 2) with dilute hydrochloric acid. The combined organic phases were washed with saturated sodium bicarbonate (50 mL) and saturated brine (50 mL × 2), dried over anhydrous sodium sulfate, filtered, and the organic solvent was removed under reduced pressure. The crude product was purified by silica gel column chromatography to obtain the compound **G1-7.** LC-MS(ESI): m/z =669.2[M+H]⁺.

### Step G

Compound **G1-7** (8.02 g, 12 mmol) and sodium ethoxide (1.36 g, 19 mmol) were dissolved in 400 mL of toluene and reacted with stirring at room temperature for 24 hours. The mixture was poured into 100 mL of water and extracted with dichloromethane (100 mL × 2). The combined organic phases were washed with saturated brine (50 mL × 2), dried over anhydrous sodium sulfate, filtered, and the organic solvent was removed under reduced pressure. The crude product was purified by silica gel column chromatography to obtain the compound **G1-8**. LC-MS(ESI): m/z =622.1[M+H]⁺.

### Step H

To a suspension of **G1-8** (3.29 g, 5.3 mmol) in water (10 mL), S-methylisothiourea sulfate (1.7 g, 6.2 mmol) and DMF (1.6 g, 11.7 mmol) were added at room temperature. The reaction mixture was stirred for 20 hours. The precipitate was collected, washed twice with water and isopropyl ether, dried, and the crude product was purified by silica gel column chromatography to obtain the compound **G1-9.** LC-MS(ESI): m/z = 648.2[M+H]⁺.

### Step I

Compound **G1-9** (1.04 g, 1.6 mmol) was dissolved in dichloromethane (10 mL), and N,N-diisopropylethylamine (0.6 g, 4.8 mmol) was added. The mixture was cooled to 0-10°C, and trifluoromethanesulfonic anhydride (0.7 g, 2.4 mmol, 400 µL) was slowly added to the reaction solution. The reaction was carried out at this temperature for 15 minutes. The reaction solution was poured into a saturated ammonium chloride aqueous solution (8 mL), and the mixture was separated. The aqueous phase was extracted with dichloromethane (5 mL × 2). The combined organic phases were dried over anhydrous sodium sulfate, filtered, and the organic solvent was removed under reduced pressure. The crude product was purified by silica gel column chromatography to obtain the compound **G1.** LC-MS(ESI): m/z = 780.1[M+H]⁺.

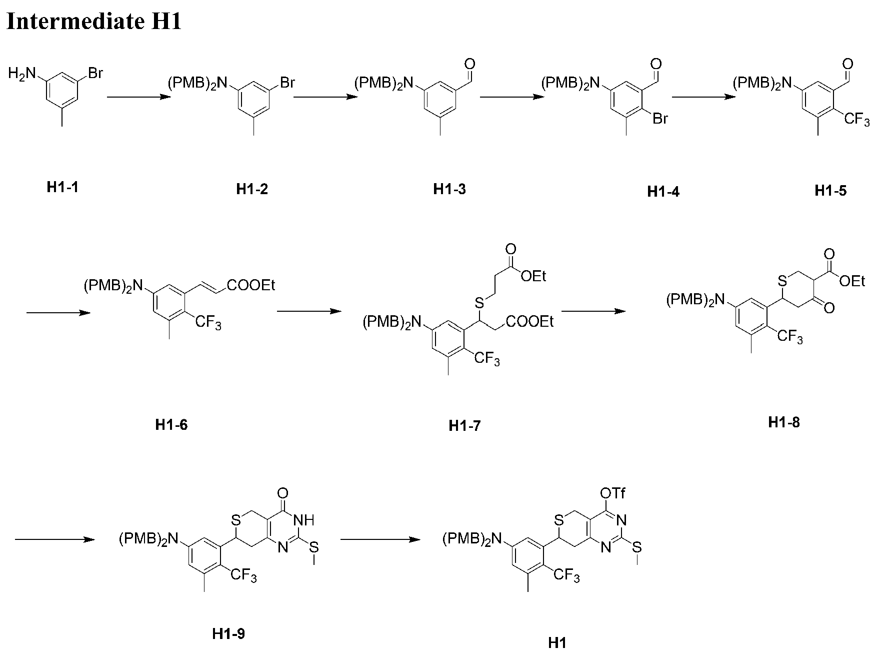

### Step A

In a dry 1L three-necked flask, DMF (27.6 g, 200 mmol) was added to DMF (100 mL). The mixture was cooled to 0 °C, and under nitrogen protection, a solution of compound **H1-1** (14.88 g, 80 mmol) in DMF (500 mL) was added dropwise. The reaction was continued at 0 °C for 0.5 h. Then, p-methoxybenzyl chloride (27.5 g, 170 mmol) was added, and the mixture was slowly heated to 20 °C and stirred under nitrogen protection for 7.5 h. The reaction solution was slowly added to 100 mL of saturated ammonium chloride and extracted with methyl MTBE (100 mL × 2). The combined organic phases were washed with 100 mL of saturated brine, dried over anhydrous sodium sulfate, filtered, and the organic solvent was removed under reduced pressure. The crude product was purified by silica gel column chromatography to obtain the compound **H1-2.** LC-MS(ESI): m/z = 426.1[M+H]⁺.

### Step B

2,2,6,6-Tetramethylpiperidine (22.3 g, 156 mmol) was added to anhydrous THF(300 mL), and the mixture was cooled to -5 °C. Butyllithium (2.5 M, 50 mL) was added dropwise, and the reaction was carried out at -5-0 °C for 15 minutes. The mixture was then cooled to -60 °C, and a THF(100 mL) solution of compound **H1-2** (26.52 g, 62.4 mmol) was added. The reaction was carried out at -60 °C for 0.5 hours. DMF (22.8 g, 0.312 mol) was rapidly added, and the reaction mixture was stirred at -60 °C for 10 minutes. 200 mL of saturated ammonium chloride was added to the reaction mixture, and the mixture was extracted with MTBE (100 mL × 2). The combined organic phases were then extracted with 100 mL of MTBE . Wash with mL of saturated saline solution, dry with anhydrous sodium sulfate, filter, remove organic solvent under reduced pressure, and the crude product is slurried with solvent for 0.5 hours, filtered, the filter cake is dried, the filtrate is concentrated, and then purified by silica gel column chromatography. The filter cake and column chromatography residues are combined to obtain the compound **H1-3.** LC-MS(ESI) : m/z = 376.2[M+H]⁺.

### Step C

Compound **H1-3** (9.34 g, 25 mmol) was added to 100 mL of DMF, followed by NBS (4.5 g, 25 mmol). The reaction mixture was stirred at 20 °C for 20 minutes. The reaction mixture was then added to 150 mL of water and extracted with MTBE (80 mL × 2). The combined organic phases were washed with saturated brine (50 mL × 2), dried over anhydrous sodium sulfate, filtered, and the organic solvent was removed under reduced pressure. The crude product was then slurried with a mixed solvent for 0.5 hours, filtered, and the filter cake was dried to obtain the compound **H1-4.** LC-MS(ESI): m/z = 454.0[M+H]⁺.

### Step D

Compound **H1-4** (9.24 g, 20.4 mmol) was added to DMF (95 mL). Under nitrogen atmosphere, cuprous iodide (7.8 g, 4.1 mmol) and methyl fluorosulfonyl difluoroacetate (20 g, 100 mmol) were added to the reaction mixture. The mixture was heated to 100 °C and stirred for 1 hour. After cooling, the reaction mixture was filtered through diatomaceous earth. The filtrate was added to 150 mL of water and extracted with MTBE (75 mL × 2). The combined organic phases were washed with saturated brine (100 mL × 2), dried over anhydrous sodium sulfate, filtered, and the organic solvent was removed under reduced pressure. The crude product was purified by silica gel column chromatography to obtain the compound**H1-5.** LC-MS(ESI): m/z = 444.1[M+H]⁺.

### Step E

Under nitrogen protection, **H1-5** (5.76 g, 13 mmol) and lithium hydroxide (0.82 g, 19.5 mmol) were dissolved in 100 mL of THF at room temperature. Then, dimethyl ethylphosphonoacetate (3.55 g, 19.5 mmol) was added. The reaction mixture was stirred at room temperature for 24 hours. The THF was recovered and added to 150 mL of water. The mixture was extracted with MTBE (75 mL × 2). The combined organic phases were washed with saturated brine (100 mL × 2), dried over anhydrous sodium sulfate, filtered, and the organic solvent was removed under reduced pressure. The crude product was purified by silica gel column chromatography to obtain the compound **H1-6.** LC-MS(ESI): m/z = 514.1[M+H]⁺.

### Step F

Compound **H1-6** (10.26 g, 20 mmol) and piperidine (0.2 mL) were added to dichloromethane (100 mL), followed by ethyl 3-mercaptopropionate (3.2 g, 24 mmol) and benzyltrimethylammonium hydroxide (1 mL, 30% methanol). The mixture was stirred at 60°C for 48 hours. The mixture was extracted from dichloromethane (75 mL × 2) with dilute hydrochloric acid. The combined organic phases were washed with saturated sodium bicarbonate (50 mL) and saturated brine (50 mL × 2), dried over anhydrous sodium sulfate, filtered, and the organic solvent was removed under reduced pressure. The crude product was purified by silica gel column chromatography to obtain the compound **H1-7.** LC-MS(ESI): m/z =648.3[M+H]⁺.

### Step G

Compound **H1-7** (7.76 g, 12 mmol) and sodium ethoxide (1.36 g, 19 mmol) were dissolved in 400 mL of toluene and reacted with stirring at room temperature for 24 hours. The mixture was poured into 100 mL of water and extracted with dichloromethane (100 mL × 2). The combined organic phases were washed with saturated brine (50 mL × 2), dried over anhydrous sodium sulfate, filtered, and the organic solvent was removed under reduced pressure. The crude product was purified by silica gel column chromatography to obtain the compound **H1-8.** LC-MS(ESI): m/z =602.2[M+H]⁺.

### Step H

S-methylisothiourea sulfate (1.7 g, 6.2 mmol) and DMF (1.6 g, 11.7 mmol) were added to a suspension of **H1-8** (3.19 g, 5.3 mmol) in water (10 mL) at room temperature. The reaction mixture was stirred for 20 hours. The precipitate was collected, washed twice with water and isopropyl ether, dried, and the crude product was purified by silica gel column chromatography to obtain the compound **H1-9.** LC-MS(ESI): m/z = 628.2[M+H]⁺.

### Step I

Compound **H1-9** (1.00 g, 1.6 mmol) was dissolved in dichloromethane (10 mL), and N,N-diisopropylethylamine (0.6 g, 4.8 mmol) was added. The mixture was cooled to 0-10°C, and trifluoromethanesulfonic anhydride (0.7 g, 2.4 mmol, 400 µL) was slowly added to the reaction solution. The reaction was carried out at this temperature for 15 minutes. The reaction solution was poured into a saturated ammonium chloride aqueous solution (8 mL), and the mixture was separated. The aqueous phase was extracted with dichloromethane (5 mL × 2). The combined organic phases were dried over anhydrous sodium sulfate, filtered, and the organic solvent was removed under reduced pressure. The crude product was purified by silica gel column chromatography to obtain the compound **H1.** LC-MS(ESI): m/z = 760.1[M+H]⁺.

### Example 1

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (1)

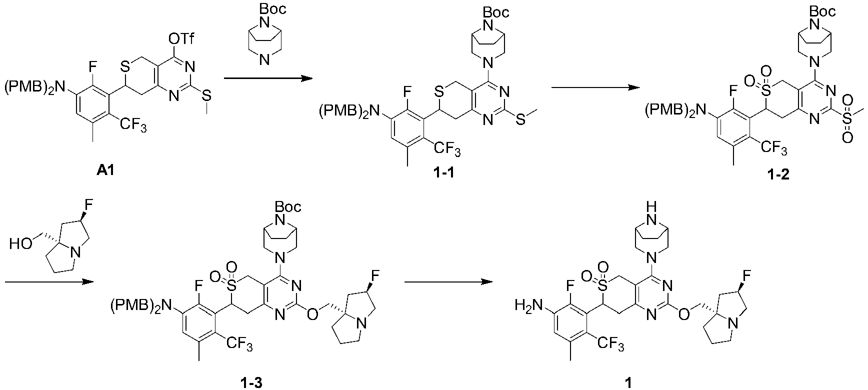

### Step A

Compound **A1** (82 mg, 105.02 µmol) and 8-tert-butoxycarbonyl-3,8-diazabicyclo[3.2.1]octane (26.63 mg, 126.03 µmol) were dissolved in N,N-dimethylformamide (2 mL), and diisopropylethylamine (40.72 mg, 315.07 µmol) was added. The reaction mixture was heated to 100°C and stirred for 1 hour. After cooling, the organic solvent was removed under reduced pressure. The crude product was purified by thin-layer chromatography to obtain the compound **1-1.** LC/MS(ESI): m/z = 840[M+H]⁺.

### Step B

Compound **1-1** (71 mg, 84.96 µmol) was dissolved in dichloromethane (2 mL), and m-chloroperoxybenzoic acid (34.50 mg, 169.92 µmol) was added. The reaction mixture was stirred at 20 °C for 3 hours. The organic solvent was removed under reduced pressure, and the crude product was purified by thin-layer chromatography to obtain compound **1-2.** LC/MS(ESI): m/z =904[M+H]⁺.

### Step C

Under ice-water bath conditions, compound ((2R,7AS)-2-fluorotetrahydro-1H-pyrrolazin-7a(5H)-yl)methanol (12.09 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **1-2** (53 mg, 58.42 µmol) was added. The reaction mixture was stirred for 2 hours under ice-water bath conditions. The organic solvent was removed under reduced pressure, and the crude product was purified by thin-layer chromatography to obtain compound **1-3.** LC/MS(ESI): m/z =983.1[M+H]⁺.

### Step D

Compounds **1-3** (42 mg, 42.78 µmol) were dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain the compound **1.** LC/MS(ESI): m/z =642.7[M+H]⁺. ¹H-NMR (DMSO-*d*₆) *δ* 1.78-2.39 (m, 12H), 2.38-2.79 (m, 3H), 3.13-3.44 (m, 3H), 3.56-3.62 (m, 2H), 3.71-4.43 (m, 12H), 5.96 (s, 2H), 6.84 (d, 1H).

### Example 2

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (1A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (1B)

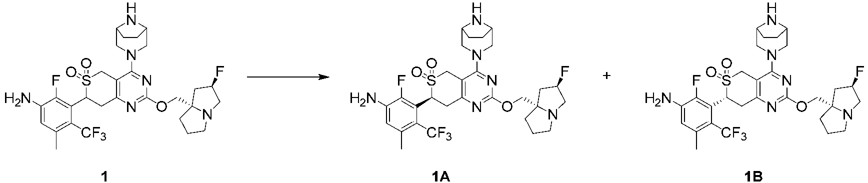

Compound **1** was purified by supercritical liquid chromatography (SFC) to obtain **1A** and **1B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800 LC/MS(ESI): m/z =642.7[M+H]⁺).

### Example 3

### 4-((1R,5S)-3,8-diazabicyclo[3.2.1]octane-3-yl)-7-(2-fluoro-3-hydroxy-5-methyl-6-(tri fluoromethyl)phenyl)-2-(((2R,7AS)-2-fluorotetrahydro-1H-pyrrolazin-7a(5H)-yl)met hoxy)-7,8-dihydro-5H-thiopyrano[4,3-d]pyrimidine-6,6-dioxide (2)

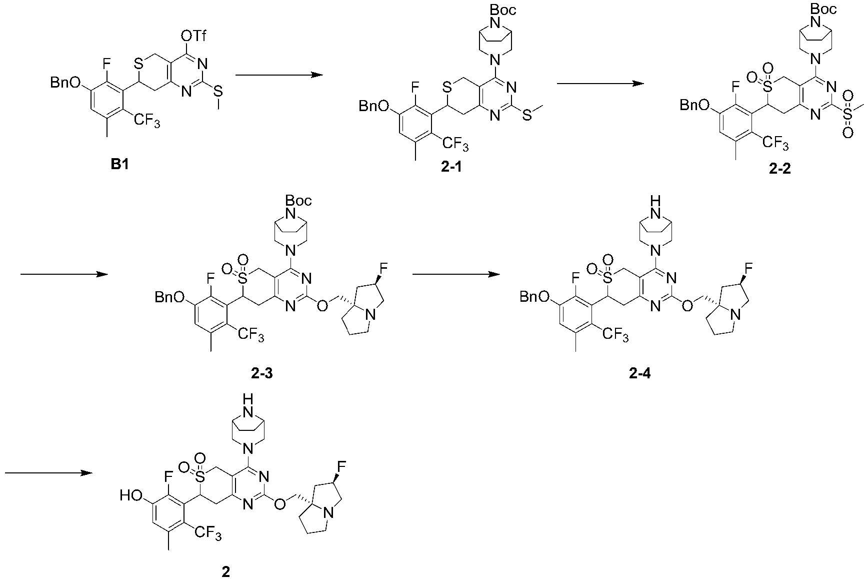

Compound **2-4** was prepared using a similar method to that used for compound 1 in **Example 1** (with **B1** as the starting material). LC/MS(ESI): m/z =733.8[M+H]⁺.

Compound **2-4** (0.1 g, 1.4 mmol) was added to a 1:1 IPA:THF (25 mL) solution and stirred. 10% Pd/C (0.1 g) was then added to the stirred solution. The reaction mixture was stirred at room temperature under hydrogen atmosphere for 16 h, filtered, concentrated under reduced pressure, and purified by silica gel column chromatography to obtain the compound 2. LC/MS(ESI): m/z =643.7[M+H]⁺.

### Example 4

### (7S)-4-((1R,5S)-3,8-diazabicyclo[3.2.1]octane-3-yl)-7-(2-fluoro-3-hydroxy-5-methyl-6-(trifluoromethyl)phenyl)-2-(((2R,7AS)-2-fluorotetrahydro-1H-pyrrolazin-7a(5H)-yl) methoxy)-7,8-dihydro-5H-thiopyrano[4,3-d]pyrimidine-6,6-dioxide (2A)

### (7R)-4-((1R,5S)-3,8-diazabicyclo[3.2.1]octane-3-yl)-7-(2-fluoro-3-hydroxy-5-methyl-6-(trifluoromethyl)phenyl)-2-(((2R,7AS)-2-fluorotetrahydro-1H-pyrrolazin-7a(5H)-yl) methoxy)-7,8-dihydro-5H-thiopyrano[4,3-d]pyrimidine-6,6-dioxide (2B)

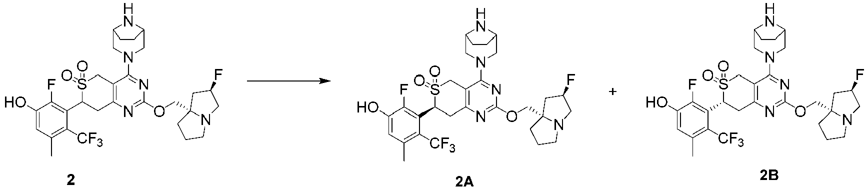

Compounds 2A and 2B were obtained using a similar preparation method to that used for compounds 1A and 1B in **Example** 2 (with the starting material replaced by compound 2). LC/MS(ESI): m/z =643.7[M+H]⁺.

### Example 5

### 7-(6-amino-4-methyl-3-(trifluoromethyl)pyridin-2-yl)-4-((1R,5S)-3,8-diazabicyclo[3. 2.1]octane-3-yl)-2-(((2R,7AS)-2-fluorotetrahydro-1H-pyrrolazin-7a(5H)-yl)methoxy) -7,8-dihydro-5H-thiopyrano[4,3-d]pyrimidine-6,6-dioxide (3)

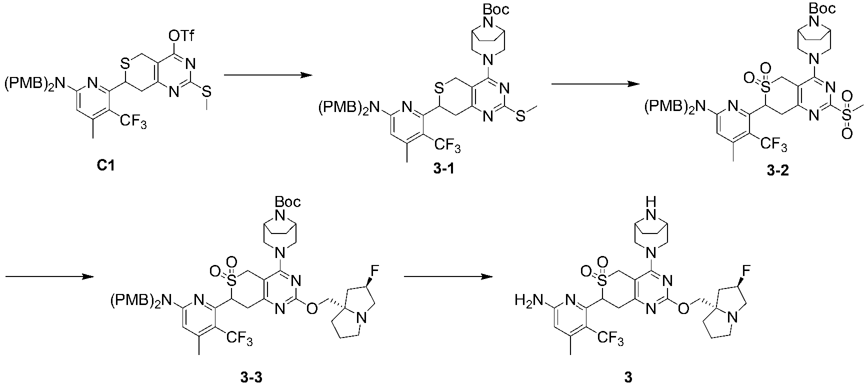

Compound **3** was prepared using a similar method to that used for **compound 1** in **Example** 1(with **C1** as the starting material). LC/MS(ESI): m/z =625.7[M+H]⁺.¹H-NMR (CDCl₃) *δ* 1.78-2.20 (m, 7H), 2.01-2.20 (m, 6H), 2.25-2.60 (m, 2H), 3.00-3.20 (m, 3H), 3.30-3.50 (m, 1H), 3.60-3.80 (m, 2H), 3.80-4.15 (m, 4H), 4.30-4.98 (m, 7H), 7.30 (s, 1H).

### Example 6

### (7S)-7-(6-amino-4-methyl-3-(trifluoromethyl)pyridin-2-yl)-4-((1R,5S)-3,8-diazabicyc lo[3.2.1]octane-3-yl)-2-(((2R,7AS)-2-fluorotetrahydro-1H-pyrrolazin-7a(5H)-yl)meth oxy)-7,8-dihydro-5H-thiopyrano[4,3-d]pyrimidine 6,6-Dioxide (3A)

### (7R)-7-(6-amino-4-methyl-3-(trifluoromethyl)pyridin-2-yl)-4-((1R,5S)-3,8-diazabicyc lo[3.2.1]octane-3-yl)-2-(((2R,7AS)-2-fluorotetrahydro-1H-pyrrolazin-7a(5H)-yl)meth oxy)-7,8-dihydro-5H-thiopyrano[4,3-d]pyrimidine 6,6-Dioxide (3B)

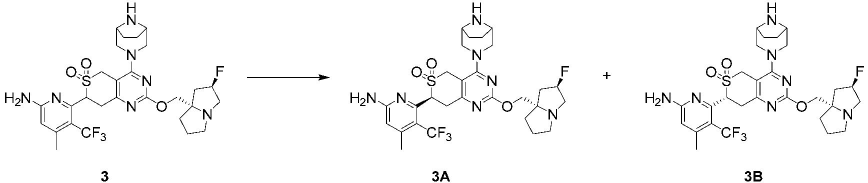

Compounds **3A** and **3B** were obtained using a similar preparation method to that used for compounds **1A** and **1B** in **Example 2** (with the starting material replaced by compound **3).** LC/MS(ESI): m/z =625.7[M+H]⁺.

### Example 7

### 4-((1R,5S)-3,8-diazabicyclo[3.2.1]octane-3-yl)-2-(((2R,7AS)-2-fluorotetrahydro-1H-pyrrolazin-7a(5H)-yl)methoxy)-7-(6-hydroxy-4-methyl-3-(trifluoromethyl)pyridin-2-yl)-7,8-dihydro-5H-thiopyrano[4,3-d]pyrimidine-6,6-dioxide (4)

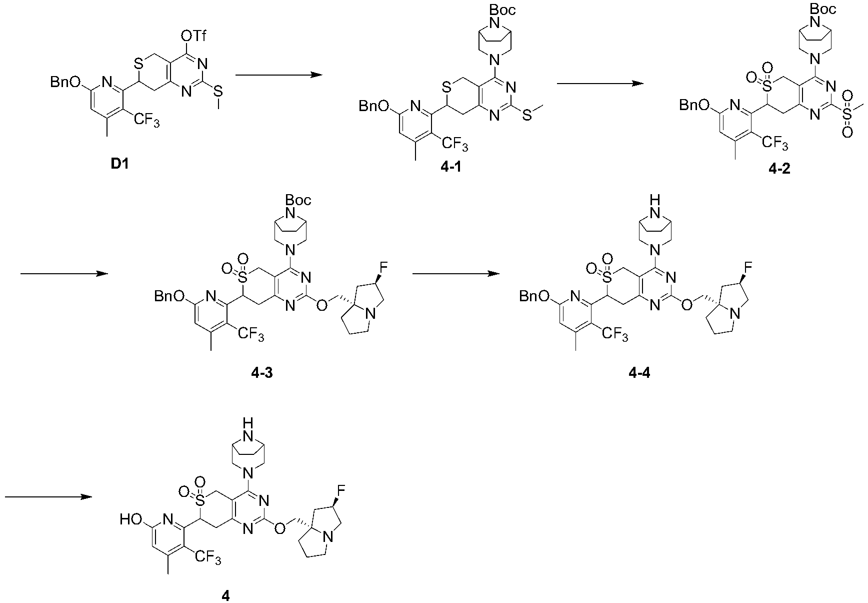

Compound **4** was prepared using a similar method to that used for compound 2 in **Example 3** (with **D1** as the starting material). LC/MS(ESI): m/z =626.7[M+H]⁺.

### Example 8

### (7S)-4-((1R,5S)-3,8-diazabicyclo[3.2.1]octane-3-yl)-2-(((2R,7AS)-2-fluorotetrahydro -1H-pyrrolazin-7a(5H)-yl)methoxy)-7-(6-hydroxy-4-methyl-3-(trifluoromethyl)pyridi n-2-yl)-7,8-dihydro-5H-thiopyrano[4,3-d]pyrimidine-6,6-dioxide (4A)

### (7R)-4-((1R,5S)-3,8-diazabicyclo[3.2.1]octane-3-yl)-2-(((2R,7AS)-2-fluorotetrahydro -1H-pyrrolazin-7a(5H)-yl)methoxy)-7-(6-hydroxy-4-methyl-3-(trifluoromethyl)pyridi n-2-yl)-7,8-dihydro-5H-thiopyrano[4,3-d]pyrimidine-6,6-dioxide (4B)

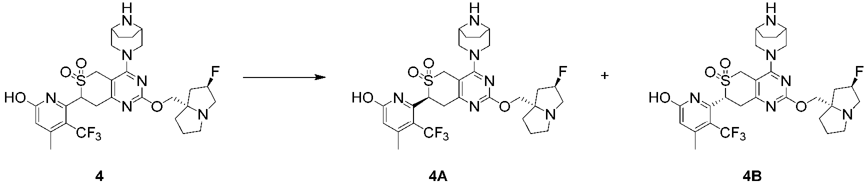

Compounds **4A** and **4B** were prepared using a similar method to that used for compounds **1A** and **1B** in **Example 2** (with compound **4** as the starting material).LC/MS(ESI): m/z =626.7[M+H]⁺.

### Example 9

### 7-(3-amino-2-fluoro-5-methyl-6-(trifluoromethyl)phenyl)-2-(((2R,7AS)-2-fluorotetra hydro-1H-pyrrolazin-7a(5H)-yl)methoxy)-4-((R)-3-hydroxy-3-methylpiperidin-1-yl)-7,8-dihydro-5H-thiopyrano[4,3-d]pyrimidine 6,6-dioxide (5)

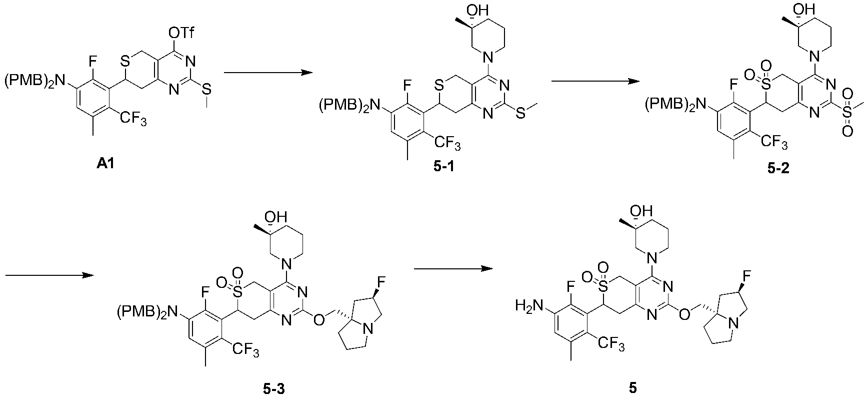

Compound **5** was prepared using a method similar to that used for compound 1 in **Example 1.** LC/MS(ESI): m/z =645.7[M+H]⁺.

### Example 10

### (7S)-7-(3-amino-2-fluoro-5-methyl-6-(trifluoromethyl)phenyl)-2-(((2R,7AS)-2-fluoro tetrahydro-1H-pyrrolazin-7a(5H)-yl)methoxy)-4-((R)-3-hydroxy-3-methylpiperidin-1-yl)-7,8-dihydro-5H-thiopyrano[4,3-d]pyrimidine-6,6-dioxide (5A)

### (7R)-7-(3-amino-2-fluoro-5-methyl-6-(trifluoromethyl)phenyl)-2-(((2R,7AS)-2-fluoro tetrahydro-1H-pyrrolazin-7a(5H)-yl)methoxy)-4-((R)-3-hydroxy-3-methylpiperidin-1-yl)-7,8-dihydro-5H-thiopyrano[4,3-d]pyrimidine-6,6-dioxide (5B)

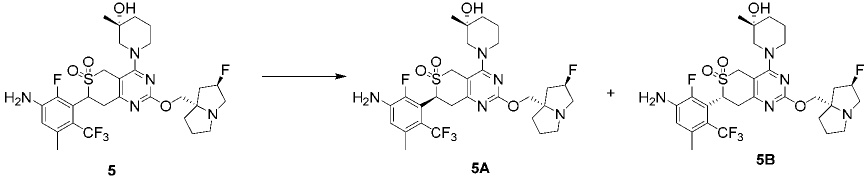

Compounds **5A** and **5B** were obtained using a similar preparation method to that used for compounds **1A** and **1B** in **Example 2** (with compound 5 as the starting material). LC/MS(ESI): m/z =645.7[M+H]⁺.

### Example 11

### 7-(2-fluoro-3-hydroxy-5-methyl-6-(trifluoromethyl)phenyl)-2-(((2R,7AS)-2-fluorotetr ahydro-1H-pyrrolazin-7a(5H)-yl)methoxy)-4-((R)-3-hydroxy-3-methylpiperidin-1-yl)

### -7,8-dihydro-5H-thiopyrano[4,3-d]pyrimidine 6,6-dioxide (6)

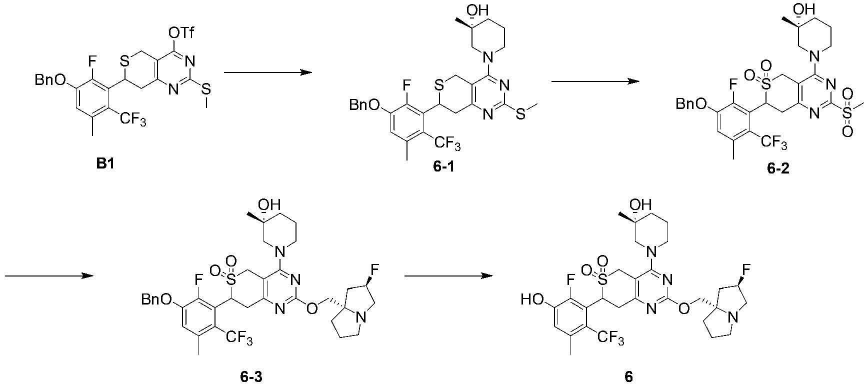

Compound **6** was prepared using a similar method to that used for compound 2 in **Example 3** (with **B1** as the starting material). LC/MS(ESI): m/z =646.7[M+H]⁺.

### Example 12

### (7S)-7-(2-fluoro-3-hydroxy-5-methyl-6-(trifluoromethyl)phenyl)-2-(((2R,7AS)-2-fluo rotetrahydro-1H-pyrrolazin-7a(5H)-yl)methoxy)-4-((R)-3-hydroxy-3-methylpiperidin-1-yl)-7,8-dihydro-5H-thiopyrano[4,3-d]pyrimidine-6,6-dioxide (6A)

### (7R)-7-(2-fluoro-3-hydroxy-5-methyl-6-(trifluoromethyl)phenyl)-2-(((2R,7AS)-2-fluo rotetrahydro-1H-pyrrolazin-7a(5H)-yl)methoxy)-4-((R)-3-hydroxy-3-methylpiperidin-1-yl)-7,8-dihydro-5H-thiopyrano[4,3-d]pyrimidine-6,6-dioxide (6B)

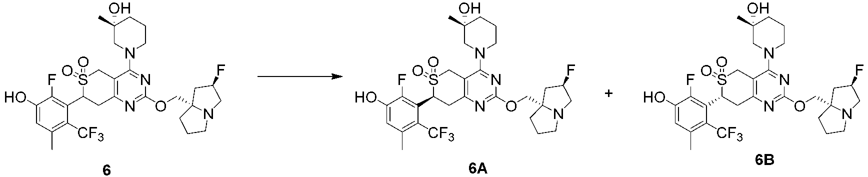

Compounds **6A** and **6B** were prepared using a similar method to that used for compounds **1A** and **1B** in **Example 2** (with compound **6** as the starting material). LC/MS(ESI): m/z =646.7[M+H]⁺.

### Example 13

### 7-(6-amino-4-methyl-3-(trifluoromethyl)pyridin-2-yl)-2-(((2R,7AS)-2-fluorotetrahydr o-1H-pyrrolazin-7a(5H)-yl)methoxy)-4-((R)-3-hydroxy-3-methylpiperidin-1-yl)-7,8-d ihydro-5H-thiopyrano[4,3-d]pyrimidine 6,6-dioxide (7)

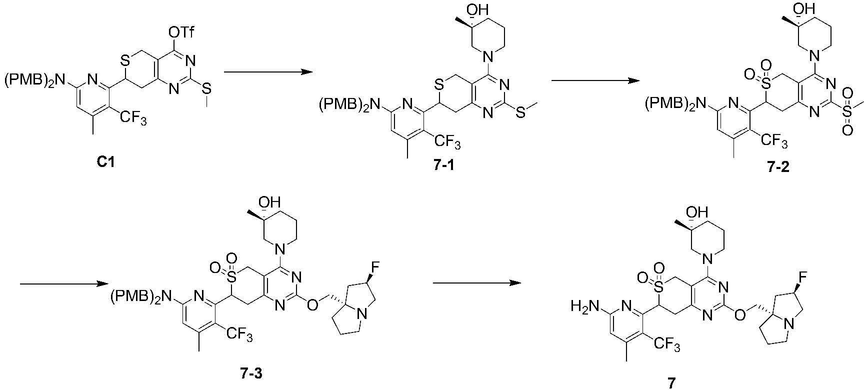

Compound **7** was prepared using a similar method to that used for compound 1 in **Example 1** (with **C1** as the starting material). LC/MS(ESI): m/z =628.7[M+H]⁺.

### Example 14

### (7S)-7-(6-amino-4-methyl-3-(trifluoromethyl)pyridin-2-yl)-2-(((2R,7AS)-2-fluorotetr ahydro-1H-pyrrolazin-7a(5H)-yl)methoxy)-4-((R)-3-hydroxy-3-methylpiperidin-1-yl) -7,8-dihydro-5H-thiopyrano[4,3-d]pyrimidine-6,6-dioxide (7A)

### (7R)-7-(6-amino-4-methyl-3-(trifluoromethyl)pyridin-2-yl)-2-(((2R,7AS)-2-fluorotetr ahydro-1H-pyrrolazin-7a(5H)-yl)methoxy)-4-((R)-3-hydroxy-3-methylpiperidin-1-yl) -7,8-dihydro-5H-thiopyrano[4,3-d]pyrimidine-6,6-dioxide (7B)

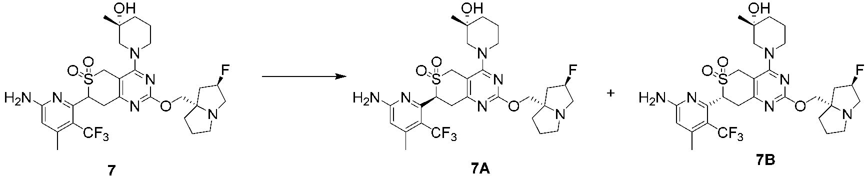

Compounds **7A** and **7B** were prepared using a similar method to that used for compounds **1A** and **1B** in **Example 2** (with compound **7** as the starting material). LC/MS(ESI): m/z =628.7[M+H]⁺.

### Example 15

### 2-(((2R,7AS)-2-fluorotetrahydro-1H-pyrrolazin-7a(5H)-yl)methoxy)-4-((R)-3-hydrox y-3-methylpiperidin-1-yl)-7-(6-hydroxy-4-methyl-3-(trifluoromethyl)pyridin-2-yl)-7, 8-dihydro-5H-thiopyrano[4,3-d]pyrimidine-6,6-dioxide (8)

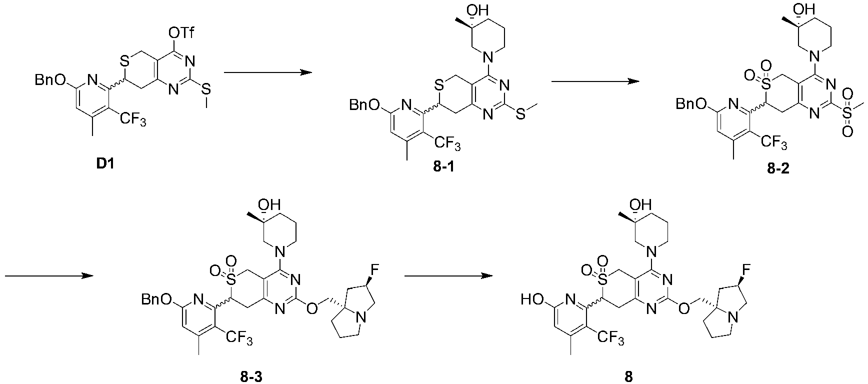

Compound 8 was prepared using a method similar to that used for compound **2** in **Example 3** (with the starting material replaced by **D1).** LC/MS(ESI): m/z =629.7[M+H]⁺.

### Example 16

### (7S)-7-(6-amino-4-methyl-3-(trifluoromethyl)pyridin-2-yl)-2-(((2R,7AS)-2-fluorotetr ahydro-1H-pyrrolazin-7a(5H)-yl)methoxy)-4-((R)-3-hydroxy-3-methylpiperidin-1-yl) -7,8-dihydro-5H-thiopyrano[4,3-d]pyrimidine-6,6-dioxide (8A)

### (7R)-7-(6-amino-4-methyl-3-(trifluoromethyl)pyridin-2-yl)-2-(((2R,7AS)-2-fluorotetr ahydro-1H-pyrrolazin-7a(5H)-yl)methoxy)-4-((R)-3-hydroxy-3-methylpiperidin-1-yl) -7,8-dihydro-5H-thiopyrano[4,3-d]pyrimidine-6,6-dioxide (8B)

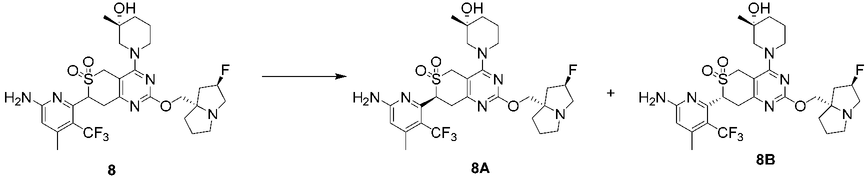

Compounds **8A** and **8B** were prepared using a similar method to that used for compounds **1A** and **1B** in **Example 2** (with compound **8** as the starting material). LC/MS(ESI): m/z =629.7[M+H]⁺.

### Example 17

### 7-(3-amino-2-fluoro-5-methyl-6-(trifluoromethyl)phenyl)-4-(3,8-diazabicyclo[3.2.1]o ctane-3-yl)-2-(((S)-2-(difluoromethylene)tetrahydro-1H-pyrrolazin-7a(5H)-yl)methox y)-7,8-dihydro-5H-thiopyrano[4,3-d]pyrimidine-6,6-dioxide (9)

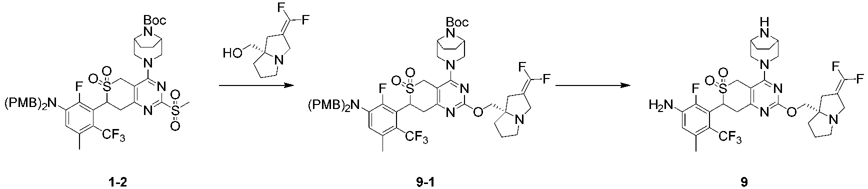

### Step A

Under ice-water bath conditions, compound ((S)-2-(difluoromethylene)tetrahydro-1H-pyrrolazin-7a(5H)-yl)methanol (12.09 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **1-2** (53 mg, 58.42 µmol) was added. The reaction mixture was stirred for 2 hours under ice-water bath conditions. The organic solvent was removed under reduced pressure, and the crude product was purified by thin-layer chromatography to obtain compounds **9-1.** LC/MS(ESI): m/z =983.1[M+H]⁺.

### Step B

Compounds **9-1** (42 mg, 42.78 µmol) were dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain compound **17.** LC/MS(ESI): m/z =642.7[M+H]⁺. ¹H-NMR (DMSO-*d*₆) *δ* 1.99 (m, 4H), 2.10-2.27 (m, 3H), 2.84 (d, 1H), 2.98 (d, 1H), 3.29-3.64 (m, 3H), 3.84 (d, 1H), 4.09-4.76 (m, 13H), 5.96 (s, 2H) 6.84 (d, 1H).

### Example 18

### (7S)-7-(3-amino-2-fluoro-5-methyl-6-(trifluoromethyl)phenyl)-4-(3,8-diazabicyclo[3. 2.1]octane-3-yl)-2-(((S)-2-(difluoromethylene)tetrahydro-1H-pyrrolazin-7a(5H)-yl)m ethoxy)-7,8-dihydro-5H-thiopyrano[4,3-d]pyrimidine-6,6-dioxide (9A)

### (7R)-7-(3-amino-2-fluoro-5-methyl-6-(trifluoromethyl)phenyl)-4-(3,8-diazabicyclo[3. 2.1]octane-3-yl)-2-(((S)-2-(difluoromethylene)tetrahydro-1H-pyrrolazin-7a(5H)-yl)m ethoxy)-7,8-dihydro-5H-thiopyrano[4,3-d]pyrimidine-6,6-dioxide (9B)

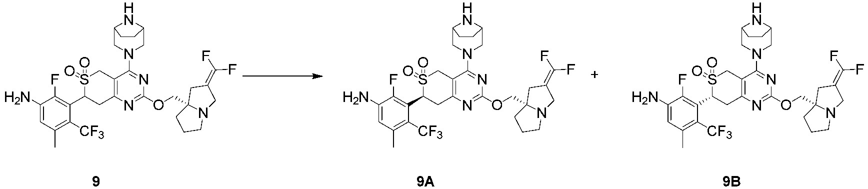

Compound **9** was purified by supercritical liquid chromatography (SFC) to obtain **9A** and **9B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =642.7[M+H]⁺).

### Example 19

### 7-7-(3-amino-2-fluoro-5-methyl-6-(trifluoromethyl)phenyl)-4-(3,8-diazabicyclo[3.2.1] octane-3-yl)-2-(((S)-2-(fluoromethylene)tetrahydro-1H-pyrrolazin-7a(5H)-yl)methoxy) -7,8-dihydro-5H-thiopyrano[4,3-d]pyrimidine-6,6-dioxide (10)

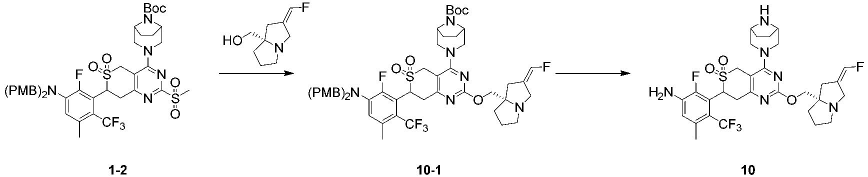

### Step A

Under ice-water bath conditions, compound ((S)-2-(fluoromethylene)tetrahydro-1H-pyrrolazin-7a(5H)-yl)methanol (12.98 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **1-2** (53 mg, 58.42 µmol) was added. The reaction mixture was stirred for 2 hours under ice-water bath conditions. The organic solvent was removed under reduced pressure, and the crude product was purified by thin-layer chromatography to obtain compound **10-1.** LC/MS(ESI): m/z =995.4[M+H]⁺.

### Step B

Compound **10-1** (43 mg, 42.78 µmol) was dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain the compound **10.** LC/MS(ESI): m/z =655.2[M+H]⁺. ¹H-NMR (DMSO-*d*₆) *δ* 1.68-2.31(m,12H), 2.75 (d, 1H), 2.94 (d, 1H), 3.17-3.61 (m, 5H), 4.05-4.78 (m, 12H), 5.96 (s, 2H), 6.84-7.05 (m, 2H).

### Example 20

### (7S)-7-(3-amino-2-fluoro-5-methyl-6-(trifluoromethyl)phenyl)-4-(3,8-diazabicyclo[3. 2.1]octane-3-yl)-2-(((S)-2-(difluoromethylene)tetrahydro-1H-pyrrolazin-7a(5H)-yl)m ethoxy)-7,8-dihydro-5H-thiopyrano[4,3-d]pyrimidine-6,6-dioxide (10A)

### (7R)-7-(3-amino-2-fluoro-5-methyl-6-(trifluoromethyl)phenyl)-4-(3,8-diazabicyclo[3. 2.1]octane-3-yl)-2-(((S)-2-(difluoromethylene)tetrahydro-1H-pyrrolazin-7a(5H)-yl)m ethoxy)-7,8-dihydro-5H-thiopyrano[4,3-d]pyrimidine-6,6-dioxide (10B)

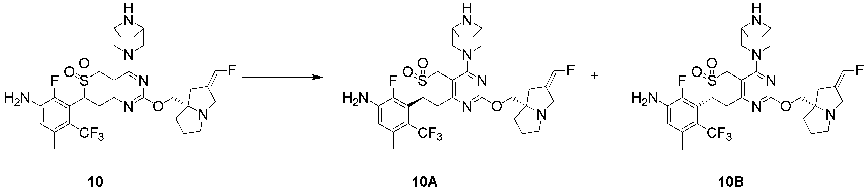

Compound **10** was purified by supercritical liquid chromatography (SFC) to obtain **10A** and **10B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =655.2[M+H]⁺).

### Example 21

### 7-(3-amino-2-fluoro-5-methyl-6-(trifluoromethyl)phenyl)-4-(3,8-diazabicyclo[3.2.1]o ctane-3-yl)-2-(((S)-2-(methylene)tetrahydro-1H-pyrrolazin-7a(5H)-yl)methoxy)-7,8-d ihydro-5H-thiopyrano[4,3-d]pyrimidine-6,6-dioxide (11)

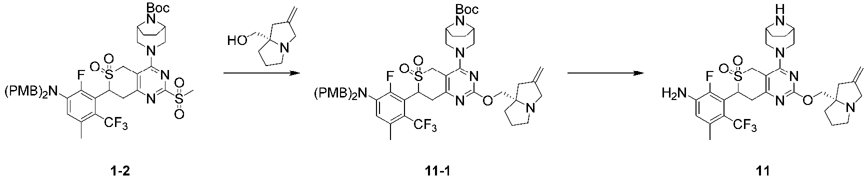

### Step A

Under ice-water bath conditions, compound ((S)-2-(methylene)tetrahydro-1H-pyrrolazin-7a(5H)-yl)methanol (11.62 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **1-2** (53 mg, 58.42 µmol) was added. The reaction mixture was stirred for 2 hours under ice-water bath conditions. The organic solvent was removed under reduced pressure, and the crude product was purified by thin-layer chromatography to obtain compound **11-1.** LC/MS(ESI): m/z =977.1[M+H]⁺.

### Step B

Compound **11-1** (42 mg, 42.78 µmol) was dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain the compound **11.** LC/MS(ESI): m/z =637.3[M+H]⁺. ¹H-NMR (DMSO-*d*₆) *δ* 1.79-2.31(m,12H), 2.55-275 (m, 1H), 2.94 (d, 1H), 3.17-3.61 (m, 5H), 4.05-4.62 (m, 11H), 5.20 (d, 2H), 6.84 (d, 1H).

### Example 22

### (7S)-7-(3-amino-2-fluoro-5-methyl-6-(trifluoromethyl)phenyl)-4-(3,8-diazabicyclo[3. 2.1]octane-3-yl)-2-(((S)-2-(methylene)tetrahydro-1H-pyrrolazin-7a(5H)-yl)methoxy)-7,8-dihydro-5H-thiopyrano[4,3-d]pyrimidine-6,6-dioxide (11A)

### (7R)-7-(3-amino-2-fluoro-5-methyl-6-(trifluoromethyl)phenyl)-4-(3,8-diazabicyclo[3. 2.1]octane-3-yl)-2-(((S)-2-(methylene)tetrahydro-1H-pyrrolazin-7a(5H)-yl)methoxy)-7,8-dihydro-5H-thiopyrano[4,3-d]pyrimidine-6,6-dioxide (11B)

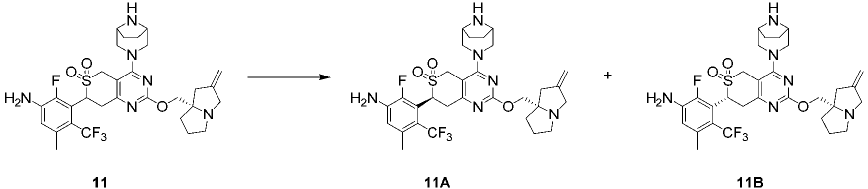

Compound **11** was purified by supercritical liquid chromatography (SFC) to obtain **11A** and **11B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =642.7[M+H]⁺).

### Example 23

### 7-(6-hydroxy-4-methyl-3-(trifluoromethyl)pyridin-2-yl)-2-(((S)-2-(methylene)tetrahy dro-1H-pyrrolazin-7a(5H)-yl)methoxy)-4-((R)-3-hydroxy-3-methylpiperidin-1-yl)-7,8 -dihydro-5H-thiopyrano[4,3-d]pyrimidine-6,6-dioxide (12)

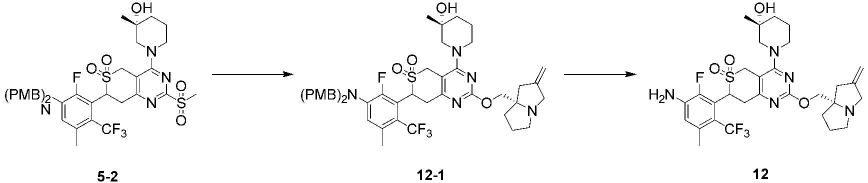

Compound **12** was prepared using a method similar to that used for compound **1** in **Example 1.** LC/MS(ESI): m/z =640.3[M+H]⁺.

### Example 24

### (7S)-7-(6-hydroxy-4-methyl-3-(trifluoromethyl)pyridin-2-yl)-2-(((S)-2-(methylene)tet rahydro-1H-pyrrolazin-7a(5H)-yl)methoxy)-4-((R)-3-hydroxy-3-methylpiperidin-1-yl) -7,8-dihydro-5H-thiopyrano[4,3-d]pyrimidine-6,6-dioxide (12A)

### (7R)-7-(6-hydroxy-4-methyl-3-(trifluoromethyl)pyridin-2-yl)-2-(((S)-2-(methylene)tet rahydro-1H-pyrrolazin-7a(5H)-yl)methoxy)-4-((R)-3-hydroxy-3-methylpiperidin-1-yl) -7,8-dihydro-5H-thiopyrano[4,3-d]pyrimidine-6,6-dioxide (12B)

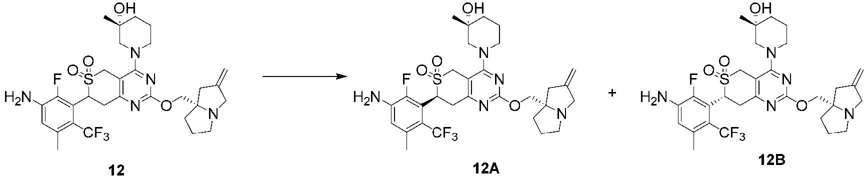

Compounds **12A** and **12B** were prepared using a similar method to that used for compounds **2A** and **2B** in **Example 2** (with compound **12** as the starting material). LC/MS(ESI): m/z ==640.3[M+H]⁺.

### Example 25

### 7-(3-amino-2-fluoro-5-methyl-6-(trifluoromethyl)phenyl)-2-(((S)-2-(fluoromethylene) tetrahydro-1H-pyrrolazin-7a(5H)-yl)methoxy)-4-((R)-3-hydroxy-3-methylpiperidin-1-yl)-7,8-dihydro-5H-thiopyrano[4,3-d]pyrimidine-6,6-dioxide (13)

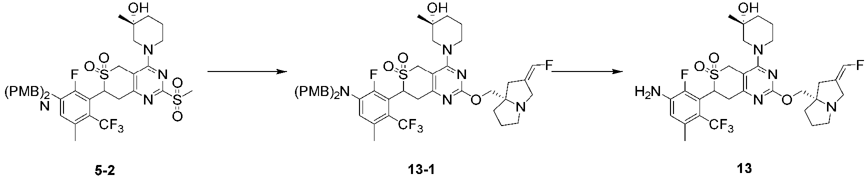

Compound **13** was prepared using a method similar to that used for compound **1** in **Example 1.** LC/MS(ESI): m/z =658.2[M+H]⁺.

### Example 26

### (7S)-7-(3-amino-2-fluoro-5-methyl-6-(trifluoromethyl)phenyl)-2-(((S)-2-(fluoromethy lene)tetrahydro-1H-pyrrolazin-7a(5H)-yl)methoxy)-4-((R)-3-hydroxy-3-methylpiperi din-1-yl)-7,8-dihydro-5H-thiopyrano[4,3-d]pyrimidine-6,6-dioxide (13A)

### (7R)-7-(3-amino-2-fluoro-5-methyl-6-(trifluoromethyl)phenyl)-2-(((S)-2-(fluorometh ylene)tetrahydro-1H-pyrrolazin-7a(5H)-yl)methoxy)-4-((R)-3-hydroxy-3-methylpiper idin-1-yl)-7,8-dihydro-5H-thiopyrano[4,3-d]pyrimidine-6,6-dioxide (13B)

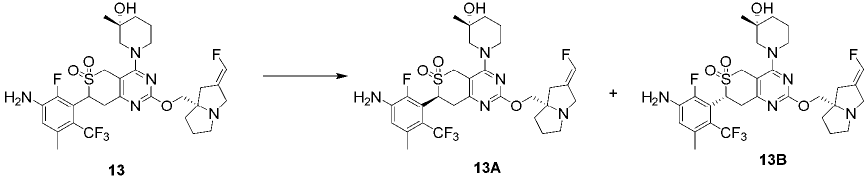

Compounds **13A** and **13B** were prepared using a similar method to that used for compounds **2A** and **2B** in **Example 2** (with compound **13** as the starting material). LC/MS(ESI): m/z =658.2[M+H]⁺.

### Example 27

### 7-(3-amino-2-fluoro-5-methyl-6-(trifluoromethyl)phenyl)-2-(((S)-2-(difluoromethylen e)tetrahydro-1H-pyrrolazin-7a(5H)-yl)methoxy)-4-((R)-3-hydroxy-3-methylpiperidin-1-yl)-7,8-dihydro-5H-thiopyrano[4,3-d]pyrimidine-6,6-dioxide (14)

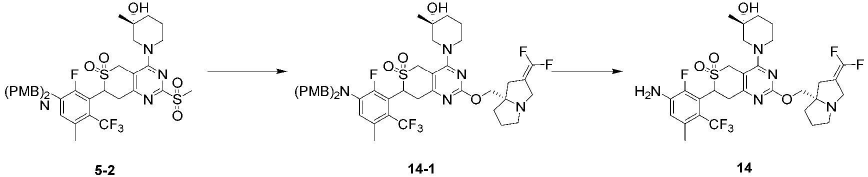

Compound **14** was prepared using a method similar to that used for compound **1** in **Example 1.** LC/MS(ESI): m/z =676.2[M+H]⁺.

### Example 28

### (7S)-7-(3-amino-2-fluoro-5-methyl-6-(trifluoromethyl)phenyl)-2-(((S)-2-(difluoromet hylene)tetrahydro-1H-pyrrolazin-7a(5H)-yl)methoxy)-4-((R)-3-hydroxy-3-methylpipe ridin-1-yl)-7,8-dihydro-5H-thiopyrano[4,3-d]pyrimidine-6,6-dioxide (14A)

### (7R)-7-(3-amino-2-fluoro-5-methyl-6-(trifluoromethyl)phenyl)-2-(((S)-2-(difluoromet hylene)tetrahydro-1H-pyrrolazin-7a(5H)-yl)methoxy)-4-((R)-3-hydroxy-3-methylpipe ridin-1-yl)-7,8-dihydro-5H-thiopyrano[4,3-d]pyrimidine-6,6-dioxide (14B)

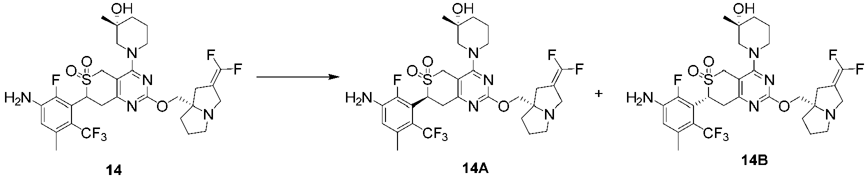

Compounds **14A** and **14B** were prepared using a similar method to that used for compounds **2A** and **2B** in **Example 2** (with compound **14** as the starting material). LC/MS(ESI): m/z =676.2[M+H]⁺.

### Example 29

### 7-(6-amino-4-methyl-3-(trifluoromethyl)pyridin-2-yl)-2-(((S)-2-(difluoromethylene)te trahydro-1H-pyrrolazin-7a(5H)-yl)methoxy)-4-((R)-3-hydroxy-3-methylpiperidin-1-y 1)-7,8-dihydro-5H-thiopyrano[4,3-d]pyrimidine-6,6-dioxide (15)

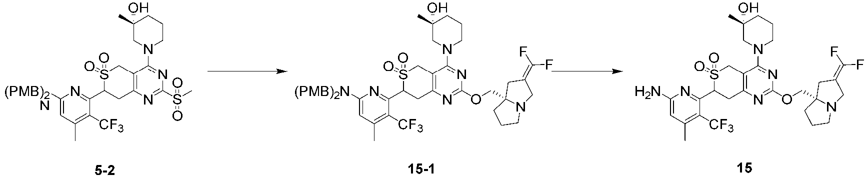

Compound **15** was prepared using a method similar to that used for compound **1** in **Example 1.** LC/MS(ESI): m/z =659.2[M+H]⁺.

### Example 30

### (7S)-7-(6-amino-4-methyl-3-(trifluoromethyl)pyridin-2-yl)-2-(((S)-2-(difluoromethyle ne)tetrahydro-1H-pyrrolazin-7a(5H)-yl)methoxy)-4-((R)-3-hydroxy-3-methylpiperidi n-1-yl)-7,8-dihydro-5H-thiopyrano[4,3-d]pyrimidine-6,6-dioxide (15A)

### (7R)-7-(6-amino-4-methyl-3-(trifluoromethyl)pyridin-2-yl)-2-(((S)-2-(difluoromethyl ene)tetrahydro-1H-pyrrolazin-7a(5H)-yl)methoxy)-4-((R)-3-hydroxy-3-methylpiperid in-1-yl)-7,8-dihydro-5H-thiopyrano[4,3-d]pyrimidine-6,6-dioxide (15B)

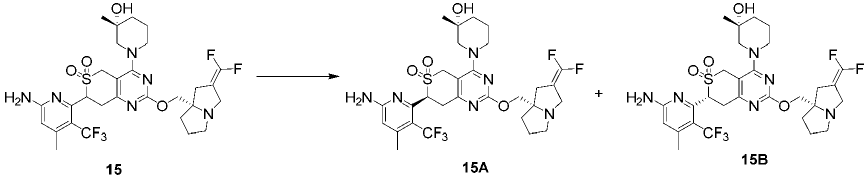

Compounds **15A** and **15B** were obtained using a similar preparation method to that used for compounds **2A** and **2B** in **Example 2** (with compound **15** as the starting material). LC/MS(ESI): m/z 659.2[M+H]⁺.

### Example 31

### 7-(6-amino-4-methyl-3-(trifluoromethyl)pyridin-2-yl)-2-(((S)-2-(fluoromethylene)tetr ahydro-1H-pyrrolazin-7a(5H)-yl)methoxy)-4-((R)-3-hydroxy-3-methylpiperidin-1-yl) -7,8-dihydro-5H-thiopyrano[4,3-d]pyrimidine-6,6-dioxide (16)

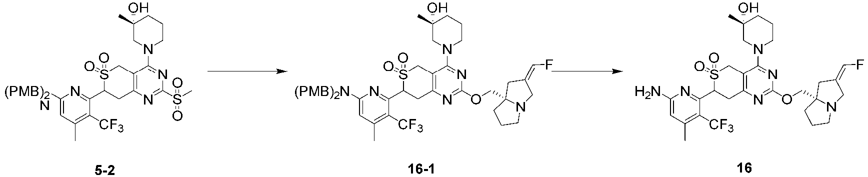

Compound **16** was prepared using a method similar to that used for compound **1** in **Example 1.** LC/MS(ESI): m/z =641.2[M+H]⁺.

### Example 32

### (7S)-7-(6-amino-4-methyl-3-(trifluoromethyl)pyridin-2-yl)-2-(((S)-2-(fluoromethylen e)tetrahydro-1H-pyrrolazin-7a(5H)-yl)methoxy)-4-((R)-3-hydroxy-3-methylpiperidin-1-yl)-7,8-dihydro-5H-thiopyrano[4,3-d]pyrimidine-6,6-dioxide (16A)

### (7R)-7-(6-amino-4-methyl-3-(trifluoromethyl)pyridin-2-yl)-2-(((S)-2-(fluoromethylen e)tetrahydro-1H-pyrrolazin-7a(5H)-yl)methoxy)-4-((R)-3-hydroxy-3-methylpiperidin-1-yl)-7,8-dihydro-5H-thiopyrano[4,3-d]pyrimidine-6,6-dioxide (16B)

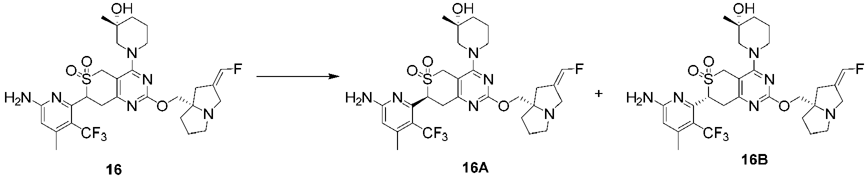

Compounds **16A** and **16B** were prepared using a similar method to that used for compounds **2A** and **2B** in **Example 2** (with compound **16** as the starting material). LC/MS(ESI): m/z =641.2[M+H]⁺.

### Example 33

### 7-(6-amino-4-methyl-3-(trifluoromethyl)pyridin-2-yl)-2-(((S)-2-(methylene)tetrahydr o-1H-pyrrolazin-7a(5H)-yl)methoxy)-4-((R)-3-hydroxy-3-methylpiperidin-1-yl)-7,8-d ihydro-5H-thiopyrano[4,3-d]pyrimidine-6,6-dioxide (17)

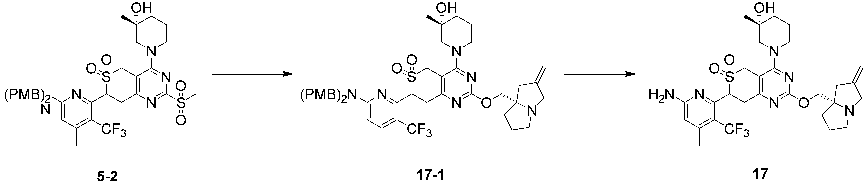

Compound **17** was prepared using a method similar to that used for compound **1** in **Example 1.** LC/MS(ESI): m/z =622.3[M+H]⁺.

### Example 34

### (7S)-7-(6-amino-4-methyl-3-(trifluoromethyl)pyridin-2-yl)-2-(((S)-2-(methylene)tetra hydro-1H-pyrrolazin-7a(5H)-yl)methoxy)-4-((R)-3-hydroxy-3-methylpiperidin-1-yl)-7,8-dihydro-5H-thiopyrano[4,3-d]pyrimidine-6,6-dioxide (17A)

### (7R)-7-(6-amino-4-methyl-3-(trifluoromethyl)pyridin-2-yl)-2-(((S)-2-(methylene)tetra hydro-1H-pyrrolazin-7a(5H)-yl)methoxy)-4-((R)-3-hydroxy-3-methylpiperidin-1-yl)-7,8-dihydro-5H-thiopyrano[4,3-d]pyrimidine-6,6-dioxide (17B)

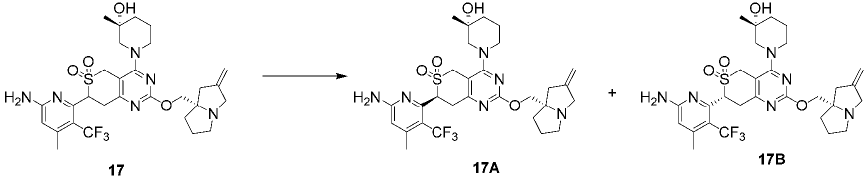

Compounds **17A** and **17B** were prepared using a similar method to that used for compounds **2A** and **2B** in **Example 2** (with compound **17** as the starting material). LC/MS(ESI): m/z =622.3[M+H]⁺.

### Example 35

### 7-(6-amino-4-methyl-3-(trifluoromethyl)pyridin-2-yl)-2-(((S)-2-(difluoromethylene)te trahydro-1H-pyrrolazin-7a(5H)-yl)methoxy)-4-(3,8-diazabicyclo[3.2.1]octane-3-yl)-7, 8-dihydro-5H-thiopyrano[4,3-d]pyrimidine-6,6-dioxide (18)

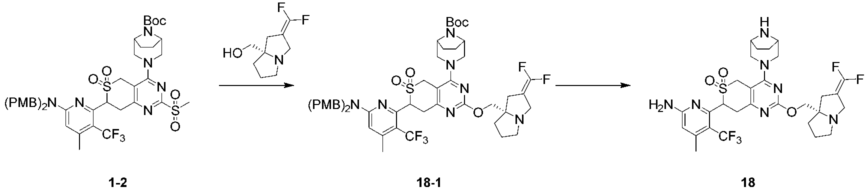

### Step A

Under ice-water bath conditions, compound ((S)-2-(difluoromethylene)tetrahydro-1H-pyrrolazin-7a(5H)-yl)methanol (14.28 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **1-2** (53 mg, 58.42 µmol) was added. The reaction mixture was stirred for 2 hours under ice-water bath conditions. The organic solvent was removed under reduced pressure, and the crude product was purified by thin-layer chromatography to obtain compound **18-1.** LC/MS(ESI): m/z =996.1[M+H]⁺.

### Step B

Compound **18-1** (43 mg, 42.78 µmol) was dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain the compound **18.** LC/MS(ESI): m/z =656.2[M+H]⁺. ¹H-NMR (CDCl₃) *δ* 1.79-2.31(m,12H), 2.75 (d, 1H), 2.94 (d, 1H), 3.17-3.61 (m, 5H), 4.05-4.68 (m, 11H), 7.30 (s, 1H).

### Example 36

### (7S)-7-(6-amino-4-methyl-3-(trifluoromethyl)pyridin-2-yl)-2-(((S)-2-(difluoromethyle ne)tetrahydro-1H-pyrrolazin-7a(5H)-yl)methoxy)-4-(3,8-diazabicyclo[3.2.1]octane-3-yl)-7,8-dihydro-5H-thiopyrano[4,3-d]pyrimidine-6,6-dioxide (18A)

### (7R)-7-(6-amino-4-methyl-3-(trifluoromethyl)pyridin-2-yl)-2-(((S)-2-(difluoromethyl ene)tetrahydro-1H-pyrrolazin-7a(5H)-yl)methoxy)-4-(3,8-diazabicyclo[3.2.1]octane-3 -yl)-7,8-dihydro-5H-thiopyrano[4,3-d]pyrimidine-6,6-dioxide (18B)

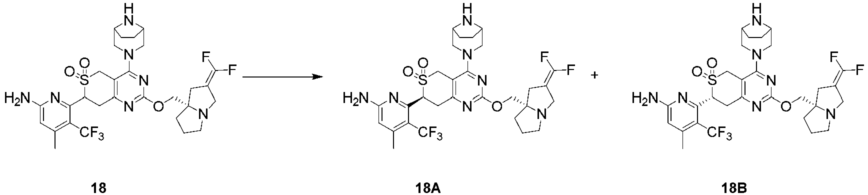

Compound **18** was purified by supercritical liquid chromatography (SFC) to yield **18A** and **18B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =656.2[M+H]⁺).

### Example 37

### 7-(6-amino-4-methyl-3-(trifluoromethyl)pyridin-2-yl)-2-(((S)-2-(fluoromethylene)tetr ahydro-1H-pyrrolazin-7a(5H)-yl)methoxy)-4-(3,8-diazabicyclo[3.2.1]octane-3-yl)-7,8 -dihydro-5H-thiopyrano[4,3-d]pyrimidine-6,6-dioxide (19)

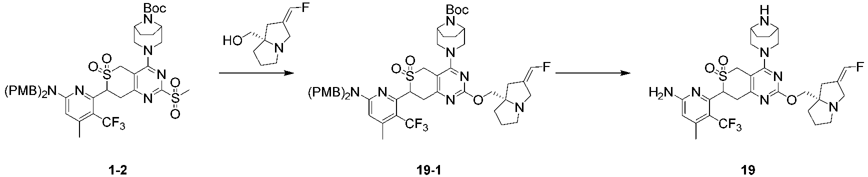

### Step A

Under ice-water bath conditions, compound ((S)-2-(fluoromethylene)tetrahydro-1H-pyrrolazin-7a(5H)-yl)methanol (12.98 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **1-2** (53 mg, 58.42 µmol) was added. The reaction mixture was stirred for 2 hours under ice-water bath conditions. The organic solvent was removed under reduced pressure, and the crude product was purified by thin-layer chromatography to obtain compound 19-1. LC/MS(ESI): m/z =983.1[M+H]⁺.

### Step B

Compound **19-1** (42 mg, 42.78 µmol) was dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain the compound **19.** LC/MS(ESI): m/z =638.3[M+H]⁺.

### Example 38

### (7S)-7-(6-amino-4-methyl-3-(trifluoromethyl)pyridin-2-yl)-2-(((S)-2-(fluoromethylen e)tetrahydro-1H-pyrrolazin-7a(5H)-yl)methoxy)-4-(3,8-diazabicyclo[3.2.1]octane-3-y 1)-7,8-dihydro-5H-thiopyrano[4,3-d]pyrimidine-6,6-dioxide (19A)

### (7R)-7-(6-amino-4-methyl-3-(trifluoromethyl)pyridin-2-yl)-2-(((S)-2-(fluoromethylen e)tetrahydro-1H-pyrrolazin-7a(5H)-yl)methoxy)-4-(3,8-diazabicyclo[3.2.1]octane-3-y 1)-7,8-dihydro-5H-thiopyrano[4,3-d]pyrimidine-6,6-dioxide (19B)

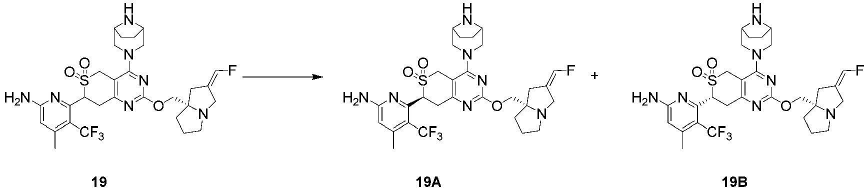

Compound **19** was purified by supercritical liquid chromatography (SFC) to obtain **19A** and **19B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =638.3[M+H]⁺).

### Example 39

### 7-(6-amino-4-methyl-3-(trifluoromethyl)pyridin-2-yl)-2-(((S)-2-(methylene)tetrahydr o-1H-pyrrolazin-7a(5H)-yl)methoxy)-4-(3,8-diazabicyclo[3.2.1]octane-3-yl)-7,8-dihy dro-5H-thiopyrano[4,3-d]pyrimidine-6,6-dioxide (20)

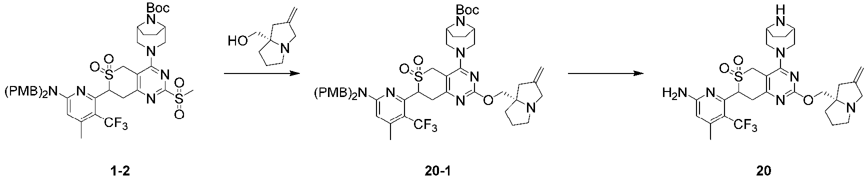

### Step A

Under ice-water bath conditions, compound ((S)-2-(methylene)tetrahydro-1H-pyrrolazin-7a(5H)-yl)methanol (11.63 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **1-2** (53 mg, 58.42 µmol) was added. The reaction mixture was stirred for 2 hours under ice-water bath conditions. The organic solvent was removed under reduced pressure, and the crude product was purified by thin-layer chromatography to obtain compound **20-1.** LC/MS(ESI): m/z =983.1[M+H]⁺.

### Step B

Compound **20-1** (42 mg, 42.78 µmol) was dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain compound **20.** LC/MS(ESI): m/z =620.3[M+H]⁺. ¹H-NMR (CDCl₃) *δ* 1.78-2.31(m,12H), 2.55-75 (m, 1H), 2.94 (d, 1H), 3.17-3.61 (m, 5H), 4.05-4.62 (m, 11H), 5.20 (d, 2H), 7.30 (s, 1H).

### Example 40

### (7S)-7-(6-amino-4-methyl-3-(trifluoromethyl)pyridin-2-yl)-2-(((S)-2-(methylene)tetra hydro-1H-pyrrolazin-7a(5H)-yl)methoxy)-4-(3,8-diazabicyclo[3.2.1]octane-3-yl)-7,8-dihydro-5H-thiopyrano[4,3-d]pyrimidine-6,6-dioxide (20A)

### (7R)-7-(6-amino-4-methyl-3-(trifluoromethyl)pyridin-2-yl)-2-(((S)-2-(methylene)tetra hydro-1H-pyrrolazin-7a(5H)-yl)methoxy)-4-(3,8-diazabicyclo[3.2.1]octane-3-yl)-7,8-dihydro-5H-thiopyrano[4,3-d]pyrimidine-6,6-dioxide (20B)

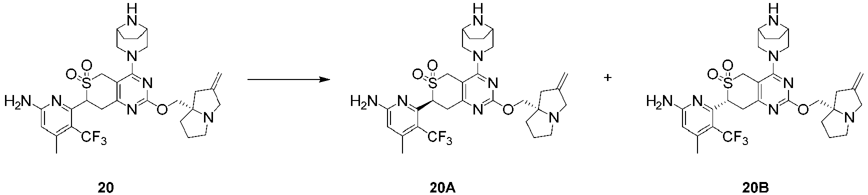

Compound **20** was purified by supercritical liquid chromatography (SFC) to obtain **20A** and **20B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =620.3[M+H]⁺).

### Example 41

### 7-(3-amino-2-fluoro-5-(prop-1-yn-1-yl)-6-(trifluoromethyl)phenyl)-4-(3,8-diazabicycl o[3.2.1]octane-3-yl)-2-(((2R,7AS)-2-fluorotetrahydro-1H-pyrrolazin-7a(5H)-yl)metho xy)-7,8-dihydro-5H-thiopyrano[4,3-d]pyrimidine-6,6-dioxide (21)

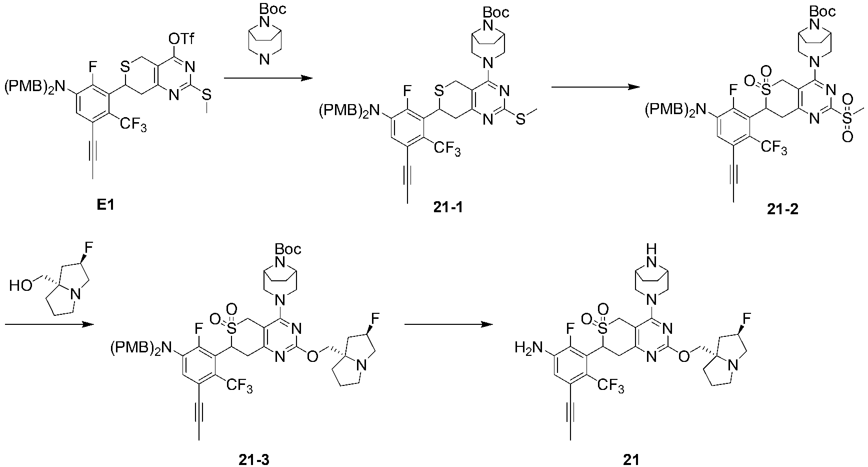

### Step A

Compound **E1** (84 mg, 105.02 µmol) and 8-tert-butoxycarbonyl-3,8-diazabicyclo[3.2.1]octane (26.63 mg, 126.03 µmol) were dissolved in N,N-dimethylformamide (2 mL), and diisopropylethylamine (40.72 mg, 315.07 µmol) was added. The reaction mixture was heated to 100°C and stirred for 1 hour. After cooling, the organic solvent was removed under reduced pressure. The crude product was purified by thin-layer chromatography to obtain the compound **21-1.** LC/MS(ESI): m/z = 864[M+H]⁺.

### Step B

Compound **21-1** (73 mg, 84.96 µmol) was dissolved in dichloromethane (2 mL), and m-chloroperoxybenzoic acid (34.50 mg, 169.92 µmol) was added. The reaction mixture was stirred at 20 °C for 3 hours. The organic solvent was removed under reduced pressure, and the crude product was purified by thin-layer chromatography to obtain the compound **21-2.** LC/MS(ESI): m/z =928[M+H]⁺.

### Step C

Under ice-water bath conditions, compound ((2R,7AS)-2-fluorotetrahydro-1H-pyrrolazine-7a(5H)-yl)methanol (12.09 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **21-2** (60 mg, 58.42 µmol) was added. The reaction mixture was stirred for 2 hours under ice-water bath conditions. The organic solvent was removed under reduced pressure, and the crude product was purified by thin-layer chromatography to obtain compound **21-3.** LC/MS(ESI): m/z =1007.4[M+H]⁺.

### Step D

Compound **21-3** (43 mg, 42.78 µmol) was dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain the compound **21.** LC/MS(ESI): m/z =667.2[M+H]⁺. ¹H-NMR (CDCl₃) *δ* 1.78-2.20 (m, 7H), 2.01-2.20 (m, 6H), 2.25-2.60 (m, 6H), 3.00-3.20 (m, 2H), 3.30-3.50 (m, 1H), 3.60-3.80 (m, 2H), 3.80-4.15 (m, 4H), 4.30-4.98 (m, 7H), 6.82 (d, 1H).

### Example 42

### (7S)-7-(3-amino-2-fluoro-5-(prop-1-yn-1-yl)-6-(trifluoromethyl)phenyl)-4-(3,8-diaza bicyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine-6,6-dioxide (21A)

### (7R)-7-(3-amino-2-fluoro-5-(prop-1-yn-1-yl)-6-(trifluoromethyl)phenyl)-4-(3,8-diaza bicyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine-6,6-dioxide (21B)

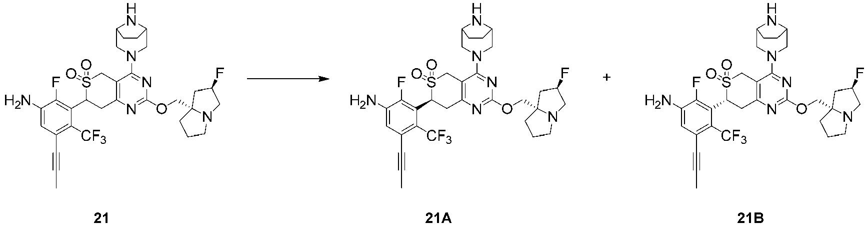

Compound **21** was purified by supercritical liquid chromatography (SFC) to obtain **21A** and **21B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =667.2[M+H]⁺).

### Example 43

### 7-(3-amino-2-fluoro-5-(prop-1-yn-1-yl)-6-(trifluoromethyl)phenyl)-4-(3,8-diazabicycl o[3.2.1]octane-3-yl)-2-(((S)-2-(methylene)tetrahydro-1H-pyrrolazin-7a(5H)-yl)metho xy)-7,8-dihydro-5H-thiopyrano[4,3-d]pyrimidine-6,6-dioxide (22)

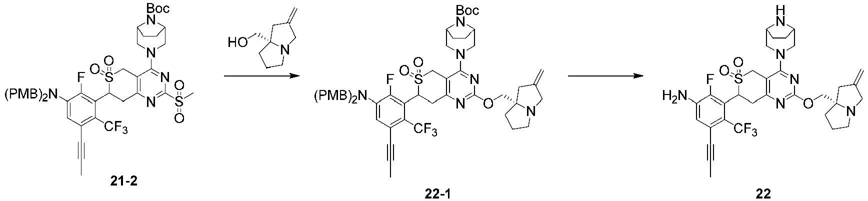

### Step A

Under ice-water bath conditions, compound ((S)-2-(methylene)tetrahydro-1H-pyrrolazin-7a(5H)-yl)methanol (11.63 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **1-2** (53 mg, 58.42 µmol) was added. The reaction mixture was stirred for 2 hours under ice-water bath conditions. The organic solvent was removed under reduced pressure, and the crude product was purified by thin-layer chromatography to obtain compound **22-1.** LC/MS(ESI): m/z =1001.4[M+H]⁺.

### Step D

Compound **22-1** (43 mg, 42.78 µmol) was dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain compound **22.** LC/MS(ESI): m/z =661.2 [M+H]⁺. ¹H-NMR (CDCl₃) *δ* 1.79-2.31(m,12H), 2.75 (d, 1H), 2.94 (d, 1H), 3.17-3.61 (m, 5H), 4.05-4.62 (m, 11H), 5.20 (d, 2H), 6.82 (d, 1H).

### Example 44

### (7S)-7-(3-amino-2-fluoro-5-(prop-1-yn-1-yl)-6-(trifluoromethyl)phenyl)-4-(3,8-diaza bicyclo[3.2.1]octane-3-yl)-2-(((S)-2-(methylene)tetrahydro-1H-pyrrolazin-7a(5H)-yl) methoxy)-7,8-dihydro-5H-thiopyrano[4,3-d]pyrimidine-6,6-dioxide (22A)

### (7R)-7-(3-amino-2-fluoro-5-(prop-1-yn-1-yl)-6-(trifluoromethyl)phenyl)-4-(3,8-diaza bicyclo[3.2.1]octane-3-yl)-2-(((S)-2-(methylene)tetrahydro-1H-pyrrolazin-7a(5H)-yl) methoxy)-7,8-dihydro-5H-thiopyrano[4,3-d]pyrimidine-6,6-dioxide (22B)

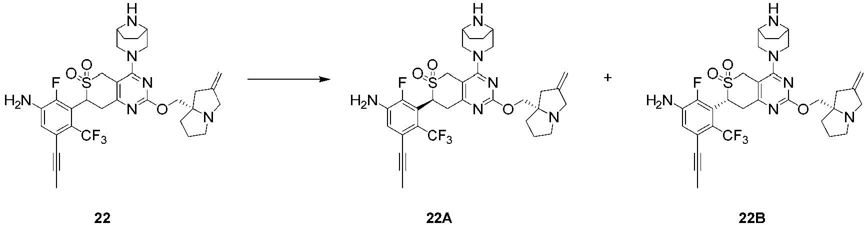

Compound **22** was purified by supercritical liquid chromatography (SFC) to obtain **22A** and **22B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =661.2 [M+H]⁺).

### Example 45

### 7-(3-amino-2-fluoro-5-(prop-1-yn-1-yl)-6-(trifluoromethyl)phenyl)-4-(3,8-diazabicycl o[3.2.1]octane-3-yl)-2-(((S)-2-(difluoromethylene)tetrahydro-1H-pyrrolazin-7a(5H)-y 1)methoxy)-7,8-dihydro-5H-thiopyrano[4,3-d]pyrimidine-6,6-dioxide (23)

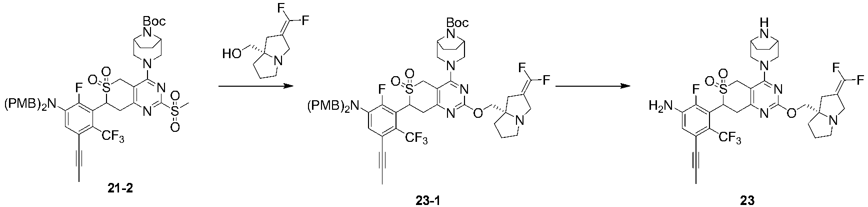

### Step A

Under ice-water bath conditions, compound ((S)-2-(difluoromethylene)tetrahydro-1H-pyrrolazin-7a(5H)-yl)methanol (14.28 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **1-2** (53 mg, 58.42 µmol) was added. The reaction mixture was stirred for 2 hours under ice-water bath conditions. The organic solvent was removed under reduced pressure, and the crude product was purified by thin-layer chromatography to obtain compound **23-1.** LC/MS(ESI): m/z =1036.4[M+H]⁺.

### Step B

Compound **23-1** (44 mg, 42.78 µmol) was dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain the compound **23.** LC/MS(ESI): m/z =697.2[M+H]⁺. ¹H-NMR (CDCl₃) *δ* 1.73-2.03 (m, 11H), 2.21-2.33 (m, 1H), 2.36-2.46 (m, 1H), 2.74 (m, 1H), 2.80 (d, 1H), 3.13-3.35 (m, 5H), 3.66 (m, 2H), 4.21-4.46 (m, 9H), 6.82 (d, 1H).

### Example 46

### (7S)-7-(3-amino-2-fluoro-5-(prop-1-yn-1-yl)-6-(trifluoromethyl)phenyl)-4-(3,8-diaza bicyclo[3.2.1]octane-3-yl)-2-(((S)-2-(difluoromethylene)tetrahydro-1H-pyrrolazin-7a( 5H)-yl)methoxy)-7,8-dihydro-5H-thiopyrano[4,3-d]pyrimidine-6,6-dioxide (23A)

### (7R)-7-(3-amino-2-fluoro-5-(prop-1-yn-1-yl)-6-(trifluoromethyl)phenyl)-4-(3,8-diaza bicyclo[3.2.1]octane-3-yl)-2-(((S)-2-(difluoromethylene)tetrahydro-1H-pyrrolazin-7a( 5H)-yl)methoxy)-7,8-dihydro-5H-thiopyrano[4,3-d]pyrimidine-6,6-dioxide (23B)

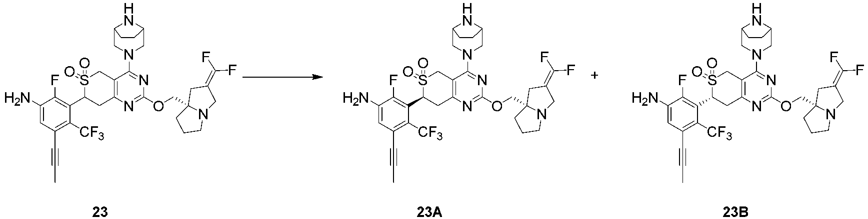

Compound **23** was purified by supercritical liquid chromatography (SFC) to obtain **23A** and **23B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =697.2[M+H]⁺).

### Example 47

### 7-(3-amino-2-fluoro-5-(prop-1-yn-1-yl)-6-(trifluoromethyl)phenyl)-4-(3,8-diazabicycl o[3.2.1]octane-3-yl)-2-(((S)-2-(fluoromethylene)tetrahydro-1H-pyrrolazin-7a(5H)-yl) methoxy)-7,8-dihydro-5H-thiopyrano[4,3-d]pyrimidine-6,6-dioxide (24)

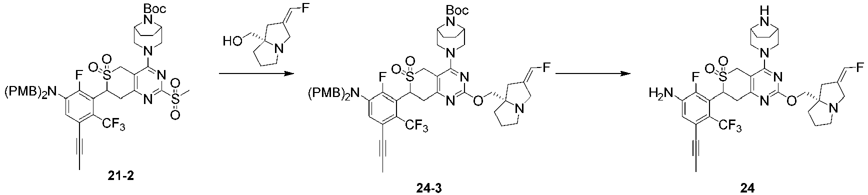

### Step A

Under ice-water bath conditions, compound ((S)-2-(fluoromethylene)tetrahydro-1H-pyrrolazin-7a(5H)-yl)methanol (12.98 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **1-2** (53 mg, 58.42 µmol) was added. The reaction mixture was stirred for 2 hours under ice-water bath conditions. The organic solvent was removed under reduced pressure, and the crude product was purified by thin-layer chromatography to obtain compound **24-3.** LC/MS(ESI): m/z =1019.1[M+H]⁺.

### Step B

Compound **24-3** (43 mg, 42.78 µmol) was dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain compound **24.** LC/MS(ESI): m/z =679.2[M+H]⁺. ¹H-NMR (CDCl₃) *δ* 1.71-2.20 (m, 5H), 2.01-2.20 (m, 6H), 2.25-2.60 (m, 6H), 3.00-3.20 (m, 3H), 3.30-3.50 (m, 1H), 3.60-3.80 (m, 2H), 3.80-4.15 (m, 4H), 4.30-4.98 (m, 7H), 6.46-6.71 (m, 1H), 6.84 (d, 1H).

### Example 48

### (7S)-7-(3-amino-2-fluoro-5-(prop-1-yn-1-yl)-6-(trifluoromethyl)phenyl)-4-(3,8-diaza bicyclo[3.2.1]octane-3-yl)-2-(((S)-2-(fluoromethylene)tetrahydro-1H-pyrrolazin-7a(5 H)-yl)methoxy)-7,8-dihydro-5H-thiopyrano[4,3-d]pyrimidine-6,6-dioxide (24A)

### (7R)-7-(3-amino-2-fluoro-5-(prop-1-yn-1-yl)-6-(trifluoromethyl)phenyl)-4-(3,8-diaza bicyclo[3.2.1]octane-3-yl)-2-(((S)-2-(fluoromethylene)tetrahydro-1H-pyrrolazin-7a(5 H)-yl)methoxy)-7,8-dihydro-5H-thiopyrano[4,3-d]pyrimidine-6,6-dioxide (24B)

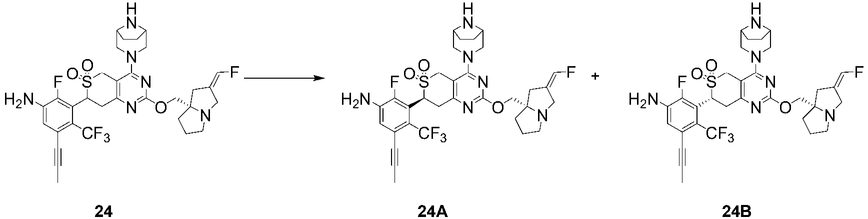

Compound **24** was purified by supercritical liquid chromatography (SFC) to obtain **24A** and **24B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =679.2[M+H]⁺).

### Example 49

### 7-(3-amino-2-fluoro-5-(prop-1-yn-1-yl)-6-(trifluoromethyl)phenyl)-4-((R)-3-hydroxy-3-methylpiperidin-1-yl)-2-(((S)-2-(difluoromethylene)tetrahydro-1H-pyrrolazin-7a(5 H)-yl)methoxy)-7,8-dihydro-5H-thiopyrano[4,3-d]pyrimidine-6,6-dioxide (25)

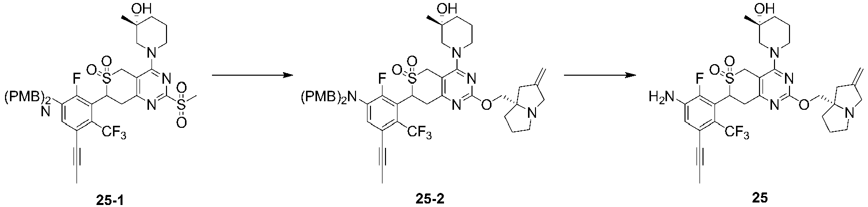

The compound **25** was prepared using a method similar to that used for compound **1** in **Example 1.** LC/MS(ESI): m/z =664.2[M+H]⁺.

### Example 50

### (7S)-7-(3-amino-2-fluoro-5-(prop-1-yn-1-yl)-6-(trifluoromethyl)phenyl)-4-((R)-3-hyd roxy-3-methylpiperidin-1-yl)-2-(((S)-2-(difluoromethylene)tetrahydro-1H-pyrrolazin-7a(5H)-yl)methoxy)-7,8-dihydro-5H-thiopyrano[4,3-d]pyrimidine-6,6-dioxide (25A)

### (7R)-7-(3-amino-2-fluoro-5-(prop-1-yn-1-yl)-6-(trifluoromethyl)phenyl)-4-((R)-3-hyd roxy-3-methylpiperidin-1-yl)-2-(((S)-2-(difluoromethylene)tetrahydro-1H-pyrrolazin-7a(5H)-yl)methoxy)-7,8-dihydro-5H-thiopyrano[4,3-d]pyrimidine-6,6-dioxide (25B)

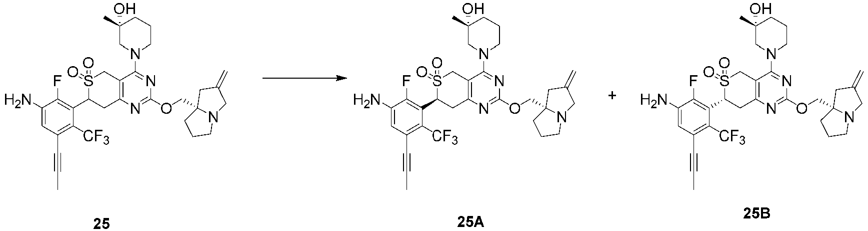

Compounds **25A** and **25B** were prepared using a similar method to that used for compounds **2A** and **2B** in **Example 2** (with compound **25** as the starting material). LC/MS(ESI): m/z =664.2[M+H]⁺.

### Example 51

### 7-(3-amino-2-fluoro-5-(prop-1-yn-1-yl)-6-(trifluoromethyl)phenyl)-4-((R)-3-hydroxy-3-methylpiperidin-1-yl)-2-(((S)-2-(fluoromethylene)tetrahydro-1H-pyrrolazin-7a(5H)-yl)methoxy)-7,8-dihydro-5H-thiopyrano[4,3-d]pyrimidine-6,6-dioxide (26)

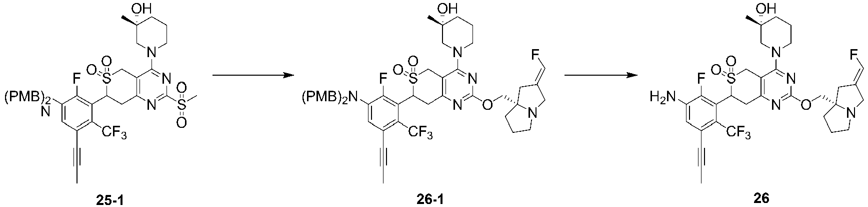

Compound **26** was prepared using a method similar to that used for compound **1** in **Example 1.** LC/MS(ESI): m/z =682.2[M+H]⁺.

### Example 52

### (7S)-7-(3-amino-2-fluoro-5-(prop-1-yn-1-yl)-6-(trifluoromethyl)phenyl)-4-((R)-3-hyd roxy-3-methylpiperidin-1-yl)-2-(((S)-2-(difluoromethylene)tetrahydro-1H-pyrrolazin-7a(5H)-yl)methoxy)-7,8-dihydro-5H-thiopyrano[4,3-d]pyrimidine-6,6-dioxide (26A)

### (7R)-7-(3-amino-2-fluoro-5-(prop-1-yn-1-yl)-6-(trifluoromethyl)phenyl)-4-((R)-3-hyd roxy-3-methylpiperidin-1-yl)-2-(((S)-2-(difluoromethylene)tetrahydro-1H-pyrrolazin-7a(5H)-yl)methoxy)-7,8-dihydro-5H-thiopyrano[4,3-d]pyrimidine-6,6-dioxide (26B)

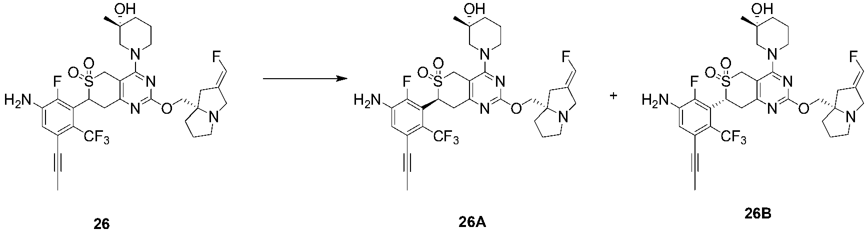

Compounds **26A** and **26B** were prepared using a similar method to that used for compounds **2A** and **2B** in **Example 2** (with compound **26** as the starting material). LC/MS(ESI): m/z =682.2[M+H]⁺.

### Example 53

### 7-(3-amino-2-fluoro-5-(prop-1-yn-1-yl)-6-(trifluoromethyl)phenyl)-4-((R)-3-hydroxy-3-methylpiperidin-1-yl)-2-(((S)-2-(difluoromethylene)tetrahydro-1H-pyrrolazin-7a(5 H)-yl)methoxy)-7,8-dihydro-5H-thiopyrano[4,3-d]pyrimidine-6,6-dioxide (27)

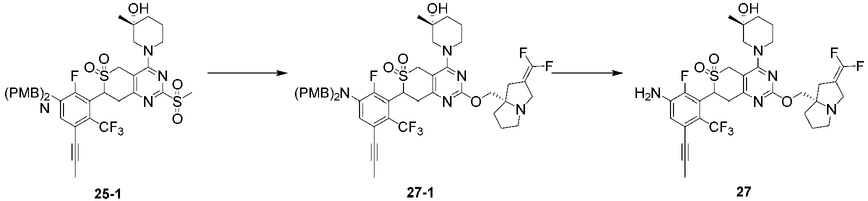

Compound **27** was prepared using a method similar to that used for compound **1** in Example **1.** LC/MS(ESI): m/z =700.2[M+H]⁺.

### Example 54

### (7S)-7-(3-amino-2-fluoro-5-(prop-1-yn-1-yl)-6-(trifluoromethyl)phenyl)-4-((R)-3-hyd roxy-3-methylpiperidin-1-yl)-2-(((S)-2-(difluoromethylene)tetrahydro-1H-pyrrolazin-7a(5H)-yl)methoxy)-7,8-dihydro-5H-thiopyrano[4,3-d]pyrimidine-6,6-dioxide (27A)

### (7R)-7-(3-amino-2-fluoro-5-(prop-1-yn-1-yl)-6-(trifluoromethyl)phenyl)-4-((R)-3-hyd roxy-3-methylpiperidin-1-yl)-2-(((S)-2-(difluoromethylene)tetrahydro-1H-pyrrolazin-7a(5H)-yl)methoxy)-7,8-dihydro-5H-thiopyrano[4,3-d]pyrimidine-6,6-dioxide (27B)

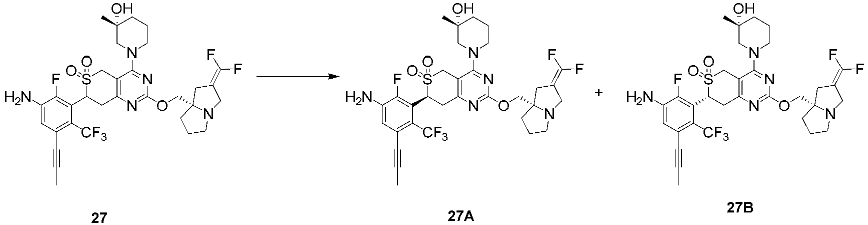

Compounds **27A** and **27B** were prepared using a similar method to that used for compounds **2A** and **2B** in **Example 2** (with compound **27** as the starting material). LC/MS(ESI): m/z =700.2 [M+H]⁺.

### Example 55

### 7-(3-amino-2-fluoro-5-(prop-1-yn-1-yl)-6-(trifluoromethyl)phenyl)-2-(((2R,7AS)-2-fl uorotetrahydro-1H-pyrrolazin-7a(5H)-yl)methoxy)-4-((R)-3-hydroxy-3-methylpiperid in-1-yl)-7,8-dihydro-5H-thiopyrano[4,3-d]pyrimidine 6,6-dioxide (28)

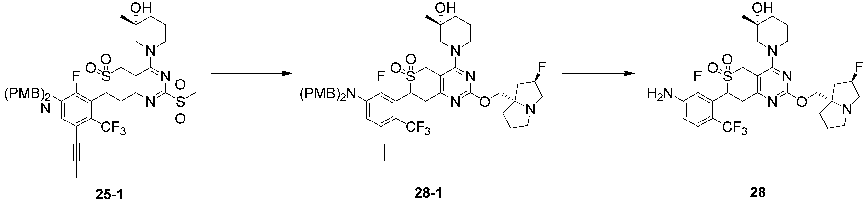

Compound **28** was prepared using a method similar to that used for compound **1** in **Example 1.** LC/MS(ESI): m/z =670.2[M+H]⁺.

### Example 56

### (7S)-7-(3-amino-2-fluoro-5-(prop-1-yn-1-yl)-6-(trifluoromethyl)phenyl)-2-(((2R,7AS) -2-fluorotetrahydro-1H-pyrrolazin-7a(5H)-yl)methoxy)-4-((R)-3-hydroxy-3-methylpi peridin-1-yl)-7,8-dihydro-5H-thiopyrano[4,3-d]pyrimidine 6,6-Dioxide (28A)

### (7R)-7-(3-amino-2-fluoro-5-(prop-1-yn-1-yl)-6-(trifluoromethyl)phenyl)-2-(((2R,7AS) -2-fluorotetrahydro-1H-pyrrolazin-7a(5H)-yl)methoxy)-4-((R)-3-hydroxy-3-methylpi peridin-1-yl)-7,8-dihydro-5H-thiopyrano[4,3-d]pyrimidine 6,6-Dioxide (28B)

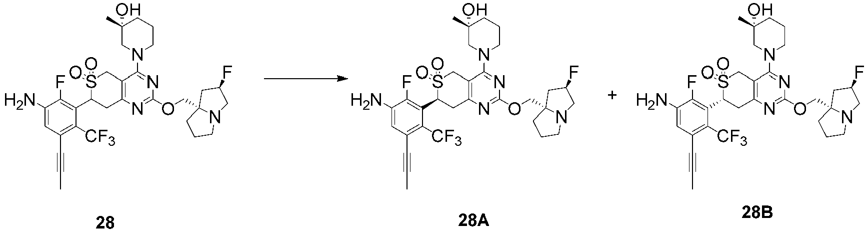

Compounds **28A** and **28B** were prepared using a similar method to that used for compounds **2A** and **2B** in **Example 2** (with compound **28** as the starting material). LC/MS(ESI): m/z =670.2[M+H]⁺.

### Example 57

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (29)

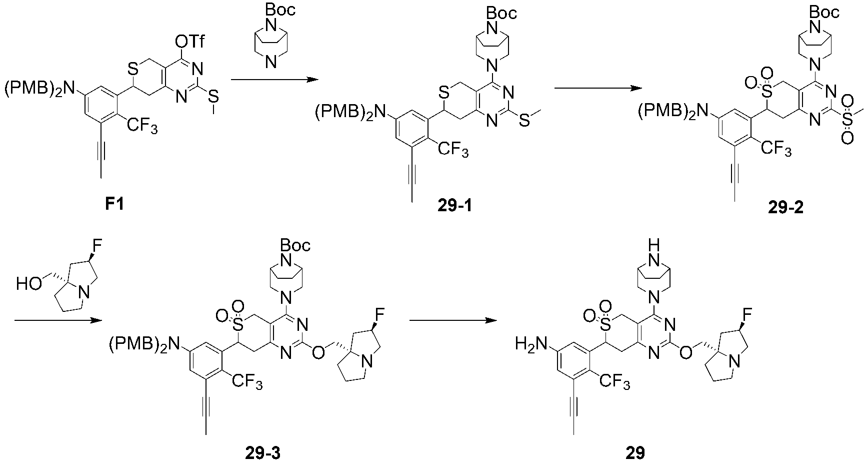

### Step A

Compound **F1** (82 mg, 105.02 µmol) and 8-tert-butoxycarbonyl-3,8-diazabicyclo[3.2.1]octane (26.63 mg, 126.03 µmol) were dissolved in N,N-dimethylformamide (2 mL), and diisopropylethylamine (40.72 mg, 315.07 µmol) was added. The reaction mixture was heated to 100°C and stirred for 1 hour. After cooling, the organic solvent was removed under reduced pressure. The crude product was purified by thin-layer chromatography to obtain the compound **29-1.** LC/MS(ESI): m/z = 846[M+H]⁺.

### Step B

Compound **29-1** (71 mg, 84.96 µmol) was dissolved in dichloromethane (2 mL), and m-chloroperoxybenzoic acid (34.50 mg, 169.92 µmol) was added. The reaction mixture was stirred at 20 °C for 3 hours. The organic solvent was removed under reduced pressure, and the crude product was purified by thin-layer chromatography to obtain the compound **29-2.** LC/MS(ESI): m/z =910[M+H]⁺.

### Step C

Under ice-water bath conditions, compound ((2R,7AS)-2-fluorotetrahydro-1H-pyrrolazin-7a(5H)-yl)methanol (12.09 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **29-2** (53 mg, 58.42 µmol) was added. The reaction mixture was stirred for 2 hours under ice-water bath conditions. The organic solvent was removed under reduced pressure, and the crude product was purified by thin-layer chromatography to obtain compound **29-3.** LC/MS(ESI): m/z =989[M+H]⁺.

### Step D

Compound **29-3** (42 mg, 42.78 µmol) was dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain the compound **29.** LC/MS(ESI): m/z =648.2[M+H]⁺. ¹H-NMR (DMSO-*d*₆) *δ* 1.78-2.39 (m, 12H), 2.38-2.79 (m, 3H), 3.13-3.44 (m, 3H), 3.56-3.62 (m, 2H), 3.71-4.78 (m, 11H), 5.92 (s, 2H), 6.69 (d, 1H), 6.88 (d, 1H).

### Example 58

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (29A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (29B)

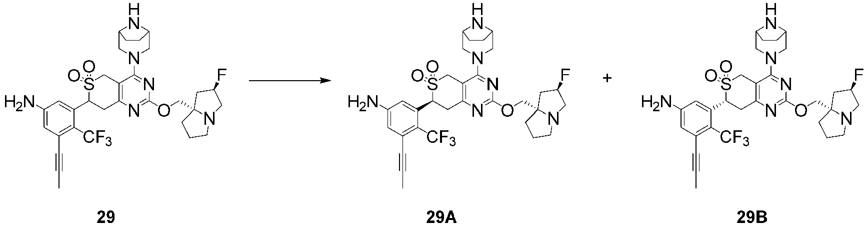

Compound **29** was purified by supercritical liquid chromatography (SFC) to obtain **29A** and **29A** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =648.2[M+H]⁺).

### Example 59

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (30)

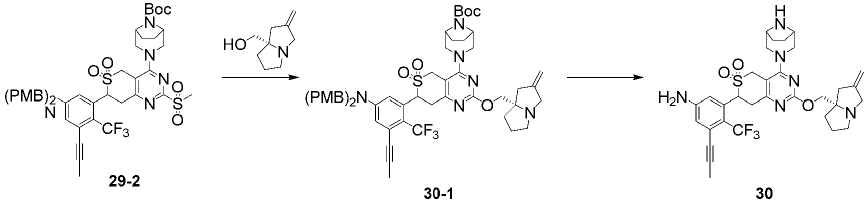

Compound **30** was prepared using a method similar to that used for compound **29** in **Example 57.** LC/MS(ESI): m/z =642.3[M+H]⁺.

### Example 60

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (30A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (30B)

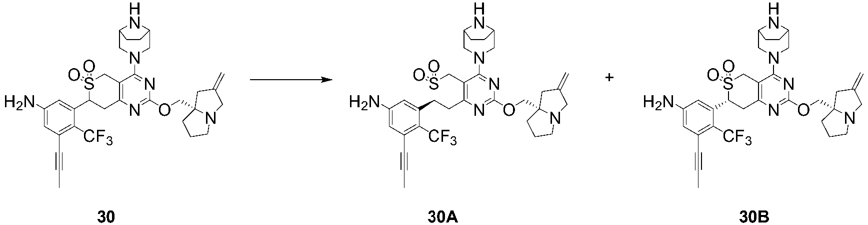

Compound **30** was purified by supercritical liquid chromatography (SFC) to obtain **30A** and **30B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =642.3[M+H]⁺).

### Example 61

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (31)

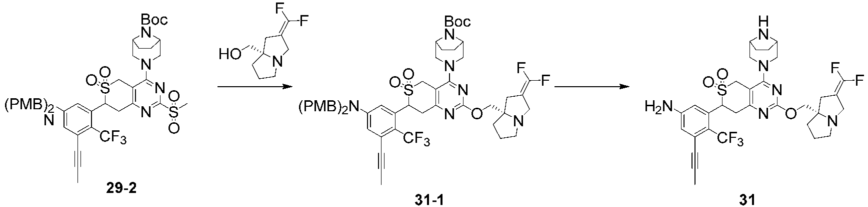

Compound **31** was prepared using a method similar to that used for compound **29** in Example 57. LC/MS(ESI): m/z =679.2[M+H]⁺. ¹H-NMR (DMSO-*d*₆) *δ* 1.99 (m, 4H), 2.10-2.27 (m, 3H), 2.84 (d, 1H), 2.98 (d, 1H), 3.29-3.64 (m, 3H), 3.84 (d, 1H), 4.09-4.33-4.76 (m, 13H), 5.92 (s, 2H), 6.69 (d, 1H), 6.88 (d, 1H).

### Example 62

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (31A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (31B)

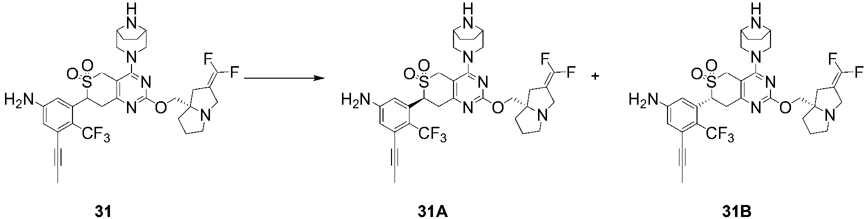

Compound **31** was purified by supercritical liquid chromatography (SFC) to obtain **31A** and **31B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =679.2[M+H]⁺).

### Example 63

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (32)

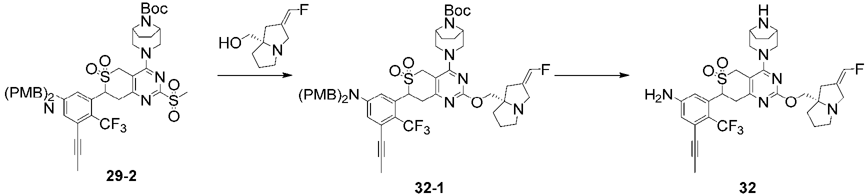

Compound **32** was prepared using a method similar to that used for compound **29** in **Example 57.** LC/MS(ESI): m/z =661.2[M+H]⁺. ¹H-NMR (DMSO-*d*₆) *δ* 1.78-2.39 (m, 10H), 2.38-2.79 (m, 3H), 3.13-3.44 (m, 3H), 3.56-3.62 (m, 2H), 3.71-4.78 (m, 11H), 5.92 (s, 2H), 6.59 -6.87 (m, 3H).

### Example 64

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (32A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (32B)

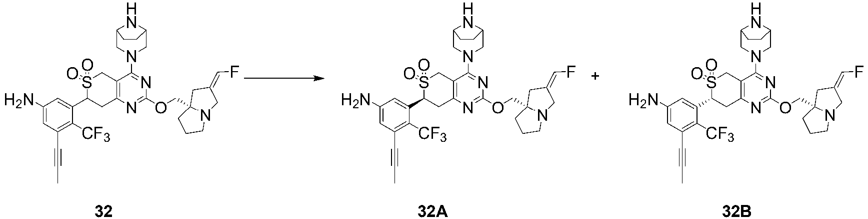

Compound **32** was purified by supercritical liquid chromatography (SFC) to obtain **32A** and **32B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =661.2[M+H]⁺).

### Example 65

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (33)

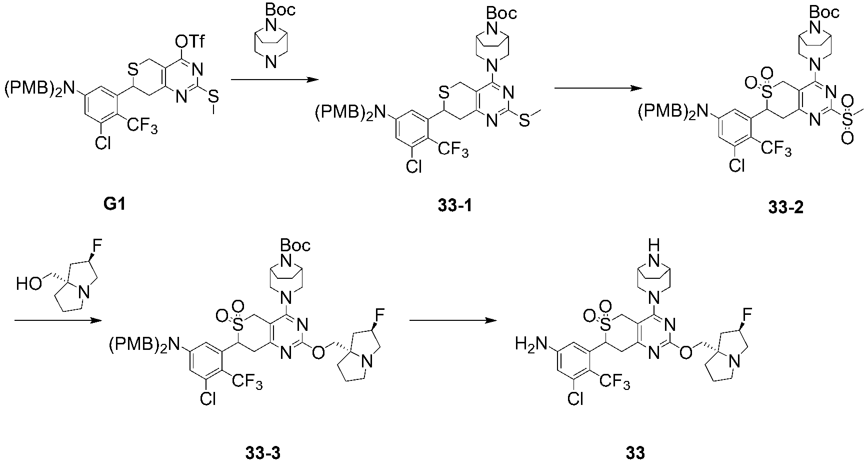

### Step A

Compound **G1** (82 mg, 105.02 µmol) and 8-tert-butoxycarbonyl-3,8-diazabicyclo[3.2.1]octane (26.63 mg, 126.03 µmol) were dissolved in N,N-dimethylformamide (2 mL), and diisopropylethylamine (40.72 mg, 315.07 µmol) was added. The reaction mixture was heated to 100°C and stirred for 1 hour. After cooling, the organic solvent was removed under reduced pressure. The crude product was purified by thin-layer chromatography to obtain the compound **33-1.** LC/MS(ESI): m/z = 842[M+H]⁺.

### Step B

Compound **33-1** (71 mg, 84.96 µmol) was dissolved in dichloromethane (2 mL), and m-chloroperoxybenzoic acid (34.50 mg, 169.92 µmol) was added. The reaction mixture was stirred at 20 °C for 3 hours. The organic solvent was removed under reduced pressure, and the crude product was purified by thin-layer chromatography to obtain the compound **33-2.** LC/MS(ESI): m/z =906[M+H]⁺.

### Step C

Under ice-water bath conditions, compound ((2R,7AS)-2-fluorotetrahydro-1H-pyrrolazin-7a(5H)-yl)methanol (12.09 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **33-2** (53 mg, 58.42 µmol) was added. The reaction mixture was stirred for 2 hours under ice-water bath conditions. The organic solvent was removed under reduced pressure, and the crude product was purified by thin-layer chromatography to obtain compound **33-3.** LC/MS(ESI): m/z =985.1[M+H]⁺.

### Step D

Compound **33-3** (42 mg, 42.78 µmol) was dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain compound **33.** LC/MS(ESI): m/z =645.2[M+H]⁺. ¹H-NMR (DMSO-*d*₆) *δ* 1.78-2.39 (m, 12H), 2.38-2.79 (m, 3H), 3.13-3.44 (m, 3H), 3.56-3.62 (m, 2H), 3.71-4.78 (m, 11H), 6.20 (s, 2H), 6.69(d, 1H), 6.89 (d, 1H).

### Example 66

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (33A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (33B)

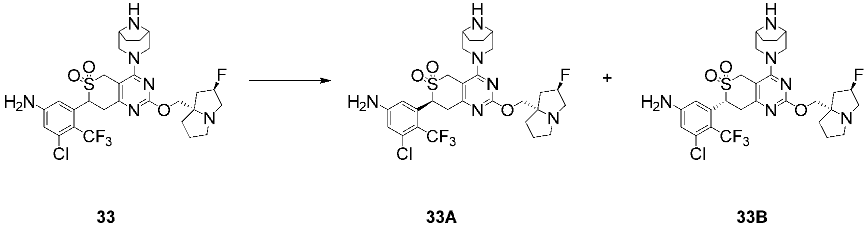

Compound **33** was purified by supercritical liquid chromatography (SFC) to obtain **33A** and **33B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =642.7[M+H]⁺).

### Example 67

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (34)

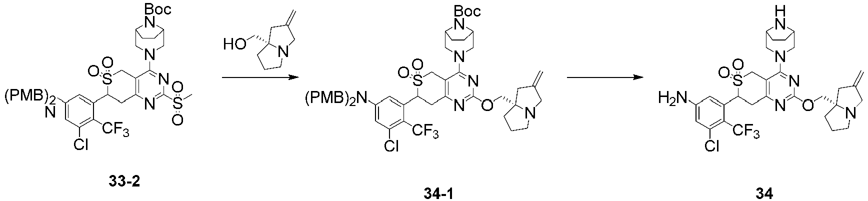

Compound **34** was prepared using a method similar to that used for compound **33** in **Example 65.** LC/MS(ESI): m/z =638.2[M+H]⁺.

### Example 68

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (34A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (34B)

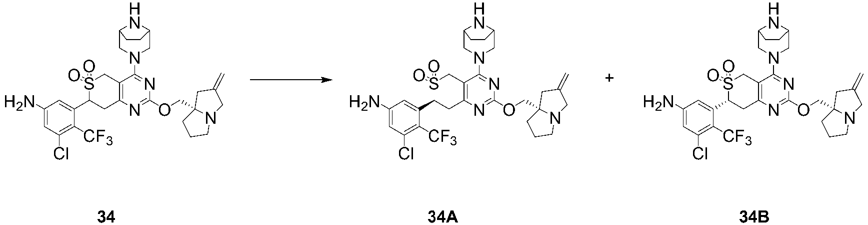

Compound **34** was purified by supercritical liquid chromatography (SFC) to obtain **34A** and **34B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =638.2[M+H]⁺).

### Example 69

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (35)

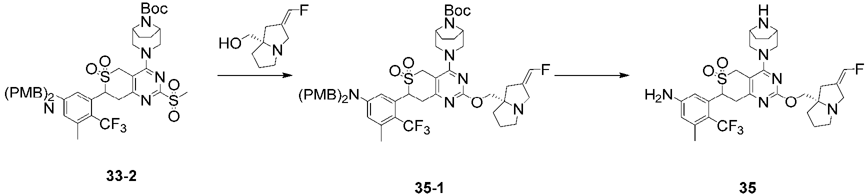

Compound **35** was prepared using a method similar to that used for compound **33** in Example **65.** LC/MS(ESI): m/z =637.2[M+H]⁺. ¹H-NMR (DMSO-*d*₆) *δ* 1.67-2.39 (m, 10H), 2.38-2.79 (m, 3H), 3.13-3.44 (m, 3H), 3.56-3.62 (m, 2H), 3.71-4.78 (m, 11H), 5 5.78 (s, 2H), 6.43(s, 1H), 6.74 (s, 1H), 6.76-7.19(m, 1H).

### Example 70

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (35A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (35B)

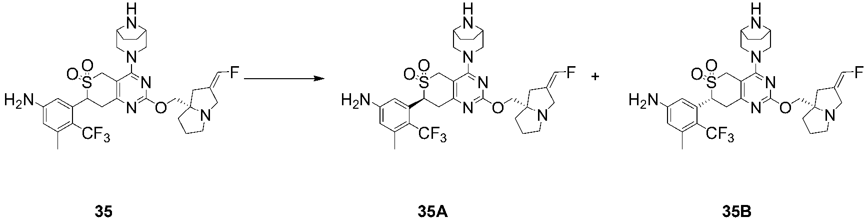

Compound **35** was purified by supercritical liquid chromatography (SFC) to obtain **35A** and **35B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =642.7[M+H]⁺).

### Example 71

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (36)

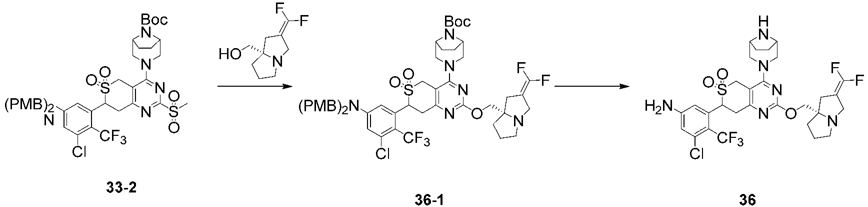

Compound **36** was prepared using a method similar to that used for compound **33** in **Example 65.** LC/MS(ESI): m/z =619.2[M+H]⁺. ¹H-NMR (DMSO-*d*₆) *δ* 1.60-2.39 (m, 9H), 2.38-2.79 (m, 3H), 3.13-3.44 (m, 3H), 3.56-3.62 (m, 2H), 3.71-4.78 (m, 11H), 6.31 (s, 2H), 6.74 (d, 1H), 6.92 (d, 1H).

### Example 72

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (36A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (36B)

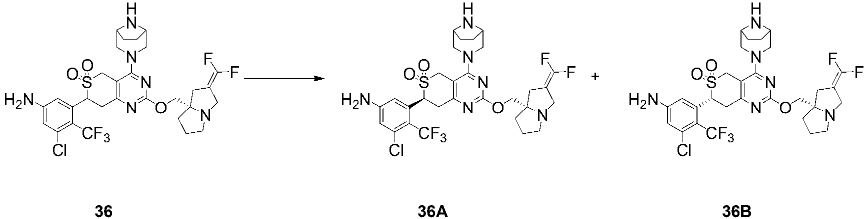

Compound **36** was purified by supercritical liquid chromatography (SFC) to obtain **36A** and **36B** (column: Chiralpak IG-3: 3 µm, 0.46 cm × 5 cm; mobile phase: A (CO2) and B (EtOH, containing 0.1% isopropylamine); gradient: B% = 5-50%, 3 min; flow rate: 3.4 mL/min; wavelength: 220 nm; pressure: 1800, LC/MS(ESI): m/z =619.2[M+H]⁺).

### Example 73

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (37)

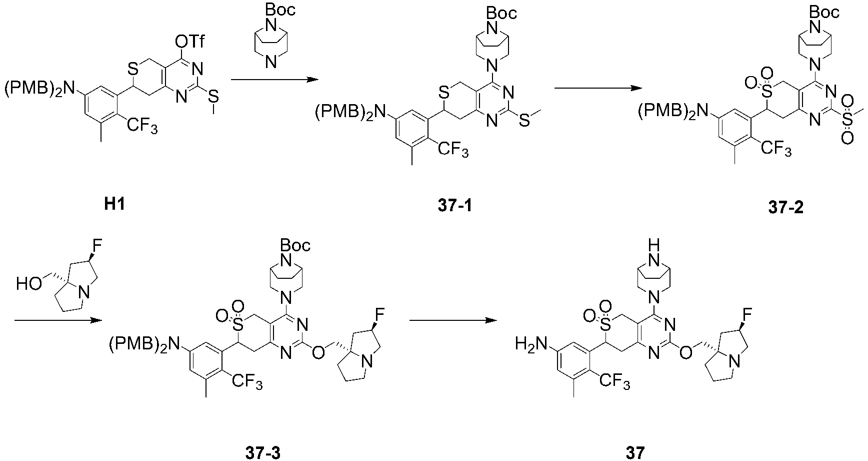

### Step A

Compound **H1** (80 mg, 105.02 µmol) and 8-tert-butoxycarbonyl-3,8-diazabicyclo[3.2.1]octane (26.63 mg, 126.03 µmol) were dissolved in N,N-dimethylformamide (2 mL), and diisopropylethylamine (40.72 mg, 315.07 µmol) was added. The reaction mixture was heated to 100°C and stirred for 1 hour. After cooling, the organic solvent was removed under reduced pressure. The crude product was purified by thin-layer chromatography to obtain the compound **37-1.** LC/MS(ESI): m/z = 822[M+H]⁺.

### Step B

Compound **37-1** (70 mg, 84.96 µmol) was dissolved in dichloromethane (2 mL), and m-chloroperoxybenzoic acid (34.50 mg, 169.92 µmol) was added. The reaction mixture was stirred at 20 °C for 3 hours. The organic solvent was removed under reduced pressure, and the crude product was purified by thin-layer chromatography to obtain the compound **37-2.** LC/MS(ESI): m/z =886[M+H]⁺.

### Step C

Under ice-water bath conditions, compound ((2R,7AS)-2-fluorotetrahydro-1H-pyrrolazin-7a(5H)-yl)methanol (12.09 mg, 75.94 µmol) was dissolved in anhydrous toluene (1 mL), and sodium tert-butoxide (7.30 mg, 75.94 µmol) was added. The reaction mixture was stirred for 30 minutes, and then a toluene (1 mL) solution of compound **37-2** (52 mg, 58.42 µmol) was added. The reaction mixture was stirred for 2 hours under ice-water bath conditions. The organic solvent was removed under reduced pressure, and the crude product was purified by thin-layer chromatography to obtain compound **37-3.** LC/MS(ESI): m/z =965.1[M+H]⁺.

### Step D

Compound **37-2** (41 mg, 42.78 µmol) was dissolved in anhydrous dichloromethane (2 mL), and trifluoroacetic acid (1 mL) was added. The reaction mixture was stirred at 20 °C for 2 hours. The solvent was removed under reduced pressure, and the crude product was purified by high performance liquid chromatography to obtain the compound**37**. LC/MS(ESI): m/z =625.3[M+H]⁺. ¹H-NMR (DMSO-*d*₆) *δ* 1.78-2.39 (m, 12H), 2.38-2.79 (m, 3H), 3.13-3.44 (m, 3H), 3.56-3.62 (m, 2H), 3.71-4.86 (m, 11H), 5.77(s, 2H), 6.41 (s, 1H), 6.74 (s, 1H).

### Example 74

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (37A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (37B)

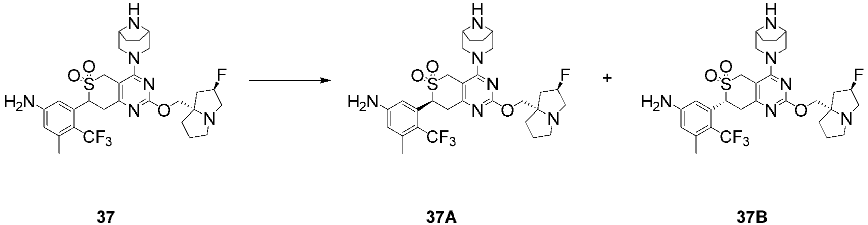

Compound **37** was purified by supercritical liquid chromatography (SFC) to obtain **37A** and **37B** (column: Chiralpak IG-3: 3 µm, 0.46 cm×5 cm; mobile phase: A (CO₂) and B (EtOH, containing 0.1% isopropylamine); gradient: B%=5-50%, 3 min; flow rate: 3.4 mL/min; wavelength: 220 nm; pressure: 1800, LC/MS(ESI): m/z =642.7[M+H]⁺).

### Example 75

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (38)

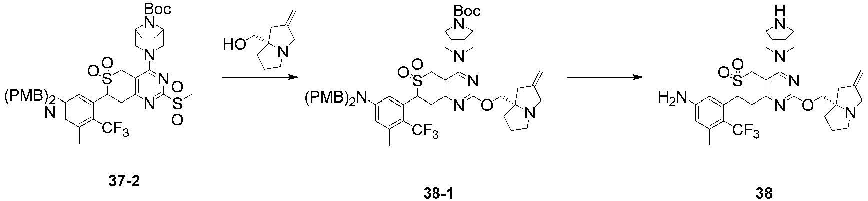

Compound **38** was prepared using a method similar to that used for compound **37** in **Example 73.** LC/MS(ESI): m/z =619.2[M+H]⁺.

### Example 76

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (38A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (38B)

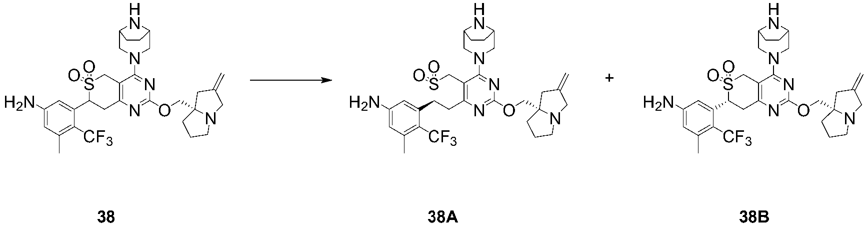

Compound **38** was purified by supercritical liquid chromatography (SFC) to obtain **38A** and **38B** (column: Chiralpak IG-3: 3 µm, 0.46 cm×5 cm; mobile phase: A (CO₂) and B (EtOH, containing 0.1% isopropylamine); gradient: B%=5-50%, 3 min; flow rate: 3.4 mL/min; wavelength: 220 nm; pressure: 1800, LC/MS(ESI): m/z =619.2[M+H]⁺).

### Example 77

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (39)

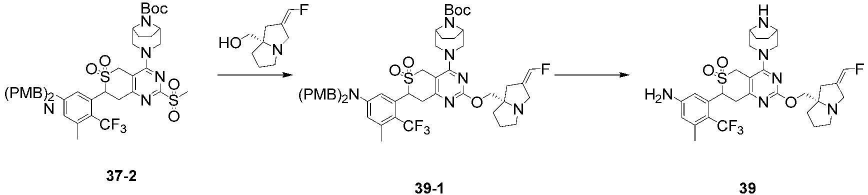

Compound **39** was prepared using a method similar to that used for compound **37** in **Example 73.** LC/MS(ESI): m/z =637.2[M+H]⁺. ¹H-NMR (DMSO-*d*₆) *δ* 1.60-2.39 (m, 10H), 2.38-2.79 (m, 3H), 3.13-3.44 (m, 3H), 3.56-3.62 (m, 2H), 3.71-4.86 (m, 11H), 5.76 (s, 2H), 6.41 (s, 1H), 6.58-6.88 (m, 2H).

### Example 78

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (39A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (39B)

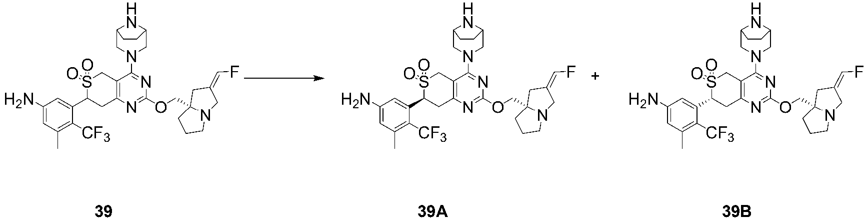

Compound **39** was purified by supercritical liquid chromatography (SFC) to obtain **39A** and **39B** (column: Chiralpak IG-3: 3 µm, 0.46 cm×5 cm; mobile phase: A (CO₂) and B (EtOH, containing 0.1% isopropylamine); gradient: B%=5-50%, 3 min; flow rate: 3.4 mL/min; wavelength: 220 nm; pressure: 1800, LC/MS(ESI): m/z =637.2[M+H]⁺).

### Example 79

### 7-(3-amino-2-fluoro-5-methyl-6-(trifluoromethyl)phenyl)-4-(1R,5S)-3,8-diazabicyclo [3.2.1]octane-3-yl)-2-(((2R,7AS)-2-fluorotetrahydro-1H-pyrrolazin-7a(5H)-yl)methox y)-7,8-dihydro-5H-thiopyrano[4,3-d]pyrimidine-6,6-dioxide (40)

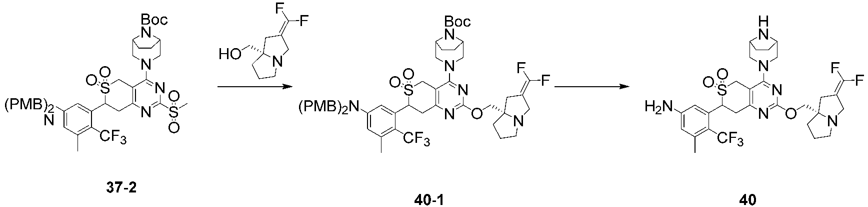

Compound **40** was prepared using a method similar to that used for compound **37** in **Example 73.** LC/MS(ESI): m/z ==655.2[M+H]⁺. ¹H-NMR (DMSO-*d*₆) *δ* 1.66-2.39 (m, 10H), 2.38-2.79 (m, 3H), 3.13-3.44 (m, 3H), 3.56-3.62 (m, 2H), 3.71-4.88 (m, 11H), 5.76 (s, 2H), 6.41 (s, 1H), 6.74 (s, 1H).

### Example 80

### (7S)-7-(3-amino-2-fluoro-5-methyl-6-(trifluoromethyl)phenyl)-4-((1R,5S)-3,8-diazabi cyclo[3.2.1]octane-3-yl)-2-(((2R,7AS)-2-fluorotetrahydro-1H-pyrrolazin-7a(5H)-yl)m ethoxy)-7,8-dihydro-5H-thiopyrano[4,3-d]pyrimidine 6,6-Dioxide (40A)

### (7R)-7-(3-amino-2-fluoro-5-methyl-6-(trifluoromethyl)phenyl)-4-((1R,5S)-3,8-diazab icyclo[3.2.1]octane-3-yl)-2-(((2R,7AS)-2-fluorotetrahydro-1H-pyrrolazin-7a(5H)-yl) methoxy)-7,8-dihydro-5H-thiopyrano[4,3-d]pyrimidine 6,6-Dioxide (40B)

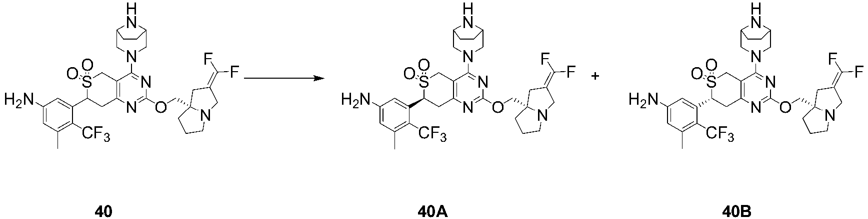

Compound **40** was purified by supercritical liquid chromatography (SFC) to obtain **40A** and **40B** (column: Chiralpak IG-3: 3 µm, 0.46 cm × 5 cm; mobile phase: A (CO₂) and B (EtOH, containing 0.1% isopropylamine); gradient: B%=5-50%, 3 min; flow rate: 3.4 mL/min; wavelength: 220 nm; pressure: 1800, LC/MS(ESI): m/z =655.2[M+H]⁺).

### Example 81 Bioactivity Test

### Experiment Example 1. KRAS Inhibitory Activity Test

1. Experimental Objective: To screen compounds that can effectively inhibit the binding of KRasG12D to GTP using the TR-FRET method.
2. Reagent Preparation:
   a. Storage Reagents:

### 1) KRAS Nucleotide Exchange Buffer

Prepare a 1L solution by mixing 20mL of 1000mM HEPES, 20mL of 500mM EDTA, 10mL of 5M sodium chloride, 0.1mL of 100% Tween 20, and 949.9mL of water. Sterilize by filtration and store at 4°C.

### 2) KRAS Experimental Buffer

Prepare a 1L solution by mixing 20mL of 1000mM HEPES, 10mL of 1000mM magnesium chloride, 30mL of 5M sodium chloride, 0.05mL of 100% Tween 20, and 939.95mL of water. Sterilize by filtration and store at 4°C. 3) KRAS/Bodipy GDP/Tb-SA Mixture: Mix 9.5 µL of 95 µM KRasG12D protein with 440.5 µL of KRAS nucleotide exchange buffer. Incubate at room temperature for 1 hour. Then, mix with 8.4 µL of 17.9 µM Tb-SA, 1.8 µL of 5 mM Bodipy GDP, and 9539.8 µL of KRAS experimental buffer to prepare a 1 L solution. After mixing, incubate at room temperature for 6 hours and store at -80°C.

### b. Experimental Reagents:

1) KRAS Enzyme Solution: Mix 73.3 µL of KRAS/Bodipy GDP/Tb-SA mixture with 2126.7 µL of KRAS experimental buffer to prepare a 2200 µL solution.
2) SOS/GTP Mixture

c. Experimental Procedure: Mix 1.59 µL of 166 µM SOS protein with 198 µL of 100 mM... GTP and 0.41 µL KRAS buffer were prepared to a 2200 µL solution. The stock solution concentration of the control compound was 1 mM, and the stock solution concentration of the test compound was 10 mM. 9 µL of the control compound and the test compound were transferred to a 384-LDV plate. The compound on the LDV plate was 3-fold diluted 10-point using Bravo. 9 nL of the compound on the LDV plate was transferred to the experimental plate using ECHO. 3 µL of a 3 nM Kras/0.5 nM TB-SA/30 nM BodipyGDP mixture and 3 µL of Ras buffer were added to each well of the experimental plate using a Dragonfly autopilot. The plate was centrifuged at 1000 rpm for 1 minute. The plate was incubated at room temperature for 1 hour. 3 µL of 120 nM SOS/9 mM KRAS buffer was added to each well of the experimental plate using a Dragonfly autopilot. The GTP mixture was centrifuged at 1000 rpm for 1 minute; the plate was incubated at room temperature for 1 hour; the plate was read and the data were recorded using Envision; the data were analyzed using Excel and Xlfit to calculate the IC₅₀of the test compound. "++++" indicates IC₅₀≤ 10 nM; "+++" indicates 10 nM < IC₅₀≤ 100 nM; "++" indicates 100 nM < IC₅₀≤ 2000 nM; "+" indicates 2000 nM < IC₅₀.

**Table 1 shows the IC₅₀ values of the compounds for inhibiting KRAS enzymes.**

| NO | G12D | NO | G12D | NO | G12D |
|---|---|---|---|---|---|
| 1 | ++++ | 8B | +++ | 30 | ++++ |
| 1A | ++++ | 9 | ++++ | 30A | ++++ |
| 1B | +++ | 9A | ++++ | 31 | ++++ |
| 2 | ++++ | 10 | ++++ | 31A | ++++ |
| 2A | +++ | 10A | ++++ | 32 | ++++ |
| 2B | ++++ | 11 | ++++ | 32A | ++++ |
| 3 | ++++ | 11A | ++++ | 33 | ++++ |
| 3A | +++ | 18 | ++++ | 33A | ++++ |
| 3B | ++++ | 18A | ++++ | 34 | ++++ |
| 4 | ++++ | 19 | ++++ | 34A | ++++ |
| 4A | ++++ | 19A | ++++ | 35 | ++++ |
| 4B | +++ | 20 | ++++ | 35A | ++++ |
| 5 | ++++ | 20A | ++++ | 36 | ++++ |
| 5A | +++ | 21 | ++++ | 35A | ++++ |
| 5B | ++++ | 21A | ++++ | 36 | ++++ |
| 6 | ++++ | 22 | ++++ | 36A | ++++ |
| 6A | ++++ | 22A | ++++ | 37 | ++++ |
| 6B | +++ | 23 | ++++ | 37A | ++++ |
| 7 | ++++ | 23A | ++++ | 38 | ++++ |
| 7A | ++++ | 24 | ++++ | 38A | ++++ |
| 7B | +++ | 24A | ++++ | 39 | ++++ |
| 8 | ++++ | 29 | ++++ | 39A | ++++ |
| 8A | ++++ | 29A | ++++ | 40 | ++++ |

### Example 2. Cellular p-ERK Inhibition Assay

Compounds that effectively inhibit p-ERK in AsPC-1 (G12D), A549 (G12S), HCT116 (G13D), NCI-H358 (G12C), NCI-H460 (Q61H), NCI-H727 (G12V), MKN1 (WTdep), and PSN-1 (G12R) cells were screened using the HTRF method. Cells were seeded in clear 96-well cell culture plates, with 80 µL of cell suspension per well, containing 8000 cells per well. The plates were incubated overnight at 37°C using a CO2 incubator. 2 µL of the compound was added to 78 µL of cell culture medium, mixed well, and then 20 µL of the compound solution was added to the corresponding wells of the cell culture plate. The plates were returned to the CO₂ incubator for another hour. After incubation, the cell supernatant was discarded, and 50 µL of the supernatant was added to the cell culture plate. Add 1X cell lysis buffer to each well and incubate at room temperature with shaking for 30 minutes. Dilute Phospho-ERK1/2 Eu Cryptate antibody and Phospho-ERK1/2 d2 antibody 20-fold using detection buffer. Transfer 16 µL of cell lysis supernatant to each well in a new 384 white microplate, then add 2 µL of Phospho-ERK1/2 Eu Cryptate antibody diluent and 2 µL of Phospho-ERK1/2 d2 antibody diluent. Incubate at room temperature for at least 4 hours. After incubation, read the HTRF excitation (320 nm) and emission (615 nm, 665 nm) using a multilabel analyzer. Calculate the IC₅₀ of the analyte. "++++" indicates IC₅₀≤ 10 nM; "+++" indicates 10 nM < IC₅₀≤ 100 nM; "++" indicates 100 nM < IC₅₀≤ 2000 nM; "+" indicates 2000 nM < IC₅₀.

**Table 2. IC₅₀ (nM) of the compounds on the inhibition of p-ERK in tumor cells.**

| N O | AsPC-1 | A54 9 | HCT11 6 | NCI-H35 8 | NCI-H46 0 | NCI-H72 7 | MKN 1 | PSN-1 |
|---|---|---|---|---|---|---|---|---|
| 1 | ++++ | +++ + | ++++ | ++++ | ++++ | ++++ | ++++ | ++ |
| 2 | ++++ | | ++++ | | | | | ++ |
| 3 | ++++ | | ++++ | | | | | ++ |
| 4 | ++++ | | ++++ | | | | | ++ |
| 5 | ++++ | +++ + | ++++ | ++++ | ++++ | ++++ | ++++ | ++ |
| 6 | ++++ | +++ + | ++++ | ++++ | ++++ | ++++ | ++++ | ++ |
| 7 | ++++ | | ++++ | | | | | ++ |
| 8 | ++++ | | ++++ | | | | | ++ |

Although the present invention has been described in detail above, those skilled in the art will understand that various modifications and changes can be made to the present invention without departing from its spirit and scope. The scope of the present invention is not limited to the detailed description above, but should be attributed to the claims.

## Claims

1. A compound having the general formula (I), its stereoisomers, pharmaceutically acceptable salts, polymorphs, or isomers, wherein the compound having the general formula (I) has the following structure: 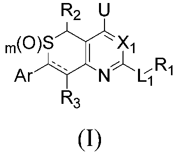
Each L1 is selected from a single bond, OC₀₋₆ alkyl, NHC₀₋₆ alkyl, C₁₋₆ alkyl, COC₀₋₆ alkyl, or SC₀₋₆ alkyl;
Each Ar is independently selected from 6-10 membered aromatic groups or 5-12 membered heteraromatic groups in each occurrence, and the heteroaryl group in each occurrence independently comprises 1, 2, 3, or 4 heteroatoms selected from N, O, or S, wherein the heteroaryl group is optionally substituted by one or more G¹ atoms;
Each X₁ is independently selected from N, CR₄;
Each R₂, R₃, R₄ is independently selected from H, D, CN, halogen, C₁₋₆ alkyl;
Each R ₁ is independently selected from H, D, C₁₋₆ alkyl, C₂₋₆ alkenyl, C₂₋₆ ynyl, CN, C₃₋₆ carbocyclic, 3-10 membered heterocycles, 4-10 membered heterofused rings, 5-12 membered spirocyclic groups; the 3-10 membered heterocycles, 4-10 membered heterofused rings, and 5-12 membered spirocyclic groups independently contain 1, 2, 3, or 4 heteroatoms selected from N, O, or S each time they appear; each R1 is independently and optionally substituted or unsubstituted by 1, 2, 3, 4, 5, or 6 substituents selected from deuterium, halogens, C1-6 alkyl, -C1-6 alkoxy, oxo, C1-6 alkyl, C3-6 carbocyclic, and 3-10 membered heterocycles;
Each U is selected from a single bond, 3-8 membered cycloalkyl, 3-8 membered heterocycloalkyl, 5-12 membered fused cycloalkyl, 5-12 membered fused heterocycloalkyl , 5-12 membered spiro cycloalkyl, 5-12 membered spiro heterocycloalkyl, 3-8 membered cycloalkyl, 3-8 membered heterocycloalkyl, 5-12 membered fused cycloalkyl, 5-12 membered fused heterocycloalkyl , 5-12 membered spiro cycloalkyl, 5-12 membered spiro heterocycloalkyl are optionally substituted with 1, 2 or 3 G²;
G¹, G² is independently selected from , CN, halogen, C₁₋₆alkyl, C₂₋₆ alkenyl, C₂₋₆ ynyl, C₃₋₈ carbocyclic, 3-8 membered heterocycles,, C₆₋₁₀ aromatic groups, 5-10 membered heteraromatic groups, -OR₅, -OC(O)NR₅R₆, -C(O)OR₅, -C(O)NR₅R₆, -C(O)R₅, -NR₅R₆, -NR₅C(O)R₆, -NR₅C(O)NR₆R₇, -S(O)ᵢR₅ or -NR₅S(O)ᵢR₆, wherein the alkyl, vinyl, alkynyl, cyclic, heterocyclic, aromatic, and heteroaromatic groups are optionally substituted with one or more D, cyano, halogen, C₁₋₆ alkyl, C₂₋₆ alkenyl, C₂₋₆ alkynyl, C₃₋₈ alkyl, or 3-8-membered heterocyclic, C₆₋₁₀ aryl, 6-10 membered heteroaromatic groups, -OR₈, -OC(O)NR₈R₉, -C(O)OR₈, -C(O)NR₈R₉, -C(O)R₈, -NR₈R₉, -NR₈C(O)R₉, -NR₈C(O)NR₉R₁₀, -S(O)ᵢR₈ or -NR₈S(O)ᵢR₉;
R₅, R₆, R₇, R₈, R₉ and R₁₀ are each independently selected from H, D, CN, halogen, C₁₋₆ alkyl, 3-8 membered cycloalkyl, 3-8 membered heterocycloalkyl, sing ring heteroaromatic or phenyl group;
m, i is selected from 1 or 2.

2. The compound of claim 1 or a pharmaceutically acceptable salt thereof, wherein the compound is represented by the general formula (II-A), (II-B), (II-C) or (II-D):

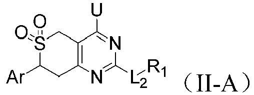

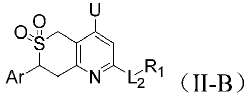

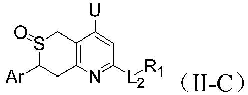

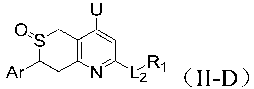

3. The compound according to claim 1 or a pharmaceutically acceptable salt thereof, wherein Ar is selected from:

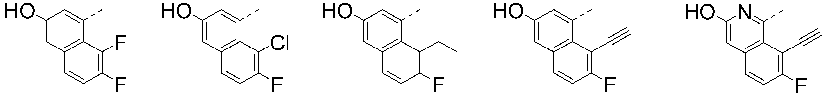

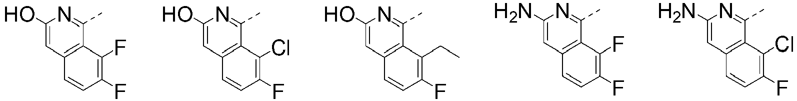

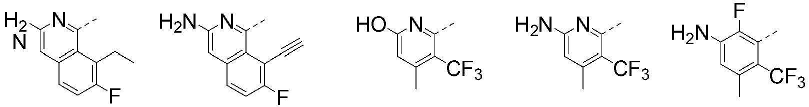

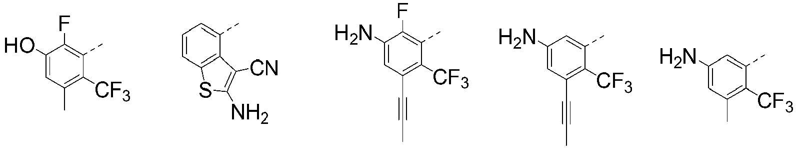

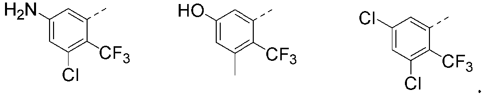

4. The compound according to claim 1 or a pharmaceutically acceptable salt thereof, wherein the compound is selected from:

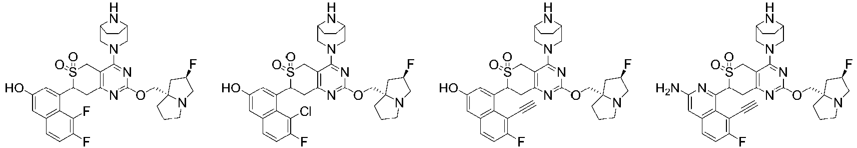

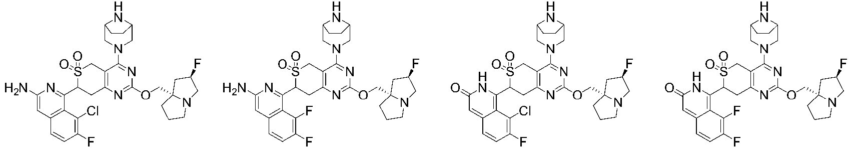

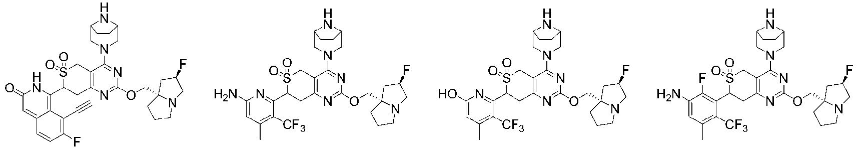

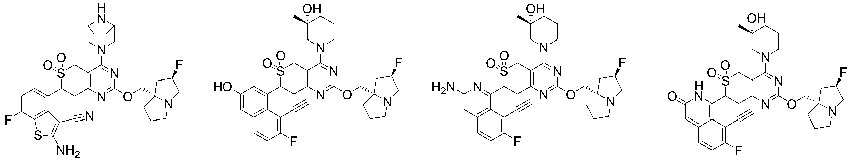

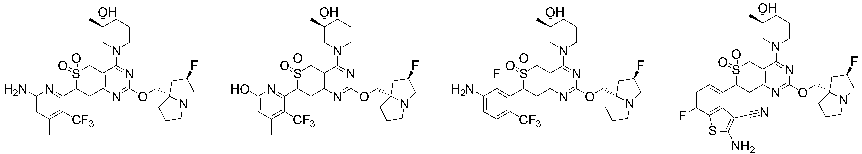

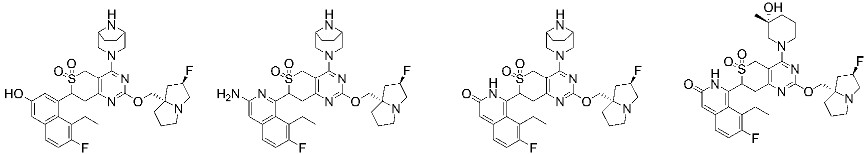

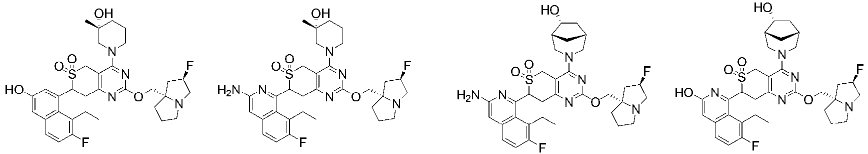

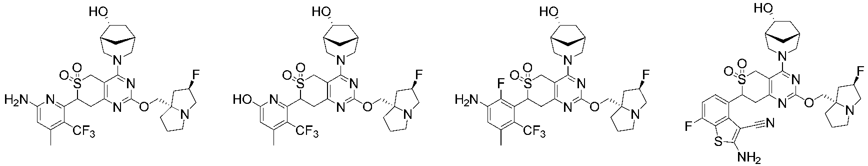

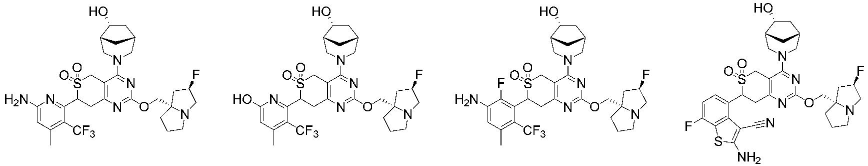

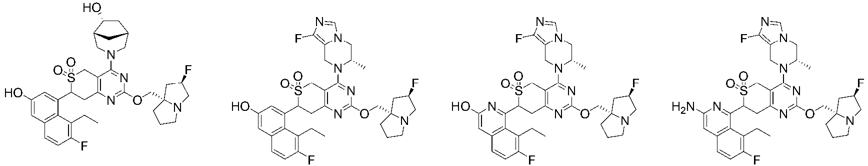

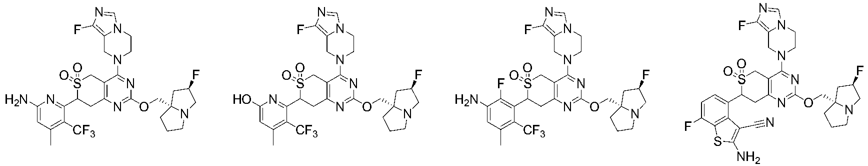

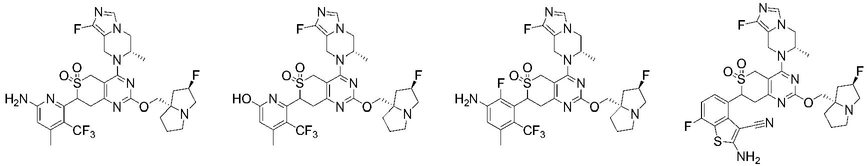

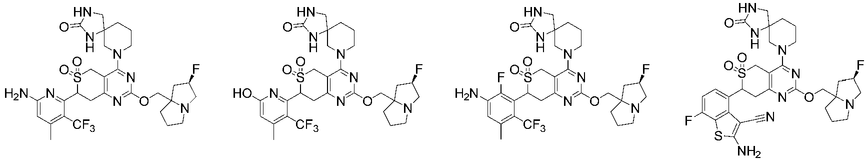

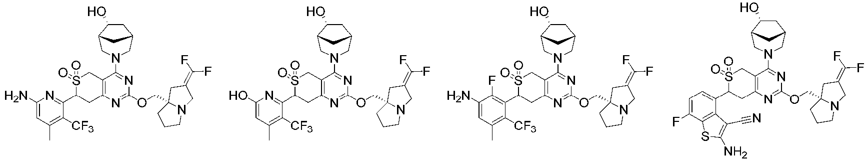

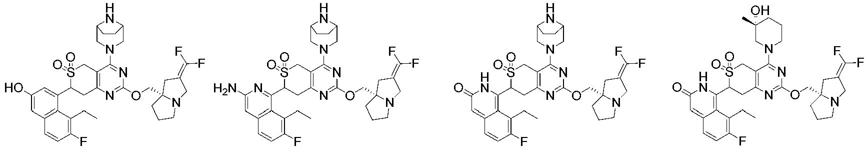

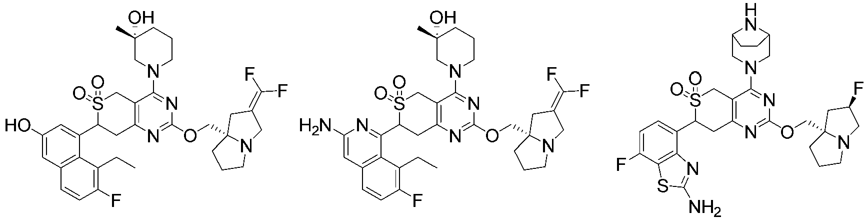

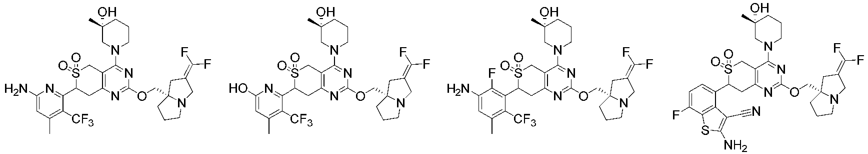

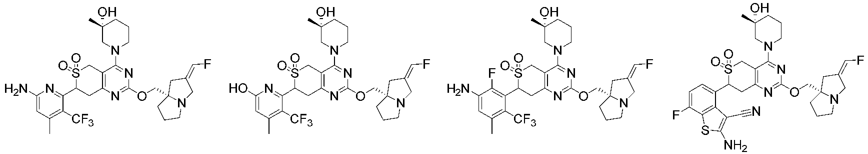

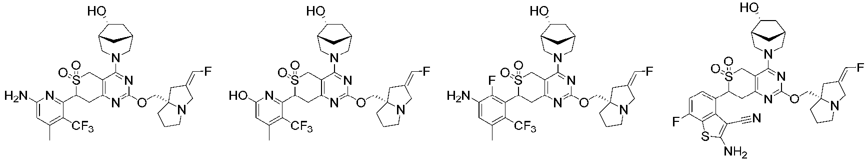

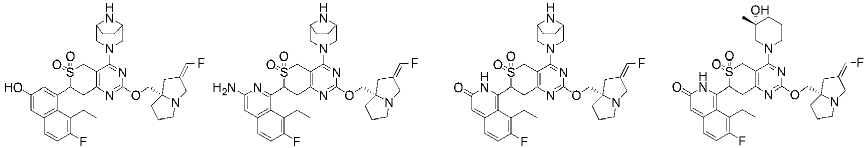

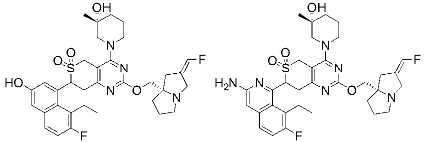

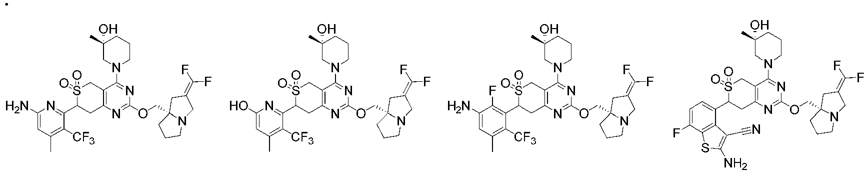

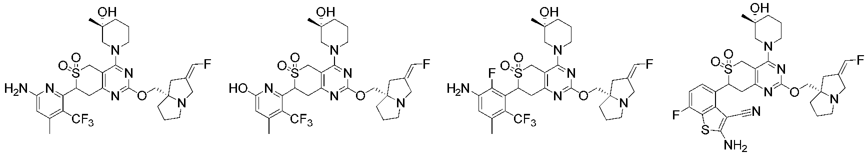

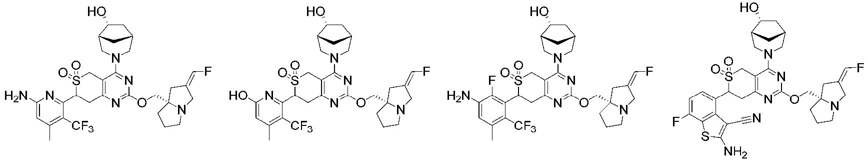

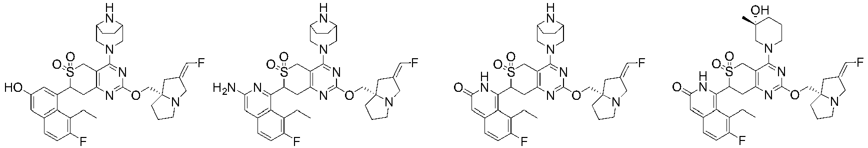

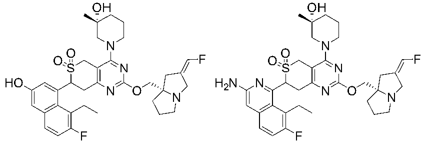

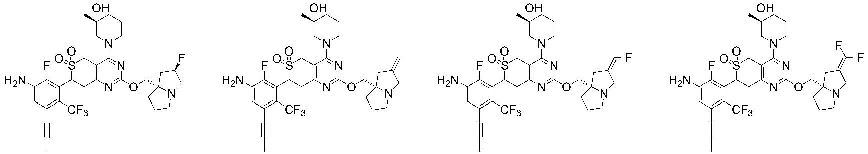

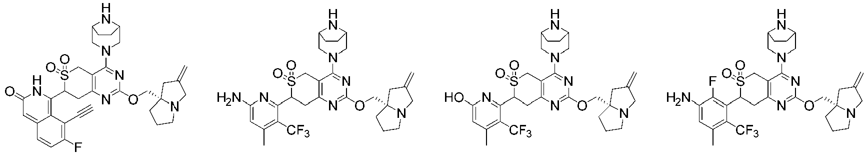

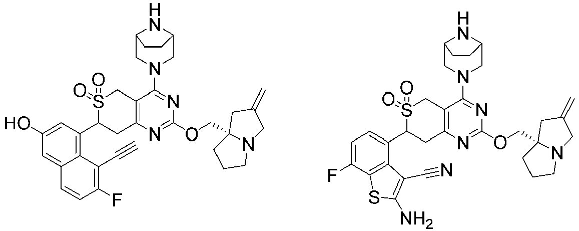

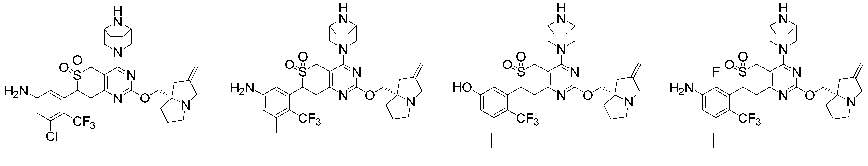

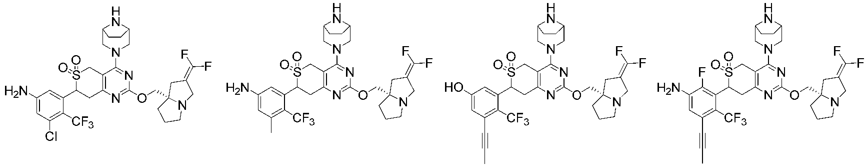

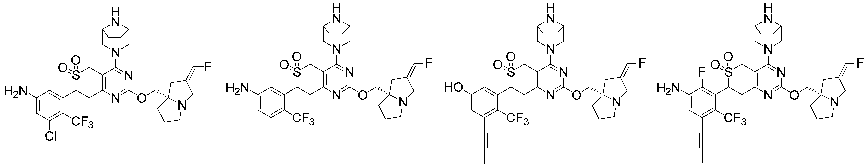

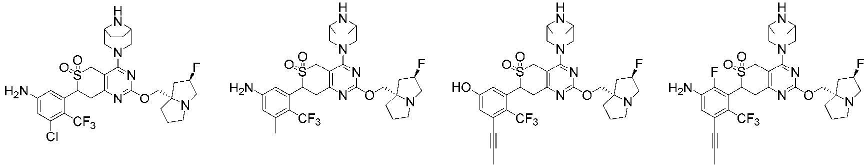

5. The compound according to claim 4 or a pharmaceutically acceptable salt thereof, wherein the compound is selected from:

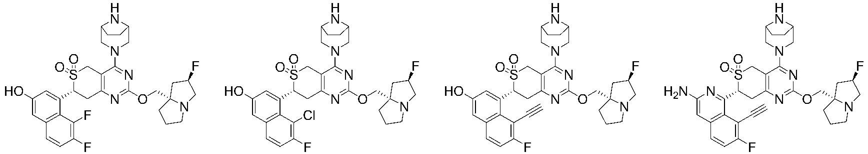

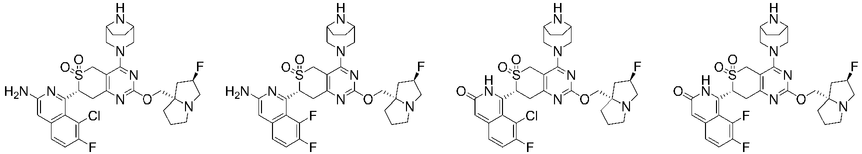

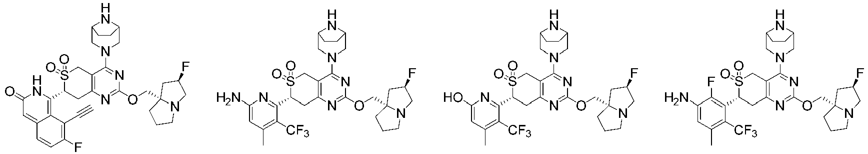

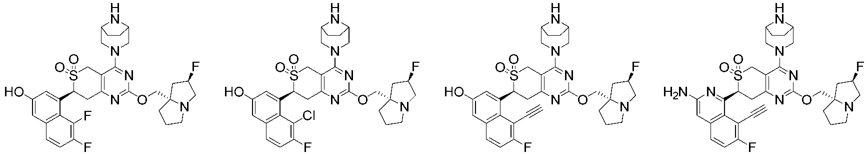

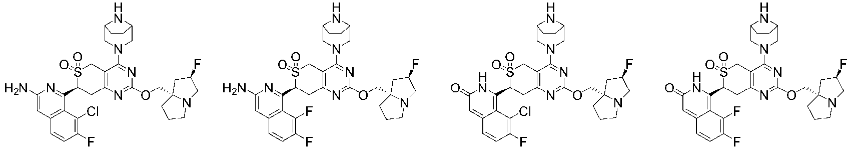

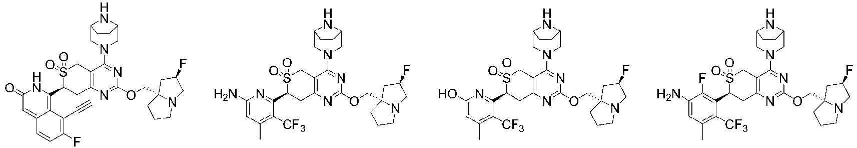

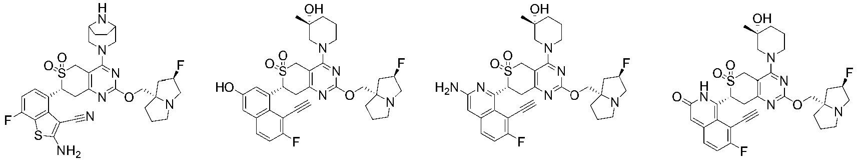

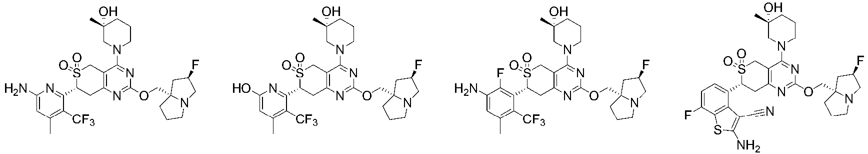

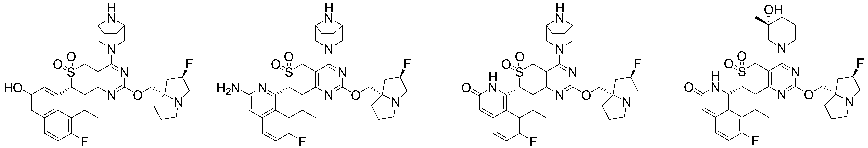

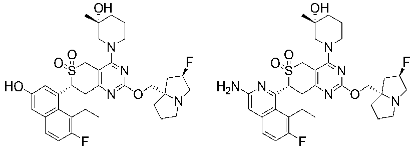

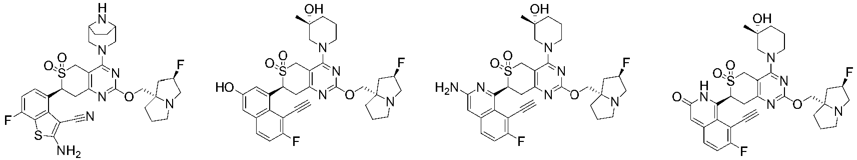

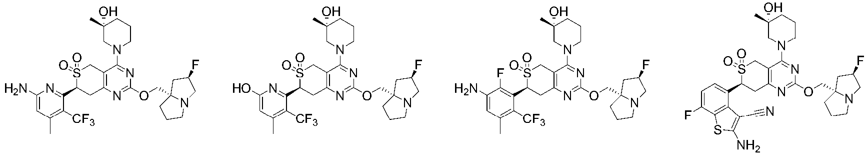

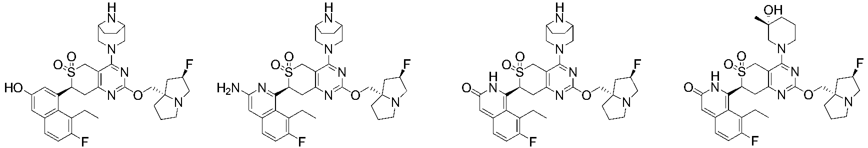

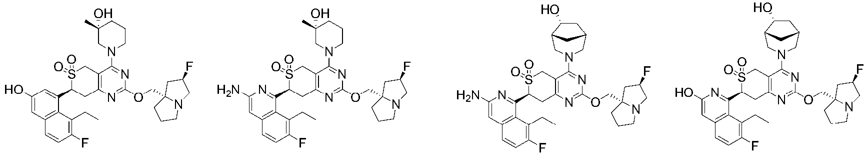

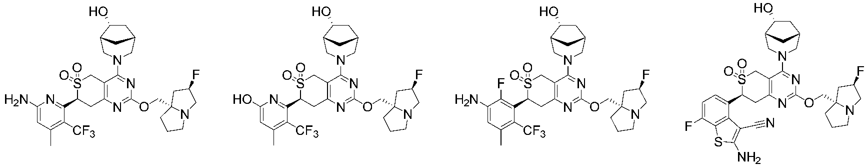

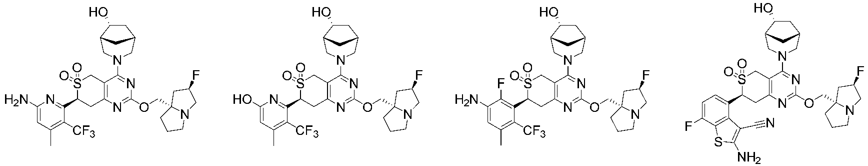

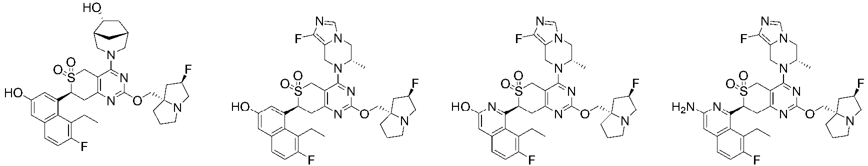

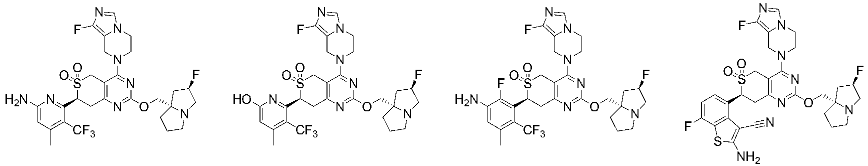

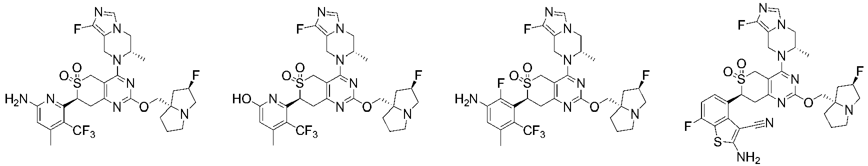

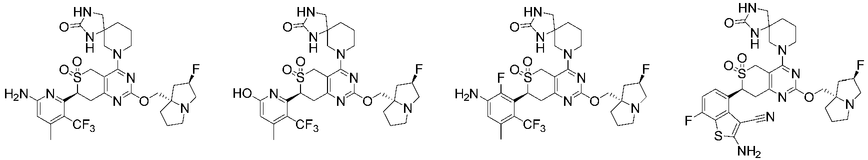

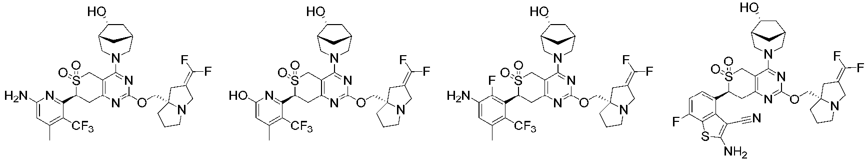

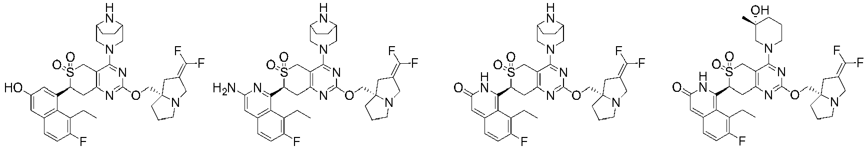

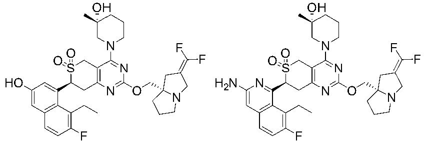

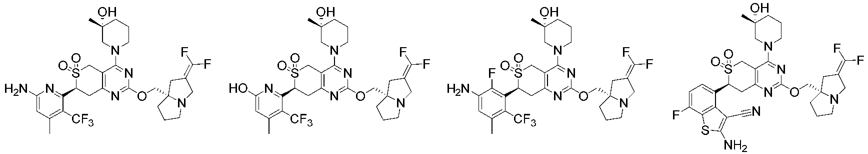

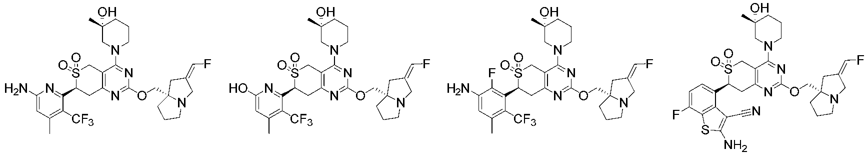

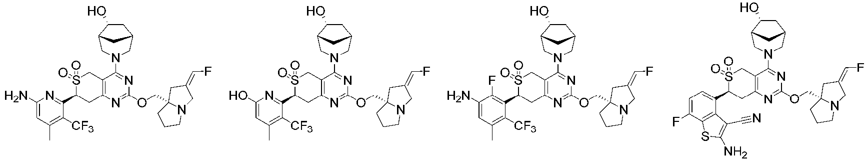

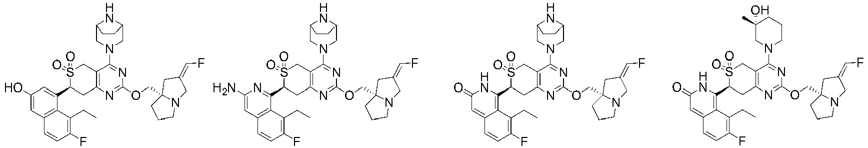

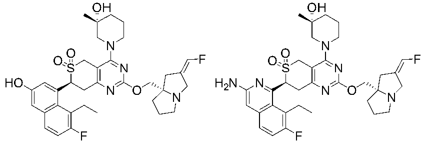

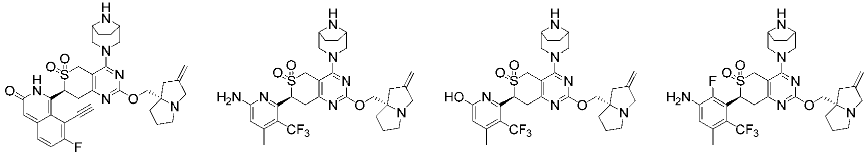

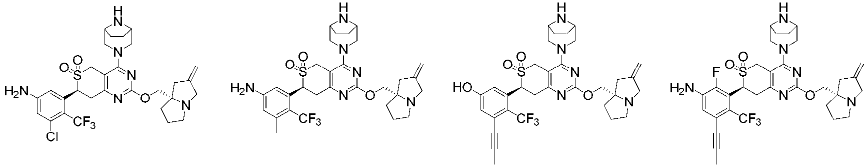

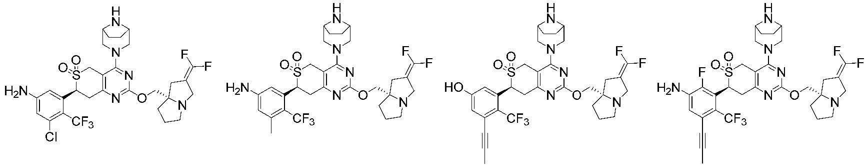

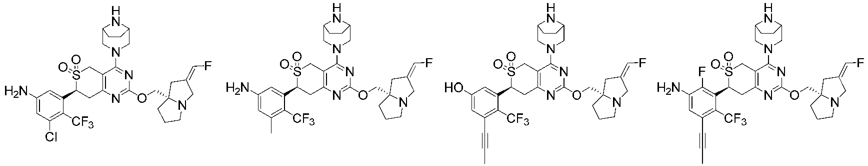

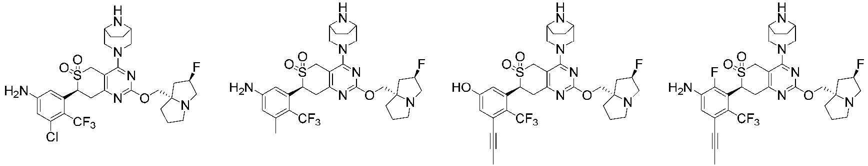

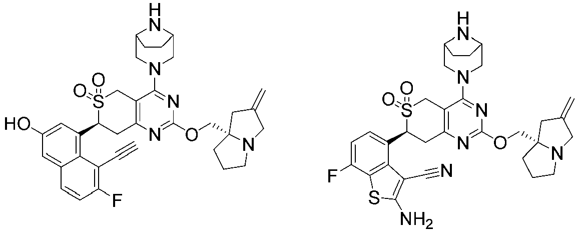

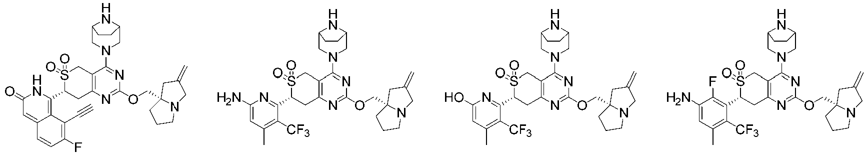

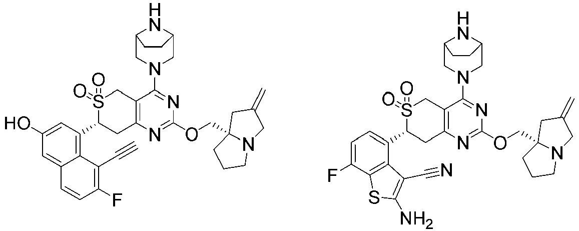

6. The compound of any one of claims 1-5, or an optical isomer thereof, a pharmaceutically acceptable salt, a prodrug, a deuterated derivative, a hydrate, or a solvate, **characterized in that** the pharmaceutically acceptable salt is selected from the group consisting of potassium, sodium, magnesium, calcium, sulfate, hydrochloride, phosphate, sulfonate, or carbonate.

7. A pharmaceutical composition, **characterized in that** it comprises the compound of any one of claims 1-6, or an optical isomer thereof, a pharmaceutically acceptable salt, a prodrug, a deuterated derivative, a hydrate, a solvate, and a pharmaceutically acceptable carrier.

8. Use of the compound of any one of claims 1-6, or an optical isomer thereof, a pharmaceutically acceptable salt, a prodrug, a deuterated derivative, a hydrate, or a solvate, **characterized in that** it is used to prepare a pharmaceutical composition for treating diseases, conditions, or symptoms related to KRas, KRas G12A, KRas G12C, KRas G12D, KRas G12R, KRas G12S, KRas G12V, KRas G13D or KRas Q61H activity or expression levels.

9. The use as described in claim 8, **characterized in that** the disease, symptom, or condition is selected from the group consisting of: pancreatic cancer, non-small cell lung cancer, small cell lung cancer, lung adenocarcinoma, lung squamous cell carcinoma, colon cancer, colorectal cancer, thyroid cancer, embryonal rhabdomyosarcoma, granulosa cell tumor of the skin, melanoma, liver cancer, rectal cancer, bladder cancer, pharyngeal cancer, breast cancer, prostate cancer, glioma, ovarian cancer, head and neck squamous cell carcinoma, cervical cancer, esophageal cancer, kidney cancer, skin cancer, lymphoma, gastric cancer, acute myeloid leukemia, myelofibrosis, B-cell lymphoma, monocytic leukemia, splenomegaly with polycythemia, eosinophilic leukocytosis, multiple myeloma, and various solid tumors and hematologic malignancies.
